# FASCICULE DE BREVET EUROPEEN

(11) **EP 1 066 022 B1**
(45) Date de publication et mention de la délivrance du brevet: **22.10.2003**
(21) Numéro de dépôt: 00900628.9
(22) Date de dépôt: 20.01.2000
(51) Int. Cl.: A61K 7/13

(54) **COMPOSITIONS POUR LA TEINTURE D'OXYDATION DES FIBRES KERATINIQUES COMPRENANT UN COUPLEUR CATIONIQUE, NOUVEAUX COUPLEURS CATIONIQUES, LEUR UTILISATION POUR LA TEINTURE D'OXYDATION, ET PROCEDES DE TEINTURE**
ZUSAMMENSETZUNGEN ZUR FÄRBUNG KERATINISCHER FASERN, ENTHALTEND EINEN KATIONISCHEN KUPPLER, NEUE KATIONISCHE KUPPLER, DEREN VERWENDUNG ZUR OXIDATIVEN FÄRBUNG UND FÄRBEVERFAHREN
COMPOSITIONS FOR OXIDATION DYEING KERATIN FIBRES COMPRISING A CATIONIC COUPLER, NOVEL CATIONIC COUPLERS, THEIR USE FOR OXIDATION DYEING AND DYEING METHODS

(30) Priorité: 21.01.1999 FR 9900638
(43) Date de publication de la demande: 10.01.2001
(73) Titulaire: L'OREAL, 75008 Paris (FR)
(72) Inventeur: VIDAL, Laurent, F-75013 Paris (FR); SAUNIER, Jean-Baptiste, F-75014 Paris (FR)
(74) Mandataire: Miszputen, Laurent
(86) Numéro de dépôt international: FR0000126
(87) Numéro de publication internationale: WO00042979

(56) Documents cités:
- EP-A- 0 345 728
- EP-A- 0 366 542
- EP-A- 0 579 204

## Description

L'invention a pour objet une composition pour la teinture d'oxydation des fibres kératiniques, et en particulier des fibres kératiniques humaines telles que les cheveux, comprenant au moins une base d'oxydation et, à titre de coupleur, au moins un 2-acylaminophénol de formule (I) comportant au moins un groupement cationique Z de formule (II), leur utilisation à titre de coupleur pour la teinture d'oxydation des fibres kératiniques, les procédés de teinture d'oxydation les mettant en oeuvre, ainsi que de nouveaux 2-acylaminophénols cationiques de formule (I').

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour obtenir des nuances rouges, on utilise généralement, seul ou en mélange avec d'autres bases, et en association avec des coupleurs appropriés, du 4-aminophénol, et pour obtenir des nuances bleues, on fait habituellement appel à des paraphénylènediamines. L'utilisation de coupleurs dérivés de métaphénylènediamines, en association avec des dérivés de paraphénylènediamines, conduit habituellement à des nuances bleues de solidité généralement médiocre.

Il a déjà été proposé, notamment dans le brevet FR-A-1 596 879, d'utiliser pour la teinture d'oxydation des fibres kératiniques, des dérivés phénoliques substitués en position 2 par un radical uréinyle ou thiouréinyle, en association avec des dérivés de paraphénylènediamines, afin d'obtenir des nuances proches de celles obtenues avec les coupleurs dérivés de métaphénylènediamines . Toutefois, les compostions tinctoriales contenant les composés cités dans ce brevet conduisent généralement sur cheveux à des couleurs trop sélectives et manquant d'intensité.

Par ailleurs, il a déjà été proposé, notamment dans le brevet BE 816 674, d'utiliser pour la teinture de fibres kératiniques, en association avec des dérivés de paraphénylènediamines, des dérivés phénoliques substitués en position 2 par un radical acétyle ou ureïque et en position 5 par un atome d'halogène, afin d'obtenir des teintures allant du vert au bleu-vert. Les ténacités à la lumière des nuances obtenues sur cheveux en mettant en oeuvre ces compositions sont généralement meilleures que celles obtenues avec des compositions tinctoriales contenant un ou plusieurs métaphénylènediamines à titre de coupleurs. Cependant, les ténacités aux intempéries et aux lavages, ainsi que les intensités des colorations obtenues sont encore trop faibles et constituent en ces points des inconvénients majeurs pour l'homme de l'art.

Le document EP 345 728 décrit des compositions tinctoriales contenant des bases d'oxydation du type 4-aminophénol.

Le document EP 366 542 décrit des compositions tinctoriales comprenant des précurseurs de colorants du type orthoaminophénol substitué en position 5 par un groupe acylamino. Les composés ne sont pas substitués par un groupe cationique ammonium quaternaire.

En outre, il a déjà été proposé, notamment dans la demande de brevet EP 0 579 204, d'utiliser pour la teinture de fibres kératiniques, des dérivés phénoliques non cationiques substitués en position 2 par un radical acylamino, carbamoyle ou uréyles, et en position 5 par un radical alkyle en C₁-C₄, en association avec des dérivés de paraphénylènediamine. Toutefois, l'utilisation des dérivés phénoliques cités dans cette demande de brevet européen ne permet pas d'obtenir une riche palette de couleurs, et de plus les nuances bleues généralement obtenues ne donnent pas entièrement satisfaction en ce qui concerne leur résistance aux lavages et à l'action de la lumière.

Or la demanderesse vient maintenant de découvrir, de façon totalement inattendue et surprenante, que l'utilisation à titre de coupleur de 2-acylaminophénols de formule (I) définie ci-après et comportant au moins un groupement cationique Z de formule (II) telle que définie ci-après, permet d'obtenir des compositions tinctoriales conduisant à des colorations puissantes, dans les nuances allant du rouge au bleu et présentant de plus une ténacité remarquable tant à la lumière qu'aux intempérie, aux lavages, à la transpiration ou encore à la permanente.

Ces découvertes sont à la base de la présente invention.

L'invention a donc pour premier objet une composition pour la teinture d'oxydation des fibres kératiniques, et en particulier des fibres kératiniques humaines telles que les cheveux, caractérisée par le fait qu'elle contient, dans un milieu approprié pour la teinture :
- au moins une base d'oxydation, et
- au moins un coupleur choisi parmi les composés de formule (I) suivante, et leurs sels d'addition avec un acide : 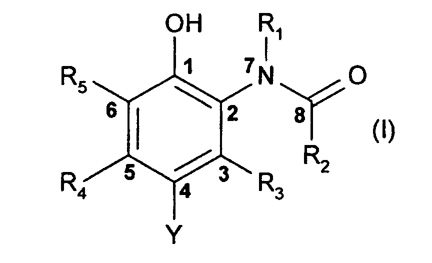
dans laquelle :
- R₁ représente un atome d'hydrogène ; un radical méthyle, éthyle, isopropyle, allyle, phényle, benzyle, fluorobenzyle, hydroxybenzyle, difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle, 2-naphtométhyl ; ou un groupement 2-hydroxyéthyle, 2-méthoxyéthyle ou 2-éthoxyéthyle;
- R₂ représente un atome d'hydrogène, un groupement Z; un radical choisi dans le groupe **(G1)** constitué par un radical méthyle, éthyle, propyle, isopropyle, butyle, isobutyle, tert-butyle, pentyle, isopentyle, néopentyle, hexyle ; cyclopropyle, cyclobutyle, cyclopentyle, cyclopentylméthyle, 3-cyclopentyl-propyle, cyclohexyle, 2-cyclohexyl-éthyle, norbornane-2-yl, vinyle, 1-méthylvinyle, 2-méthylvinyle, 2,2-diméthylvinyle, allyle, 3-butényle ; phényle, méthylphényle, diméthylphényle, 2,4,6-triméthylphényle, 4-éthylphényle, (trifluorométhyl)phényle, hydroxyphényle, méthoxyphényle, éthoxyphényle, acétoxyphényle, (trifluorométhoxy)phényle, aminophényle, 4-diméthylamino-phényle, fluorophényle, difluorophényle, fluoro(trifluorométhyl)phényle, chlorophényle, dichlorophényle, bromophényle, napht-1-yle, napht-2-yle, (2-méthoxy)napht-1-yl, benzyle, 4'-méthoxybenzyle, 2',5'-diméthoxybenzyle, 3',4'-diméthoxybenzyle, 4'-fluorobenzyle, 4'-chlorobenzyle, phénéthyle, 2-phénylvinyle, (1-naphtyl)méthyle, (2-naphtyl)méthyle; tétrahydrofuran-2-yl, furan-2-yl, 5-méthyl-2-(trifluorométhyl)furan-3-yl, 2-méthyl-5-phénylfuran-3-yl, thiophène-2-yl, (thiophène-2-yl)méthyle, 3-chlorothiophène-2-yl, 2,5-dichlorothiophène-3-yl, benzothiophène-2-yl, 3-chlorobenzothiophène-2-yl, isoxazole-5-yl, 5-méthylisoxazole-3-yl, 3,5-diméthylisoxazole-4-yl, 1,3-diméthylpyrazole-5-yl, 1-éthyl-3-méthylpyrazole-5-yl, 1-tertbutyl-3-méthylpyrazole-5-yl, 3-tertbutyl-1-méthylpyrazole-5-yl, 4-bromo-1-éthyl-3-méthylpyrazole-5-yl, indole-3-ylcarboxyl, pyridinyl, chloropyridinyl, dichloropyridinyl, 5-(bromo)pyridin-3-yl, pipérazin-2-yl, quinoxal-2-yl ; fluorométhyle, difluorométhyle, trifluorométhyle, 1,1,2,2-tétrafluoroéthyle, pentafluoroéthyle, heptafluoropropyle, 1,1,2,2,3,3,4,4-octafluorobutyle, nonafluorobutyle, chlorométhyle, chloroéthyle, 1,1-diméthyl-2-chloroéthyle, 1,2-dichloroéthyle, 1-chloropropyle, 3-chloropropyle,4-chlorobutyle, hydroxyméthyle, méthoxyméthyle, phénoxyméthyle, (4-chlorophénoxy)méthyle, benzyloxyméthyle, acétoxyméthyle, 1,2-dihydroxyéthyle, 1-phénoxyéthyle, 1-acétoxyéthyle, 2-(2-carboxyéthoxy)éthyle, 1-phénoxyéthyle, 1-acétoxyéthyle, méthoxycarbonyl, éthoxycarbonyl, (méthoxycarbonyl)méthyle, 2-carboxyéthyle, 2-(méthoxycarbonyl)éthyle, 2-carboxycylopropyle, 2-carboxycyclohexane; méthoxy, éthoxy, propoxy, isopropoxy, butoxy, isobutoxy, pentoxy, néopentoxy, hexyloxy, cyclopentyloxy, cyclohexyloxy, vinyloxy, allyloxy, propargyloxy, chlorométhoxy, 1-chloroéthoxy, 2-méthoxyéthoxy, 4-chlorobutoxy, phénoxy, 4-méthyphénoxy, 4-fluorophénoxy, 4-bromophénoxy, 4-chlorophénoxy, 4-méthoxyphénoxy, naphth-2-yloxy, benzyloxy; amino, méthylamino, éthylamino, propylamino, isopropylamino, butylamino, cyclohexylamino, allylamino, 2-chloroéthylamino, 3-chloropropylamino, carboxyméthylamino, phénylamino, fluorophénylamino, (trifluorométhyl)phénylamino, chlorophénylamino, bromophénylamino, 4-acétylphénylamino, méthoxyphénylamino, (trifluorométhoxy)phénylamino, naphth-1-ylamino, benzylamino, phénéthylamino, pyrid-3-ylamino, diméthylamino, et 1-pyrolidinyle, 4-morpholinyle,
- R₃ et R₄ identiques ou différents représentent un atome d'hydrogène ou d'halogène ; un groupement hydroxyle ou amino ; un groupement Z; un groupement A₁, A₄, ou A₅ tels que définis ci dessous; un groupement A₁, A₂, A₃, A₄ ou A₅ tels que définis ci dessous et séparés du noyau phénolique de la formule (I) par un atome d'oxygène ou par un groupement -NH-, -Nalkyl(C₁-C₃)-, -O(CO)-, -NH(CO)-, -Nalkyl(C₁-C₃)CO)-, -NH[C=NH]-, -NH(CO)NH-, -NH(CO)Nalkyl(C₁-C₃)-, -NH(CO)O-, -NHSO₂-, -NHSO₂NH-, ou -NHSO₂Nalkyl(C₁-C₃)-
   - le groupement A₁ représente les radicaux alkyle en C₁-C₈, linéaire ou ramifié, pouvant porter une ou deux doubles liaisons ou une triple liaison, être substitué ou non substitué par un groupement choisi parmi un groupement A₂, A₄, et A₅ tels que définis ci-dessous, être substitué ou non substitué par un ou deux groupements, identiques ou différents, choisis parmi les groupements N-alkyl(C₁-C₃)amino, N-alkyl(C₁-C₃)-N-alkyl(C₁-C₃)amino, alcoxy(C₁-C₆), oxo, alcoxycarbonyle, acyloxy, amide, acylamino, uréyle, sulfoxy, sulfonyle, sulfonamido, sulfonylamino, bromo, cyano, carboxy, et être substitué ou non substitué par un ou plusieurs groupements hydroxyle, fluoro ou chloro ;
   - le groupement A₂ représente un groupement aromatique de type phényle ou naphtyle, pouvant être substitué ou non substitué par un à trois groupements, identiques ou différents, choisis parmi les groupements méthyle, trifluorométhyle, éthyle, isopropyle, butyle, pentyle, fluoro, chloro, bromo, méthoxy, trifluorométhoxy, éthoxy, propyloxy, acétyloxy, acétyle, et cyano ;
   - le groupement A₃ représente des groupements hétéroaromatiques choisis parmi les groupements furanyle, thiophényle, pyrrolyle, imidazolyle, thiazolyle, oxazolyle, 1,2,3-triazolyle, 1,2,4-triazolyle, isoxazolyle, isothiazolyle, pyrazolyle, pyrazoltriazolyle, pyrazoloimidazolyle, pyrrolotriazolyle, pyrazolopyrimidyle, pyrazolopyridyle, pyridyle, pyrimidyle, benzoimidazolyle, benzoxazolyle, benzothiazolyle, indolyle, indolidinyle, isoindolyle, indazolyle, benzotriazolyle, quinolinyle, benzoimidazolyle, benzopyrimidyle, lesdits groupements étant substitués ou non substitués par 1 à 3 radicaux choisis parmi les radicaux alkyle en C₁-C₄, linéaire ou ramifié, monohydroxyalkyle en C₁-C₄, polyhydroxyalkyle en C₂-C₄, carboxy, alkoxycarbonyle, halogène, amido, amino et hydroxy ;
   - le groupement A₄ représente un radical cycloalkyle en C₃-C₇, un radical norbornanyle, portant ou non une double liaison et substitué ou non substitué par 1 ou 2 radicaux choisi parmi les radicaux alkyle en C₁-C₄, linéaire ou ramifié, monohydroxyalkyle en C₁-C₄, polyhydroxyalkyle en C₂-C₄, carboxy, alkoxycarbonyle, halogène, amido, amino et hydroxy ; ou
   - le groupement A₅ représente un hétérocycle choisi parmi les cycles dihydrofuranyle, tétrahydrofuranyle, butyrolact-one-yle, dihydrothiophényle, tétrahydrothiophényle, tétrahydrothiophén-one-yle, iminothiolane, dihydropyrrolyle, pyrrolidinyle, pyrrolidin-one-yle, imidazolidin-one-yle, imidazolidinthione-yle, oxazolidinyle, oxazolidin-one-yle, oxazolanethione, thiazolidinyle, isothiazol-one-yle, mercaptothiazolinyle, pyrazolidin-one-yle, iminothiolane, dioxolanyle, pentalactone, dioxanyle, dihydropyridinyle, pipéridinyle, pentalactame, morpholinyle, pyrazoli(di)nyle, pyrimi(di)nyl, pyrazinyle, pipérazinyle et azépinyle,
- R₅ représente un atome d'hydrogène ou d'halogène ; un groupement Z ; un groupement A₁, A₄, ou A₅; un groupement A₁, A₂, A₃, A₄ ou A₅ séparés du noyau phénolique des composés de formule (I) par un atome d'oxygène, de soufre, ou par un groupement -NH-, -Nalkyl(C₁-C₃)-, -O(CO)-, -NH(CO)-,-Nalkyl(C₁-C₃)(CO)-, -NH[C=NH]-, -NH(CO)NH-, -NH(CO)Nalkyl(C₁-C₃)-, ou-NH(CO)O-,
- Y représente un atome d'hydrogène ou d'halogène ; un groupement -OCH₂COOH, -OCH₂COOCH₃, -OCH₂COOC₂H₅, -OR₆, -SR₆ ou -NH-SO₂R₆ dans lesquels R₆ représente un radical alkyle en C₁-C₆, linéaire ou ramifié (la ou les ramifications pouvant alors former un ou plusieurs cycles comportant de 3 à 6 chaînons), substitué ou non substitué par un ou plusieurs radicaux choisis dans le groupe constitué par un atome d'halogène, un radical hydroxy, alcoxy en C₁-C₄, amino et aminoalkyl en C₁-C₄ ; un radical phényle substitué ou non substitué par un ou deux radicaux choisi dans le groupe constitué par un radical alkyle en C₁-C₄, trifluorométhyle, carboxy, alcoxycarbonyle en C₁-C₄, halogène, hydroxy, alcoxy en C₁-C₄, amino, et aminoalkyl en C₁-C₄ ; ou un radical benzyle ; et étant entendu que Y ne peut représenter -NH-SO₂R₆ lorsque R₃ représente un radical hydroxyle ;
- Z est un groupement cationique représenté par la formule (II) suivante: 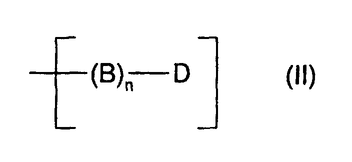
dans laquelle :
- B représente un radical comportant de 1 à 15 atomes de carbone, linéaire ou ramifié (la ou les ramifications pouvant alors former un ou plusieurs cycles comportant de 3 à 7 chaînons), pouvant contenir une ou plusieurs liaisons doubles et/ou une ou plusieurs liaisons triples, lesdites liaisons doubles conduisant éventuellement à des groupements aromatiques, et dont un ou plusieurs atomes de carbone peuvent être remplacés par un atome d'oxygène, d'azote, ou de soufre ou par un radical SO₂ ; et dont un ou plusieurs atomes de carbone peuvent, indépendamment les uns des autres, être substitués par un ou plusieurs atomes d'halogène ou par un ou plusieurs groupements Z ; ledit radical B ne comportant pas de liaison peroxyde ni de radicaux diazo, nitro ou nitroso ;
- n est égal à 0 ou 1,
- D est choisi parmi les groupements cationiques imidazolinium, thiazolinium, oxazolinium, pyrrolinium, 1,2,3-triazolinium, 1,2,4-triazolinium, isoxazolinium, ixothiazolinium, imidazolidinium, thiazolidinium, pyrazolinium, pyrazolidinium, oxazolidinium, pyrazoltriazolinium, pyrazoloimidazolinium, pyrrolotriazolinium, pyrazolopyrimidinium, pyrazolopyridinium, pyridinium, pyrimidinium, pyrazinium, triazinium, benzoimidazolinium, benzoxazolinium, benzothiazolinium, indolinium, indolidinium, isoindolinium, indazolinium, benzotriazolinium, quinolinium, tétrahydroquinolinium, benzoimidazolidinium, benzopyrimidinium, tétra-alkyl(C₁-C₄)ammonium, polyhydroxyltétra-alkyl(C₁-C₄)ammonium, dialkylpipéridinium, dialkylpyrrolidinium, dialkylmorpholinium, dialkylthiomorpholinium, dialkylpipérazinium, azépinium, et 1,4-diazabicyclo[2,2,2]octanium, étant entendu qu'au moins un des groupements R₁ à R₅ représente un groupement Z

Comme indiqué précédemment, la composition de teinture d'oxydation contenant le ou les composés de formule (I) conforme à l'invention permet d'obtenir des colorations puissantes dans des nuances allant du rouge au bleu et présentant de plus une ténacité remarquable aux différents traitements que peuvent subir les fibres kératiniques. Ces propriétés sont particulièrement remarquables notamment en ce qui concerne la résistance des colorations obtenues vis à vis de l'action de la lumière, des intempéries, des lavages, de l'ondulation permanente et de la transpiration.

Selon l'invention, on entend par groupement A₁ un radical alkyle en C₁-C₈, linéaire ou ramifié, pouvant porter une ou deux doubles liaisons ou une triple liaison, être substitué ou non substitué par un groupement choisi parmi un groupement A₂, A₄, et A₅, être substitué ou non substitué par un ou deux groupements, identiques ou différents, choisis parmi les groupements N-alkyl(C₁-C₃)amino, N-alkyl(C₁-C₃)-N-alkyl(C₁-C₃)amino, alcoxy(C₁-C₆), oxo, alcoxycarbonyl, acyloxy, amide, acylamino, uréyle, sulfoxy, sulfonyle, sulfonamido, sulfonylamino, bromo, cyano, carboxy, et être substitué ou non substitué par un ou plusieurs groupements hydroxyle, fluoro ou chloro.

On entend par groupement A₂, un groupement aromatique de type phényle ou naphtyle, pouvant être substitué ou non substitué par un à trois groupements, identiques ou différents, choisis parmi les groupements méthyle, trifluorométhyle, éthyle, isopropyle, butyle, pentyle, fluoro, chloro, bromo, méthoxy, trifluorométhoxy, éthoxy, propyloxy, acétyloxy, acétyle, et cyano.

On entend par groupement A₃, des groupements hétéroaromatiques choisis parmi les groupements furanyle, thiophènyle, pyrrolyle, imidazolyle, thiazolyle, oxazolyle, 1,2,3-triazolyle, 1,2,4-triazolyle, isoxazolyle, isothiazolyle, pyrazolyle, pyrazoltriazolyle, pyrazoloimidazolyle, pyrrolotriazolyle, pyrazolopyrimidyle, pyrazolopyridyle, pyridyle, pyrimidyle, benzoimidazolyle, benzoxazolyle, benzothiazolyle, indolyle, indolidinyle, isoindolyle, indazolyle, benzotriazolyle, quinolinyle, benzoimidazolyle, benzopyrimidyle, lesdits groupements étant substitués ou non substitués par 1 à 3 radicaux choisis parmi les radicaux alkyle en C₁-C₄, linéaire ou ramifié, monohydroxyalkyle en C₁-C₄, polyhydroxyalkyle en C₂-C₄, carboxy, alkoxycarbonyl, halogène, amido, amino et hydroxy.

On entend par groupement A₄, un radical cycloalkyle en C₃-C₇, un radical norbornanyle, portant ou non une double liaison et substitué ou non substitué par 1 ou 2 radicaux choisi parmi les radicaux alkyle en C₁-C₄, linéaire ou ramifié, monohydroxyalkyle en C₁-C₄, polyhydroxyalkyle en C₂-C₄, carboxy, alkoxycarbonyl, halogène, amido, amino et hydroxy.

On entend par groupement A₅ un hétérocycle choisi parmi les cycles dihydrofuranyle, tétrahydrofuranyle, butyrolact-one-yle, dihydrothiophènyle, tétrahydrothiophènyle, tétrahydrothiophén-one-yle, iminothiolane, dihydropyrrolyle, pyrrolidinyle, pyrrolidin-one-yle, imidazolidin-one-yle, imidazolidinthione-yle, oxazolidinyle, oxazolidin-one-yle, oxazolanethione, thiazolidinyle, isothiazol-one-yle, mercaptothiazolinyle, pyrazolidin-one-yle, iminothiolane, dioxolanyle, pentalactone, dioxanyle, dihydropyridinyle, pipéridinyle, pentalactame, morpholinyle, pyrazoli(di)nyle, pyrimi(di)nyl, pyrazinyle, pipérazinyle et azépinyle.

De façon encore plus préférentielle, R₁ représente un atome d'hydrogène ou un radical méthyle.

De façon encore plus préférentielle, R₂ représente un radical choisi dans le groupe **(G2)** constitué par un radical méthyle, éthyle, propyle, allyle, phényle, tétrahydrofuran-2-yl, furan-2-yl, thiophène-2-yl, pyridinyle, pipérazin-2-yl, fluorométhyle, chlorométhyle, 2-chloroéthyle, méthoxyméthyle, acétoxyméthyle, 1,2-dihydroxyéthyle, méthoxycarbonyl, 2-carboxyéthyle, méthoxy, éthoxy, propoxy, allyloxy, 2-chloroéthoxy, 2-méthoxyéthoxy, amino, éthylamino, allylamino, 2-chloroéthylamino, pyridylamino, diméthylamino, 1-pyrolidinyle, et 4-morpholinyle ; ou un groupement -D', -E-D', -O-E-D', -NH-E-D', dans lesquels -E- représente un bras -(CH₂)_{q}-, q étant un nombre entier égal à 1 ou 2, et D' représente un groupement choisi parmi les groupements 3-méthylimidazolidinium-1-yl, 3-(2-hydroxyéthyl)-imidazolidinium-1-yl, 1,2,4-triazolinium-1-yl, 1,2,4-triazolinium-4-yl, N-alkyl(C₁-C₄)pyridinium-2-yl, N-alkyl(C₁-C₄)pyridinium-3-yl, N-alkyl(C₁-C₄)pyridinium-4-yl, N-(2-hydroxyéthyl)pyridinium-2-yl, N-(2-hydroxyéthyl)pyridinium-3-yl, N-(2-hydroxyéthyl)pyridinium-4-yl, pyridinium-1-yl, trialkyl(C₁-C₄)ammonium-N-yl, 1-méthylpipéridinium-1-yl et 1,4-diméthylpipérazinium-1-yl.

De façon encore plus préférentielle, R₂ représente un radical méthyle, méthoxyméthyle, 2-carboxyéthyle, méthoxy, amino, éthylamino, 1-pyrolidinyle; un groupement -D'₁, -E-D', -O-E-D', -NH-E-D', dans lesquels -E- représente un bras -(CH₂)_{q}-, q=1 à 2,

Parmi ces substituants, R₃ représente encore plus préférentiellement un atome d'hydrogène ou de chlore; un groupement Z; un radical méthyle, hydroxyméthyle, méthoxyméthyle, 1-hydroxyéthyle, aminométhyle, méthylaminométhyle, hydroxy, méthoxy, acétoxy, amino, méthylamino, ou 2-hydroxyéthylamino ; un groupement -NH(CO)R₁₂ dans lequel R₁₂ représente un des radicaux listés dans le groupe **(G1)** tel que défini ci-dessus ; un groupement -NHSO₂R₁₃, dans lequel R₁₃ représente un des radicaux listés dans le groupe **(G3)** constitué par les radicaux méthyle, trifluorométhyle, éthyle, 2-chloroéthyle, propyle, 3-chloropropyle, isopropyle, butyle, thiophène-2-yl, hydroxy, éthoxy et diméthylamino.

De façon encore plus préférentielle, R₃ représente un atome d'hydrogène ; un radical méthyle, hydroxyméthyle, aminométhyle, hydroxy, méthoxy, amino, méthylamino ; un groupement méthanesulfonylamino ; éthanesulfonylamino ; diméthylamino-sulfonylamino ; un groupement -NH(CO)R₁₄ dans lequel R₁₄ représente l'un des radicaux listés dans le groupe **(G2)** tel que défini ci-dessus ; ou un groupement -O-E-D', -NH-E-D', -CH₂O-E-D', -CH₂NH-E-D', -CH₂NH(CO)-D', -NH(CO)-D', -NH(CO)-E-D', -NH(CO)O-E-D', -NH(CO)NH-E-D', ou -NH(SO₂)-E-D'.

Parmi ces substituants, R₄ représente de préférence un atome d'hydrogène ou de chlore ; un groupement Z ; un radical méthyle, éthyle, hydroxyméthyle, méthoxyméthyle, aminométhyle, méthylaminométhyle, hydroxy, méthoxy, acétoxy, amino, méthylamino, N-pipéridino, ou N-morpholino ; un groupement -NH(CO)R₁₅ dans lequel R₁₅ représente l'un des radicaux listés dans le groupe **(G1)** défini ci-dessus ; ou un groupement -NHSO₂R₁₆ dans lequel R₁₆ représente l'un des radicaux listés dans le groupe **(G3)** défini ci-dessus.

De façon encore plus préférentielle, R₄ représente un atome d'hydrogène ou de chlore ; un radical méthyle, hydroxyméthyle, aminométhyle, hydroxy, méthoxy, amino, ou méthylamino ; un groupement méthanesulfonylamino ; éthanesulfonylamino ; diméthylaminosulfonylamino ; un groupement -NH(CO)R₁₇ dans lequel R₁₇ représente un des radicaux listés dans le groupe **(G2)** défini ci-dessus ; ou un groupement -O-E-D', -NH-E-D', -CH₂O-E-D', -CH₂NH-E-D', -CH₂NH(CO)-D', -NH(CO)-D', -NH(CO)-E-D', -NH(CO)O-E-D', -NH(CO)NH-E-D', ou -NH(SO₂)-E-D', dans lesquels -E- a la même signification que celle indiquée ci-dessus.

Parmi les substituants de R₅, R₅ représente de préférence un atome d'hydrogène, de chlore, de fluor, ou de brome ; un groupement Z ; un radical méthyle, trifluorométhyle, allyle, hydroxyméthyle, méthoxyméthyle, 1-hydroxyéthyle, aminométhyle, méthylaminométhyle, méthoxy, acétoxy, ou méthylamino ; ou un groupement -NH(CO)R₁₈ dans lequel R₁₈ représente l'un des radicaux listés dans le groupe **(G1)** défini ci-dessus.

De façon encore plus préférentielle, R₅ représente un atome d'hydrogène, de chlore, ou de fluor ; un radical méthyle, hydroxyméthyle, aminométhyle, méthoxy, ou méthylamino ; un groupement -NH(CO)R₁₉ dans lesquels R₁₉ représente un des radicaux listés dans le groupe (G2) défini ci-dessus ; ou un groupement -O-E-D', -NH-E-D', -CH₂O-E-D', -CH₂NH-E-D', -CH₂NH(CO)-D', -NH(CO)-D', -NH(CO)-E-D', -NH(CO)O-E-D', -NH(CO)NH-E-D'.

Selon l'invention, Y est de préférence choisi parmi un atome d'hydrogène, de chlore, de fluor, ou de brome; un groupement méthoxy, éthoxy, propoxy, benzyloxy, phénoxy, -OCH₂CH₂OCH₃; -OCH₂CH₂OCH₃; -OCH₂CH₂N(CH₃)₂; -OCH₂(CO)OH, -OCH₂(CO)OCH₃, -OCH₂(CO)OC₂H₅, -SCH₂CH₂CO₂H, ou -NHSO₂CH₃ ; étant entendu que Y ne peut représenter un groupement -NHSO₂CH₃ lorsque R₃ représente un radical hydroxyle.

De façon encore plus préférentielle, Y représente un atome d'hydrogène ou de chlore un groupement méthoxy, -OCH₂(CO)OH, ou -OCH₂(CO)OCH₃.

De façon encore plus préférentielle, D représente un groupement 3-méthylimidazolidinium-1-yl, 3-(2-hydroxyéthyl)imidazolidinium-1-yl, 1,2,4-triazolinium-1-yl, 1,2,4-triazolinium-4-yl, N-alkyl(C₁-C₄)pyridin-2-yl, N-alkyl(C₁-C₄)pyridin-3-yl, N-alkyl(C₁-C₄)pyridin-4-yl, N-(2-hydroxyéthyl) pyridin-2-yl, N-(2-hydroxyéthyl)pyridin-3-yl, N-(2-hydroxyéthyl)pyridin-4-yl, pyridin-1-yl, trialkyl(C₁-C₄)ammonium-N-yl, 1-méthylpipéridinium-1-yl, thiazolinium-3-yl ou 1,4-diméthylpipérazinium-1-yl.

Parmi les composés de formule (I), on préfère particulièrement ceux dans lesquels :
i)
   - R₁ représente un atome d'hydrogène ;
   - R₂ représente un groupement -D', -E-D', -O-E-D', -NH-E-D', tels que définis ci-dessus ; ou un radical choisi dans le groupe **(G4)** constitué par un radical méthyle, méthoxyméthyle, 2-carboxyéthyle, méthoxy, amino, éthylamino, 1-pyrolidinyle ;
   - R₃ représente un radical hydroxy, amino, ou méthylamino ; un groupement -NH(CO)R₂₀ dans lequel R₂₀ représente l'un des radicaux listés dans le groupe **(G4)** défini ci-dessus ; un groupement méthanesulfonylamino, éthanesulfonylamino, ou diméthylaminosulfonylamino ; un groupement -O-E-D', -NH-E-D', -NH(CO)-D', -NH(CO)-E-D', -NH(CO)O-E-D', -NH(CO)NH-E-D', ou -NH(SO₂)-E-D', tels que définis ci-dessus ;
   - R₄ représente un atome d'hydrogène ou de chlore, ou un groupement méthyle ;
   - R₅ représente un atome d'hydrogène, de chlore ou de fluor, ou un groupement méthyle ;
   - Y représente un atome d'hydrogène ou de chlore ; un groupement méthoxy, ou -OCH₂(CO)OCH₃ ; étant entendu qu'au moins un des groupements R₂ et R₃ contient un groupement Z ;
ii)
   - R₁ représente un atome d'hydrogène ;
   - R₂ représente un groupement -D', -E-D', -O-E-D', ou -NH-E-D', tels que définis ci-dessus ; ou l'un des radicaux listés dans le groupe **(G4)** défini précédemment ;
   - R₃ représente un atome d'hydrogène ; ou un radical méthyle ;
   - R₄ représente un radical hydroxy, amino, méthylamino, méthanesulfonylamino, éthanesulfonylamino, ou diméthylamino-sulfonylamino ; un groupement -NH(CO)R₂₁ dans lequel R₂₁ représente un des radicaux listés dans le groupe **(G4)** défini ci-dessus ; ou un groupement -O-E-D', -NH-E-D', -NH(CO)-D', -NH(CO)-E-D', -NH(CO)O-E-D', -NH(CO)NH-E-D', ou -NH(SO₂)-E-D', tels que définis ci-dessus ;
   - R₅ représente un atome d'hydrogène, de chlore, ou de fluor ; ou un groupement méthyle, méthoxy, ou méthylamino ;
   - Y représente un atome d'hydrogène ou de chlore ; un groupement méthoxy, ou -OCH₂(CO)OCH₃ ; étant entendu qu'au moins un des groupements R₂ et R₄ contient un groupement Z ;
iii)
   - R₁ représente un atome d'hydrogène ;
   - R₂ représente un des radicaux listés dans le groupe **(G4)** défini précédemment ; ou un groupement -D', -E-D', -O-E-D', -NH-E-D', tels que définis ci-dessus ;
   - R₃ représente un atome d'hydrogène ; ou un radical méthyle ;
   - R₄ représente un atome d'hydrogène ou de chlore ; un radical méthyle ; ou un groupement méthoxy, ou méthylamino ;
   - R₅ représente un groupement -NH(CO)R₂₂ dans lequel R₂₂ représente l'un des radicaux listés dans le groupe **(G4)** défini ci-dessus ; ou un groupement -O-E-D', -NH-E-D', -NH(CO)-D', -NH(CO)-E-D', -NH(CO)O-E-D', ou -NH(CO)NH-E-D', tels que définis ci-dessus ;
   - Y représente un atome d'hydrogène ou de chlore ; ou un groupement méthoxy, ou -OCH₂(CO)OCH₃; étant entendu qu'au moins un des groupements R₂ et R₅ contient un groupement Z ;
iv)
   - R₁ représente un atome d'hydrogène ;
   - R₂ représente un groupement -D', -E-D', -O-E-D', ou -NH-E-D', tels que définis précédemment ;
   - R₃ représente un atome d'hydrogène ; ou un radical méthyle ;
   - R₄ représente un atome d'hydrogène ou de chlore ; ou un radical méthyle ;
   - R₅ représente un atome d'hydrogène, de chlore ou de fluor ; ou un groupement méthyle ;
   - Y représente un atome d'hydrogène, ou de chlore ; ou un groupement méthoxy, ou -OCH₂(CO)OCH₃.
Parmi les composés de formule (I) ci-dessus, on peut tout particulièrement citer :
   - le chlorure de 3-[(2-hydroxy-phénylcarbamoyl)-méthyl]-1-méthyl-3H-imidazol-1-ium ;
   - le dichlorure de 3-[(2-hydroxy-3-(2-(3-méthyl-1H-imidazol-3-ium-1-yl)-acétylamino)-phénylcarbamoyl)-méthyl]-1-méthyl-3H-imidazol-1-ium ;
   - le dichlorure de 3-[(2-hydroxy-4-(2-(3-méthyl-1H-imidazol-3-ium-1-yl)-acétylamino)-phénylcarbamoyl)-méthyl]-1-méthyl-3H-imidazol-1-ium ;
   - le chlorure de 3-[(2-hydroxy-4-méthyl-phénylcarbamoyl)-méthyl]-1-méthyl-3H-imidazol-1-ium ;
   - le chlorure de 3-[(2-hydroxy-4-amino-phénylcarbamoyl)-méthyl]-1-méthyl-3H-imidazol-1-ium ;
   - le chlorure de 3-[(2-hydroxy-4-acétylamino-phénylcarbamoyl)-méthyl]-1-méthyl-3H-imidazol-1-ium ;
   - le chlorure de 3-[(2-hydroxy-4-méthoxycarbonylamino-phénylcarbamoyl)-méthyl]-1-méthyl-3H-imidazol-1-ium ;
   - le chlorure de 3-[(3-hydroxy-4-acétylamino-phénylcarbamoyl)-méthyl]-1-méthyl-3H-imidazol-1-ium ;
   - le chlorure de 3-[(3-hydroxy-4-méthoxycarbonylamino-phénylcarbamoyl)-méthyl]-1-méthyl-3H-imidazol-1-ium ;
   - le chlorure de 3-[(2-hydroxy-5-chloro-phénylcarbamoyl)-méthyl]-1-méthyl-3H-imidazol-1-ium ;
   - le chlorure de 3-[(2-hydroxy-4-méthyl-5-chloro-phénylcarbamoyl)-méthyl]-1-méthyl-3H-imidazol-1-ium ;
   - le chlorure de 3-[(2-hydroxy-4-amino-5-chloro-phénylcarbamoyl)-méthyl]-1-méthyl-3H-imidazol-1-ium ;
   - le chlorure de 3-[(2-hydroxy-4-acétylamino-5-chloro-phénylcarbamoyl)-méthyl]-1-méthyl-3H-imidazol-1-ium ;
   - le chlorure de 3-[(2-hydroxy-4-méthoxycarbonylamino-5-chloro-phénylcarbamoyl)-méthyl]-1-méthyl-3H-imidazol-1-ium ;
   - le chlorure de 3-[(3-hydroxy-4-acétylamino-5-chloro-phénylcarbamoyl)-méthyl]-1-méthyl-3H-imidazol-1-ium ;
   - le chlorure de 3-[(3-hydroxy-4-méthoxycarbonylamino-6-chloro-phénylcarbamoyl)-méthyl]-1-méthyl-3H-imidazol-1-ium ;
   - le chlorure de 3-[(2-hydroxy-5-méthoxy-phénylcarbamoyl)-méthyl]-1-méthyl-3H-imidazol-1-ium ;
   - le chlorure de 3-[(2-hydroxy-4-méthyl-5-méthoxy-phénylcarbamoyl)-méthyl]-1-méthyl-3H-imidazol-1-ium ;
   - le chlorure de 3-[(2-hydroxy-4-amino-5-méthoxy-phénylcarbamoyl)-méthyl]-1-méthyl-3H-imidazol-1-ium ;
   - le chlorure de 3-[(2-hydroxy-4-acétylamino-5-méthoxy-phénylcarbamoyl)-méthyl]-1-méthyl-3H-imidazol-1-ium ;
   - le chlorure de 3-[(2-hydroxy-4-méthoxycarbonylamino-5-méthoxy-phénylcarbamoyl)-méthyl]-1-méthyl-3H-imidazol-1-ium ;
   - le chlorure de 3-[(3-hydroxy-4-acétylamino-6-méthoxy-phénylcarbamoyl)-méthyl]-1-méthyl-3H-imidazol-1-ium ;
   - le chlorure de 3-[(3-hydroxy-4-méthoxycarbonylamino-6-méthoxy-phénylcarbamoyl)-méthyl]-1-méthyl-3H-imidazoi-1-ium ;
   - le chlorure de 3-[(2-hydroxy-6-amino-phénylcarbamoyl)-méthyl]-1-méthyl-3H-imidazol-1-ium ;
   - le chlorure de 3-[(2-hydroxy-6-acétylamino-phénylcarbamoyl)-méthyl]-1-méthyl-3H-imidazol-1-ium ;
   - le chlorure de 3-[(2-hydroxy-4,6-diamino-phénylcarbamoyl)-méthyl]-1-méthyl-3H-imidazol-1-ium ;
   - le chlorure de 3-[(2-hydroxy-4-acétylamino-6-amino-phénylcarbamoyl)-méthyl]-1-méthyl-3H-imidazol-1-ium ;
   - le chlorure de 3-[(2-hydroxy-3,5-dichloro-4-méthyl-phénylcarbamoyl)-méthyl]-1-méthyl-3H-imidazol-1-ium ;
   - le chlorure de 3-[(2-hydroxy-3,5-dichloro-4-amino-phénylcarbamoyl)-méthyl]-1-méthyl-3H-imidazol-1-ium ;
   - le chlorure de 3-[(2-hydroxy-3,5-dichloro-4-acétylamino-phénylcarbamoyl)-méthyl]-1-méthyl-3H-imidazol-1-ium ;
   - le chlorure de 3-[(2-hydroxy-3,5-dichloro-4-méthoxycarbonylaminophénylcarbamoyl)-méthyl]-1-méthyl-3H-imidazol-1-ium ;
   - le chlorure de 3-[(2-hydroxy-3-acétylamino-phénylcarbamoyl)-méthyl]-1-méthyl-3H-imidazol-1-ium ;
   - le chlorure de 1-[(2-hydroxy-phénylcarbamoyl)-méthyl]-pyridinium ;
   - le dichlorure de 1-[(2-hydroxy-3-(2-(pyridinium-1-yl)acétylamino)-phénylcarbamoyl)-méthyl]-pyridinium ;
   - le dichlorure de 1-[(2-hydroxy-4-(2-(pyridinium-1-yl)acétylamino)-phénylcarbamoyl)-méthyl]-pyridinium ;
   - le chlorure de 1-[(2-hydroxy-4-méthyl-phénylcarbamoyl)-méthyl]-pyridinium ;
   - le chlorure de 1-[(2-hydroxy-4-amino-phénylcarbamoyl)-méthyl]-pyridinium ;
   - le chlorure de 1-[(2-hydroxy-4-acétylamino-phénylcarbamoyl)-méthyl]-pyridinium ;
   - le chlorure de 1-[(2-hydroxy-4-méthoxycarbonylamino-phénylcarbamoyl)-méthyl]-pyridinium ;
   - le chlorure de 1-[(3-hydroxy-4-acétylamino-phénylcarbamoyl)-méthyl]-pyridinium ;
   - le chlorure de 1-[(3-hydroxy-4-méthoxycarbonylamino-phénylcarbamoyl)-méthyl]-pyridinium ;
   - le chlorure de 1-[(2-hydroxy-5-chloro-phénylcarbamoyl)-méthyl]-pyridinium ;
   - le chlorure de 1-[(2-hydroxy-4-méthyl-5-chloro-phénylcarbamoyl)-méthyl]-pyridinium ;
   - le chlorure de 1-[(2-hydroxy-4-amino-5-chloro-phénylcarbamoyl)-méthyl]-pyridinium ;
   - le chlorure de 1-[(2-hydroxy-4-acétylamino-5-chloro-phénylcarbamoyl)-méthyl]-pyridinium ;
   - le chlorure de 1-[(2-hydroxy-4-méthoxycarbonylamino-5-chlorophénylcarbamoyl)-méthyl]-pyridinium ;
   - le chlorure de 1-[(3-hydroxy-4-acétylamino-6-chloro-phénylcarbamoyl)-méthyl]-pyridinium ;
   - le chlorure de 1-[(3-hydroxy-4-méthoxycarbonylamino-6-chlorophénylcarbamoyl)-méthyl]-pyridinium ;
   - le chlorure de 1-[(2-hydroxy-5-méthoxy-phénylcarbamoyl)-méthyl]-pyridinium ;
   - le chlorure de 1-[(2-hydroxy-4-méthyl-5-méthoxy-phénylcarbamoyl)-méthyl]-pyridinium ;
   - le chlorure de 1-[(2-hydroxy-4-amino-5-méthoxy-phénylcarbamoyl)-méthyl]-pyridinium ;
   - le chlorure de 1-[(2-hydroxy-4-acétylamino-5-méthoxy-phénylcarbamoyl)-méthyl]-pyridinium ;
   - le chlorure de 1-[(2-hydroxy-4-méthoxycarbonylamino-5-méthoxy-phénylcarbamoyl)-méthyl]-pyridinium ;
   - le chlorure de 1-[(3-hydroxy-4-acétylamino-6-méthoxy-phénylcarbamoyl)-méthyl]-pyridinium ;
   - le chlorure de 1-[(3-hydroxy-4-méthoxycarbonylamino-6-méthoxyphénylcarbamoyl)-méthyl]-pyridinium ;
   - le chlorure de 1-[(2-hydroxy-6-amino-phénylcarbamoyl)-méthyl]-pyridinium ;
   - le chlorure de 1-[(2-hydroxy-6-acétylamino-phénylcarbamoyl)-méthyl]-pyridinium ;
   - le chlorure de 1-[(2-hydroxy-4,6-diamino-phénylcarbamoyl)-méthyl]-pyridinium ;
   - le chlorure de 1-[(2-hydroxy-4-acétylamino-6-amino-phénylcarbamoyl)-méthyl]-pyridinium ;
   - le chlorure de 1-[(2-hydroxy-3,5-dichloro-4-méthyl-phénylcarbamoyl)-méthyl]-pyridinium ;
   - le chlorure de 1-[(2-hydroxy-3,5-dichloro-4-amino-phénylcarbamoyl)-méthyl]-pyridinium ;
   - le chlorure de 1-[(2-hydroxy-3,5-dichloro-4-acétylamino-phénylcarbamoyl)-méthyl]-pyridinium ;
   - le chlorure de 1-[(2-hydroxy-3,5-dichloro-4-méthoxycarbonylamino-phénylcarbamoyl)-méthyl]-pyridinium ;
   - le chlorure de 1-[(2-hydroxy-3-acétylamino-phénylcarbamoyl)-méthyl]-pyridinium ;
   - le chlorure de 1-[(2-hydroxy-phénylcarbamoyl)-méthyl]-1,4-diméthyl-pipérazin-1-ium ;
   - le dichlorure de 1-[(2-hydroxy-3-(2-(1,4-diméthyl-pipérazin-1-ium-1-yl)-acétyl)amino-phénylcarbamoyl)-méthyl]-1,4-diméthyl-pipérazin-1-ium ;
   - le dichlorure de 1-[(2-hydroxy-4-(2-(1,4-diméthyl-pipérazin-1-ium-1-yl) acétyl)amino-phényl-carbamoyl)-méthyl]-1,4-diméthyl-pipérazin-1-ium ;
   - le chlorure de 1-[(2-hydroxy-4-méthyl-phénylcarbamoyl)-méthyl]-1,4-diméthyl-pipérazin-1-ium ;
   - le chlorure de 1-[(2-hydroxy-4-amino-phénylcarbamoyl)-méthyl]-1,4-diméthyl-pipérazin-1-ium ;
   - le chlorure de 1-[(2-hydroxy-4-acétylamino-phénylcarbamoyl)-méthyl]-1,4-diméthyl-pipérazin-1-ium ;
   - le chlorure de 1-[(2-hydroxy-4-méthoxycarbonylamino-phénylcarbamoyl)-méthyl]-1,4-diméthyl-pipérazin-1-ium ;
   - le chlorure de 1-[(3-hydroxy-4-acétylamino-phénylcarbamoyl)-méthyl]-1,4-diméthyl-pipérazin-1-ium ;
   - le chlorure de 1-[(3-hydroxy-4-méthoxycarbonylamino-phénylcarbamoyl)-méthyl]-1,4-diméthyl-pipérazin-1-ium ;
   - le chlorure de 1-[(2-hydroxy-5-chloro-phénylcarbamoyl)-méthyl]-1,4-diméthyl-pipérazin-1-ium ;
   - le chlorure de 1-[(2-hydroxy-4-méthyl-5-chloro-phénylcarbamoyl)-méthyl]-1,4-diméthyl-pipérazin-1-ium ;
   - le chlorure de 1-[(2-hydroxy-4-amino-5-chloro-phénylcarbamoyl)-méthyl]-1,4-diméthyl-pipérazin-1-ium ;
   - le chlorure de 1-[(2-hydroxy-4-acétylamino-5-chloro-phénylcarbamoyl)-méthyl]-1,4-diméthyl-pipérazin-1-ium ;
   - le chlorure de 1-[(2-hydroxy-4-méthoxycarbonylamino-5-chloro-phénylcarbamoyl)-méthyl]-1,4-diméthyl-pipérazin-1-ium ;
   - le chlorure de 1-[(3-hydroxy-4-acétylamino-6-chloro-phénylcarbamoyl)-méthyl]-1,4-diméthyl-pipérazin-1-ium ;
   - le chlorure de 1-[(3-hydroxy-4-méthoxycarbonylamino-6-chloro-phénylcarbamoyl)-méthyl]-1,4-diméthyl-pipérazin-1-ium ;
   - le chlorure de 1-[(2-hydroxy-5-méthoxy-phénylcarbamoyl)-méthyl]-1,4-diméthyl-pipérazin-1-ium ;
   - le chlorure de 1-[(2-hydroxy-4-méthyl-5-méthoxy-phénylcarbamoyl)-méthyl]-1,4-diméthyl-pipérazin-1-ium ;
   - le chlorure de 1-[(2-hydroxy-4-amino-5-méthoxy-phénylcarbamoyl)-méthyl]-1,4-diméthyl-pipérazin-1-ium ;
   - le chlorure de 1-[(2-hydroxy-4-acétylamino-5-méthoxy-phénylcarbamoyl)-méthyl]-1,4-diméthyl-pipérazin-1-ium ;
   - le chlorure de 1-[(2-hydroxy-4-méthoxycarbonylamino-5-méthoxy-phénylcarbamoyl)-méthyl]-1,4-diméthyl-pipérazin-1-ium ;
   - le chlorure de 1-[(3-hydroxy-4-acétylamino-6-méthoxy-phénylcarbamoyl)-méthyl]-1,4-diméthyl-pipérazin-1-ium ;
   - le chlorure de 1-[(3-hydroxy-4-méthoxycarbonylamino-6-méthoxy-phénylcarbamoyl)-méthyl]-1,4-diméthyl-pipérazin-1-ium ;
   - le chlorure de 1-[(2-hydroxy-6-amino-phénylcarbamoyl)-méthyl]-1,4-diméthyl-pipérazin-1-ium ;
   - le chlorure de 1-[(2-hydroxy-6-acétylamino-phénylcarbamoyl)-méthyl]-1,4-diméthyl-pipérazin-1-ium ;
   - le chlorure de 1-[(2-hydroxy-4,6-diamino-phénylcarbamoyl)-méthyl]-1,4-diméthyl-pipérazin-1-ium ;
   - le chlorure de 1-[(2-hdroxy-4-acétylamino-6-amino-phénylcarbamoyl)-méthyl]-1,4-diméthyl-pipérazin-1-ium;
   - le chlorure de 1-[(2-hydroxy-3,5-dichloro-4-méthyl-phénylcarbamoyl)-méthyl]-1,4-diméthyl-pipérazin-1-ium ;
   - le chlorure de 1-[(2-hydroxy-3,5-dichloro-4-amino-phénylcarbamoyl)-méthyl]-1,4-diméthyl-pipérazin-1-ium ;
   - le chlorure de 1-[(2-hydroxy-3,5-dichloro-4-acétylamino-phénylcarbamoyl)-méthyl]-1,4-diméthyl-pipérazin-1-ium ;
   - le chlorure de 1-[(2-hydroxy-3,5-dichloro-4-méthoxycarbonylamino-phénylcarbamoyl)-méthyl]-1,4-diméthyl-pipérazin-1-ium ;
   - le chlorure de 1-[(2-hydroxy-3-acétylamino-phénylcarbamoyl)-méthyl]-1,4-diméthyl-pipérazin-1-ium ;
et leurs sels d'addition avec un acide.

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale conforme à l'invention n'est pas critique. Elles sont de préférence choisies parmi les bases d'oxydation classiquement utilisées en teinture d'oxydation et parmi lesquelles on peut notamment citer les paraphénylènediamines, les bis-phénylalkylènediamines, les paraaminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.
Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs formes tautomères, lorsqu'il existe un équilibre tautomérique, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon l'invention, les compositions tinctoriales renfermant une ou plusieurs paraphénylènediamines et/ou une ou plusieurs bases d'oxydation hétérocycliques sont particulièrement préférées.

La ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 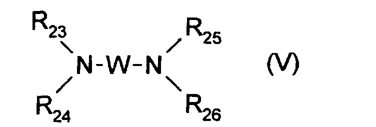 dans laquelle W est un reste propylène substitué ou non substitué par un groupement hydroxyle ou un radical alkyle en C₁-C₆ ; R₂₃, R₂₄, R₂₅ et R₂₆, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.
L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Certains composés de formule (I) sont nouveaux en soi et constituent à ce titre un autre objet de l'invention. Ces nouveaux composés, ainsi que leurs sels d'addition avec un acide, répondent à la formule (I)' suivante : 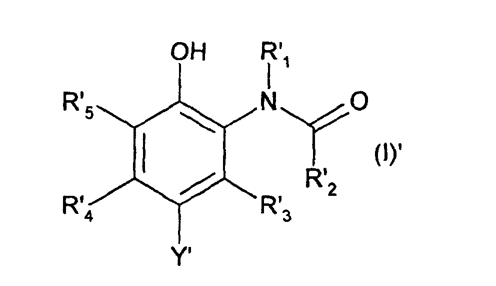 dans laquelle R'₁, R'₂, R'₃, R'₄, R'₅ et Y peuvent prendre les mêmes significations que celles indiquées ci-dessus pour R₁, R₂, R₃, R₄, R₅ et Y, dans lesquels au moins un des groupements R'₁ à R'₅ représente un groupement Z' pouvant prendre les mêmes significations que celles indiquées ci-dessus pour le groupement Z ; à l'exclusion des composés suivants : 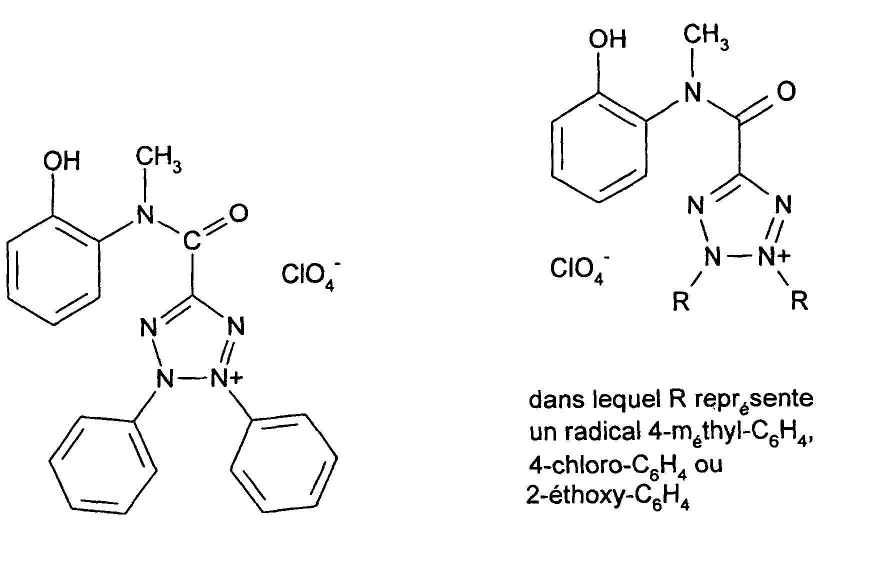 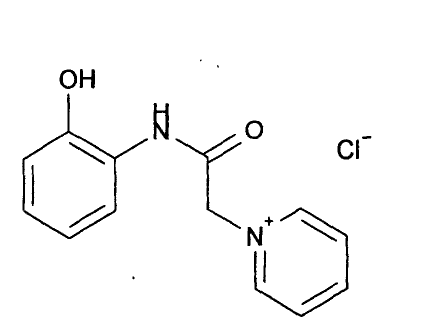 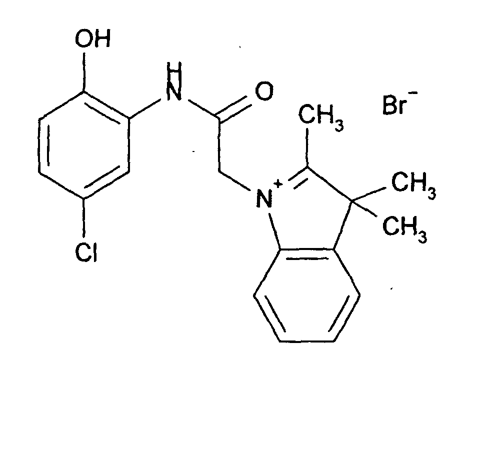 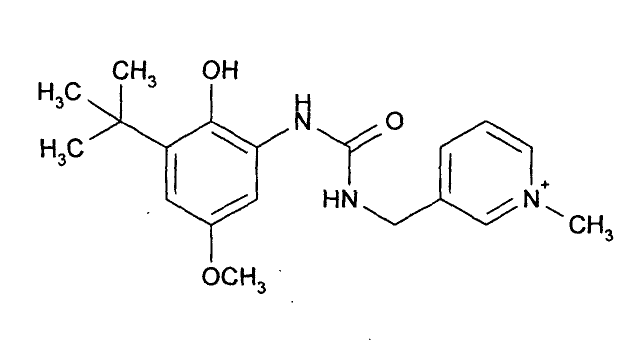 et 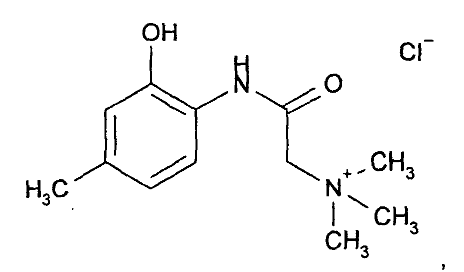 qui sont connus dans le domaine photographique ou médical, voir notamment les documents Bull. Soc. Chim. Fr. (1966), 400-3 ; Bull. Soc. Chim. Fr. (1969), 4390-4 ; Zh. Org. Khim. (1972), 8, 2429-31 ; Des. Monomers Polym. (1998), 1, 15-36 ; et les demandes de brevet EP 0 303 301 ; JP 07 271 075 ; et EP 0 790 240.

Parmi les composés de formule (I') conformes à l'invention, on peut en particulier citer :
- le chlorure de 3-[(2-hydroxy-phénylcarbamoyl)-méthyl]-1-méthyl-3H-imidazol-1-ium ;
- le dichlorure de 3-[(2-hydroxy-3-(2-(3-méthyl-1H-imidazol-3-ium-1-yl)-acétylamino)-phénylcarbamoyl)-méthyl]-1-méthyl-3H-imidazol-1-ium ;
- le dichlorure de 3-[(2-hydroxy-4-(2-(3-méthyl-1H-imidazol-3-ium-1-yl)-acétylamino)-phénylcarbamoyl)-méthyl]-1-méthyl-3H-imidazol-1-ium ;
- le chlorure de 3-[(2-hydroxy-4-méthyl-phénylcarbamoyl)-méthyl]-1-méthyl-3H-imidazol-1-ium ;
- le chlorure de 3-[(2-hydroxy-4-amino-phénylcarbamoyl)-méthyl]-1-méthyl-3H-imidazol-1-ium ;
- le chlorure de 3-[(2-hydroxy-4-acétylamino-phénylcarbamoyl)-méthyl]-1-méthyl-3H-imidazol-1-ium ;
- le chlorure de 3-[(2-hydroxy-4-méthoxycarbonylamino-phénylcarbamoyl)-méthyl]-1-méthyl-3H-imidazol-1-ium ;
- le chlorure de 3-[(3-hydroxy-4-acétylamino-phénylcarbamoyl)-méthyl]-1-méthyl-3H-imidazol-1-ium ;
- le chlorure de 3-[(3-hydroxy-4-méthoxycarbonylamino-phénylcarbamoyl)-méthyl]-1-méthyl-3H-imidazol-1-ium ;
- le chlorure de 3-[(2-hydroxy-5-chloro-phénylcarbamoyl)-méthyl]-1-méthyl-3H-imidazol-1-ium ;
- le chlorure de 3-[(2-hydroxy-4-méthyl-5-chloro-phénylcarbamoyl)-méthyl]-1-méthyl-3H-imidazol-1-ium ;
- le chlorure de 3-[(2-hydroxy-4-amino-5-chloro-phénylcarbamoyl)-méthyl]-1-méthyl-3H-imidazol-1-ium ;
- le chlorure de 3-[(2-hydroxy-4-acétylamino-5-chloro-phénylcarbamoyl)-méthyl]-1-méthyl-3H-imidazol-1-ium ;
- le chlorure de 3-[(2-hydroxy-4-méthoxycarbonylamino-5-chloro-phényl-carbamoyl)-méthyl]-1-méthyl-3H-imidazol-1-ium ;
- le chlorure de 3-[(3-hydroxy-4-acétylamino-5-chloro-phénylcarbamoyl)-méthyl]-1-méthyl-3H-imidazol-1-ium ;
- le chlorure de 3-[(3-hydroxy-4-méthoxycarbonylamino-6-chloro-phénylcarbamoyl)-méthyl]-1-méthyl-3H-imidazol-1-ium ;
- le chlorure de 3-[(2-hydroxy-5-méthoxy-phénylcarbamoyl)-méthyl]-1-méthyl-3H-imidazol-1-ium ;
- le chlorure de 3-[(2-hydroxy-4-méthyl-5-méthoxy-phénylcarbamoyl)-méthyl]-1-méthyl-3H-imidazol-1-ium ;
- le chlorure de 3-[(2-hydroxy-4-amino-5-méthoxy-phénylcarbamoyl)-méthyl]-1-méthyl-3H-imidazol-1-ium ;
- le chlorure de 3-[(2-hydroxy-4-acétylamino-5-méthoxy-phénylcarbamoyl)-méthyl]-1-méthyl-3H-imidazol-1-ium ;
- le chlorure de 3-[(2-hydroxy-4-méthoxycarbonylamino-5-méthoxy-phénylcarbamoyl)-méthyl]-1-méthyl-3H-imidazol-1-ium ;
- le chlorure de 3-[(3-hydroxy-4-acétylamino-6-méthoxy-phénylcarbamoyl)-méthyl]-1-méthyl-3H-imidazol-1-ium ;
- le chlorure de 3-[(3-hydroxy-4-méthoxycarbonylamino-6-méthoxyphénylcarbamoyl)-méthyl]-1-méthyl-3H-imidazol-1-ium ;
- le chlorure de 3-[(2-hydroxy-6-amino-phénylcarbamoyl)-méthyl]-1-méthyl-3H-imidazol-1-ium ;
- le chlorure de 3-[(2-hydroxy-6-acétylamino-phénylcarbamoyl)-méthyl]-1-méthyl-3H-imidazol-1-ium ;
- le chlorure de 3-[(2-hydroxy-4,6-diamino-phénylcarbamoyl)-méthyl]-1-méthyl-3H-imidazol-1-ium ;
- le chlorure de 3-[(2-hydroxy-4-acétylamino-6-amino-phénylcarbamoyl)-méthyl]-1-méthyl-3H-imidazol-1-ium ;
- le chlorure de 3-[(2-hydroxy-3,5-dichloro-4-méthyl-phénylcarbamoyl)-méthyl]-1-méthyl-3H-imidazol-1-ium ;
- le chlorure de 3-[(2-hydroxy-3,5-dichloro-4-amino-phénylcarbamoyl)-méthyl]-1-méthyl-3H-imidazol-1-ium ;
- le chlorure de 3-[(2-hydroxy-3,5-dichloro-4-acétylamino-phénylcarbamoyl)-méthyl]-1-méthyl-3H-imidazol-1-ium ;
- le chlorure de 3-[(2-hydroxy-3,5-dichloro-4-méthoxycarbonylaminophénylcarbamoyl)-méthyl]-1-méthyl-3H-imidazol-1-ium ;
- le chlorure de 3-[(2-hydroxy-3-acétylamino-phénylcarbamoyl)-méthyl]-1-méthyl-3H-imidazol-1-ium ;
- le chlorure de 1-[(2-hydroxy-phénylcarbamoyl)-méthyl]-pyridinium ;
- le dichlorure de 1-[(2-hydroxy-3-(2-(pyridinium-1-yl)acétylamino)-phénylcarbamoyl)-méthyl]-pyridinium ;
- le dichlorure de 1-[(2-hydroxy-4-(2-(pyridinium-1-yl)acétylamino)-phénylcarbamoyl)-méthyl]-pyridinium ;
- le chlorure de 1-[(2-hydroxy-4-méthyl-phénylcarbamoyl)-méthyl]-pyridinium ;
- le chlorure de 1-[(2-hydroxy-4-amino-phénylcarbamoyl)-méthyl]-pyridinium ;
- le chlorure de 1-[(2-hydroxy-4-acétylamino-phénylcarbamoyl)-méthyl]-pyridinium ;
- le chlorure de 1-[(2-hydroxy-4-méthoxycarbonylamino-phénylcarbamoyl)-méthyl]-pyridinium ;
- le chlorure de 1-[(3-hydroxy-4-acétylamino-phénylcarbamoyl)-méthyl]-pyridinium ;
- le chlorure de 1-[(3-hydroxy-4-méthoxycarbonylamino-phénylcarbamoyl)-méthyl]-pyridinium ;
- le chlorure de 1-[(2-hydroxy-5-chloro-phénylcarbamoyl)-méthyl]-pyridinium ;
- le chlorure de 1-[(2-hydroxy-4-méthyl-5-chloro-phénylcarbamoyl)-méthyl]-pyridinium ;
- le chlorure de 1-[(2-hydroxy-4-amino-5-chloro-phénylcarbamoyl)-méthyl]-pyridinium ;
- le chlorure de 1-[(2-hydroxy-4-acétylamino-5-chloro-phénylcarbamoyl)-méthyl]-pyridinium ;
- le chlorure de 1-[(2-hydroxy-4-méthoxycarbonylamino-5-chlorophénylcarbamoyl)-méthyl]-pyridinium ;
- le chlorure de 1-[(3-hydroxy-4-acétylamino-6-chloro-phénylcarbamoyl)-méthyl]-pyridinium ;
- le chlorure de 1-[(3-hydroxy-4-méthoxycarbonylamino-6-chlorophénylcarbamoyl)-méthyl]-pyridinium ;
- le chlorure de 1-[(2-hydroxy-5-méthoxy-phénylcarbamoyl)-méthyl]-pyridinium ;
- le chlorure de 1-[(2-hydroxy-4-méthyl-5-méthoxy-phénylcarbamoyl)-méthyl]-pyridinium ;
- le chlorure de 1-[(2-hydroxy-4-amino-5-méthoxy-phénylcarbamoyl)-méthyl]-pyridinium ;
- le chlorure de 1-[(2-hydroxy-4-acétylamino-5-méthoxy-phénylcarbamoyl)-méthyl]-pyridinium ;
- le chlorure de 1-[(2-hydroxy-4-méthoxycarbonylamino-5-méthoxy-phénylcarbamoyl)-méthyl]-pyridinium ;
- le chlorure de 1-[(3-hydroxy-4-acétylamino-6-méthoxy-phénylcarbamoyl)-méthyl]-pyridinium ;
- le chlorure de 1-[(3-hydroxy-4-méthoxycarbonylamino-6-méthoxyphénylcarbamoyl)-méthyl]-pyridinium ;
- le chlorure de 1-[(2-hydroxy-6-amino-phénylcarbamoyl)-méthyl]-pyridinium ;
- le chlorure de 1-[(2-hydroxy-6-acétylamino-phénylcarbamoyl)-méthyl]-pyridinium ;
- le chlorure de 1-[(2-hydroxy-4,6-diamino-phénylcarbamoyl)-méthyl]-pyridinium ;
- le chlorure de 1-[(2-hydroxy-4-acétylamino-6-amino-phénylcarbamoyl)-méthyl]-pyridinium ;
- le chlorure de 1-[(2-hydroxy-3,5-dichloro-4-méthyl-phénylcarbamoyl)-méthyl]-pyridinium ;
- le chlorure de 1-[(2-hydroxy-3,5-dichloro-4-amino-phénylcarbamoyl)-méthyl]-pyridinium ;
- le chlorure de 1-[(2-hydroxy-3,5-dichloro-4-acétylamino-phénylcarbamoyl)-méthyl]-pyridinium ;
- le chlorure de 1-[(2-hydroxy-3,5-dichloro-4-méthoxycarbonylamino-phényl-carbamoyl)-méthyl]-pyridinium ;
- le chlorure de 1-[(2-hydroxy-3-acétylamino-phénylcarbamoyl)-méthyl]-pyridinium ;
- le chlorure de 1-[(2-hydroxy-phénylcarbamoyl)-méthyl]-1,4-diméthyl-pipérazin-1-ium ;
- le dichlorure de 1-[(2-hydroxy-3-(2-(1,4-diméthyl-pipérazin-1-ium-1-yl)-acétyl)amino-phénylcarbamoyl)-méthyl]-1,4-diméthyl-pipérazin-1-ium ;
- le dichlorure de 1-[(2-hydroxy-4-(2-(1,4-diméthyl-pipérazin-1-ium-1-yl) acétyl)amino-phényl-carbamoyl)-méthyl]-1,4-diméthyl-pipérazin-1-ium ;
- le chlorure de 1-[(2-hydroxy-4-méthyl-phénylcarbamoyl)-méthyl]-1,4-diméthyl-pipérazin-1-ium ;
- le chlorure de 1-[(2-hydroxy-4-amino-phénylcarbamoyl)-méthyl]-1,4-diméthyl-pipérazin-1-ium ;
- le chlorure de 1-[(2-hydroxy-4-acétylamino-phénylcarbamoyl)-méthyl]-1,4-diméthyl-pipérazin-1-ium ;
- le chlorure de 1-[(2-hydroxy-4-méthoxycarbonylamino-phénylcarbamoyl)-méthyl]-1,4-diméthyl-pipérazin-1-ium ;
- le chlorure de 1-[(3-hydroxy-4-acétylamino-phénylcarbamoyl)-méthyl]-1,4-diméthyl-pipérazin-1-ium ;
- le chlorure de 1-[(3-hydroxy-4-méthoxycarbonylamino-phénylcarbamoyl)-méthyl]-1,4-diméthyl-pipérazin-1-ium ;
- le chlorure de 1-[(2-hydroxy-5-chloro-phénylcarbamoyl)-méthyl]-1,4-diméthyl-pipérazin-1-ium ;
- le chlorure de 1-[(2-hydroxy-4-méthyl-5-chloro-phénylcarbamoyl)-méthyl]-1,4-diméthyl-pipérazin-1-ium ;
- le chlorure de 1-[(2-hydroxy-4-amino-5-chloro-phénylcarbamoyl)-méthyl]-1,4-diméthyl-pipérazin-1-ium ;
- le chlorure de 1-[(2-hydroxy-4-acétylamino-5-chloro-phénylcarbamoyl)-méthyl]-1,4-diméthyl-pipérazin-1-ium ;
- le chlorure de 1-[(2-hydroxy-4-méthoxycarbonylamino-5-chloro-phénylcarbamoyl)-méthyl]-1,4-diméthyl-pipérazin-1-ium ;
- le chlorure de 1-[(3-hydroxy-4-acétylamino-6-chloro-phénylcarbamoyl)-méthyl]-1,4-diméthyl-pipérazin-1-ium ;
- le chlorure de 1-[(3-hydroxy-4-méthoxycarbonylamino-6-chloro-phénylcarbamoyl)-méthyl]-1,4-diméthyl-pipérazin-1-ium ;
- le chlorure de 1-[(2-hydroxy-5-méthoxy-phénylcarbamoyl)-méthyl]-1,4-diméthyl-pipérazin-1-ium ;
- le chlorure de 1-[(2-hydroxy-4-méthyl-5-méthoxy-phénylcarbamoyl)-méthyl]-1,4-diméthyl-pipérazin-1-ium ;
- le chlorure de 1-[(2-hydroxy-4-amino-5-méthoxy-phénylcarbamoyl)-méthyl]-1,4-diméthyl-pipérazin-1-ium ;
- le chlorure de 1-[(2-hydroxy-4-acétylamino-5-méthoxy-phénylcarbamoyl)-méthyl]-1,4-diméthyl-pipérazin-1-ium ;
- le chlorure de 1-[(2-hydroxy-4-méthoxycarbonylamino-5-méthoxy-phénylcarbamoyl)-méthyl]-1,4-diméthyl-pipérazin-1-ium ;
- le chlorure de 1-[(3-hydroxy-4-acétylamino-6-méthoxy-phénylcarbamoyl)-méthyl]-1,4-diméthyl-pipérazin-1-ium ;
- le chlorure de 1-[(3-hydroxy-4-méthoxycarbonylamino-6-méthoxy-phénylcarbamoyl)-méthyl]-1,4-diméthyl-pipérazin-1-ium ;
- le chlorure de 1-[(2-hydroxy-6-amino-phénylcarbamoyl)-méthyl]-1,4-diméthyl-pipérazin-1-ium ;
- le chlorure de 1-[(2-hydroxy-6-acétylamino-phénylcarbamoyl)-méthyl]-1,4-diméthyl-pipérazin-1-ium ;
- le chlorure de 1-[(2-hydroxy-4,6-diamino-phénylcarbamoyl)-méthyl]-1,4-diméthyl-pipérazin-1-ium ;
- le chlorure de 1-[(2-hydroxy-4-acétylamino-6-amino-phénylcarbamoyl)-méthyl]-1,4-diméthyl-pipérazin-1-ium;
- le chlorure de 1-[(2-hydroxy-3,5-dichloro-4-méthyl-phénylcarbamoyl)-méthyl]-1,4-diméthyl-pipérazin-1-ium ;
- le chlorure de 1-[(2-hydroxy-3,5-dichloro-4-amino-phénylcarbamoyl)-méthyl]-1,4-diméthyl-pipérazin-1-ium ;
- le chlorure de 1-[(2-hydroxy-3,5-dichloro-4-acétylamino-phénylcarbamoyl)-méthyl]-1,4-diméthyl-pipérazin-1-ium ;
- le chlorure de 1-[(2-hydroxy-3,5-dichloro-4-méthoxycarbonylamino-phénylcarbamoyl)-méthyl]-1,4-diméthyl-pipérazin-1-ium ;
- le chlorure de 1-[(2-hydroxy-3-acétylamino-phénylcarbamoyl)-méthyl]-1,4-diméthyl-pipérazin-1-ium ; et leurs sels d'addition avec un acide.

Les sels d'addition avec un acide des composés de formule (I') peuvent notamment être choisis parmi les chlorhydrates, les bromhydrates, les sulfates, les citrates, les succinates, les tartrates, les lactates et les acétates.

Ces nouveaux composés de formule (I') peuvent être préparés selon des méthodes bien connues de l'état de la technique et décrites par exemple dans les demandes de brevet ou brevets FR-A-1 596 879 ; BE 816 674 ; EP 0 579 204 ; DE 2 846 931 ; JP-54-115 230 ; GB 2 070 000 ; DE 3 027 128 ; EP 0 065 874 ; EP0 115 194 ; EP 0 079 141 ; EP 0 081 321 ; DE 3 246 238 ; EP 0 168 729 ; DE 3 414 051 ; JP-59-059656 ; FR-A-2 575 470; EP 0 193 051 ; JP-63-208562 ; JP-62-173469 ; JP-62-108859 ; JP-62-173469 ; DD253997 ; DE 3 641 825 , JP-63-208562 ; DE 3 621 215 ; JP-01-249739 ; JP-64-002045 ; JP-02-255674 ; JP-01-032261 ; JP-02-255674 ; EP 0 608 896 , WO 94/19316 ; JP-09-169705 ; EP 0 790 240 ; ainsi que dans les documents Res. Discl. (1981), 202, 76-8 ; Synthesis (1982), 940-2 ; Res. Discl. (1983), 235, 352-3 ; Res. Discl. (1984), 247, 554-6 ; Res. Discl. (1985), 251, 134-9 ; Chem. Ind. (Dekker) (1992), 47 (Catal. Org. React.), 147-51 ; et J. Med. Chem. (1998), 41, 4062-79.

L'invention a enfin pour objet l'utilisation des composés de formule (I') à titre de coupleur pour la teinture d'oxydation des fibres kératiniques et en particulier des fibres kératiniques humaines telles que le cheveux.

Les exemples qui suivent sont destinés à illustrer l'invention sans pour autant en limiter la portée.

### EXEMPLES DE PREPARATION

### EXEMPLE DE PREPARATION 1 : Synthèse du chlorure de 3-[(3,5-dichloro-2-hydroxy-4-méthyl-phénylcarbamoyl)-méthyl]-1-méthyl-3H-imidazol-1-ium

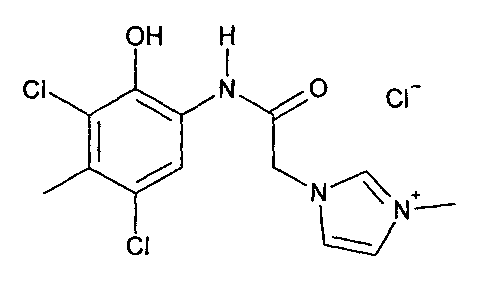

### Etape n°1 :

A une suspension de chlorhydrate de 6-amino-2,4-dichloro-3-méthylphénol (20 g, 87 mmole) dans 1 litre de tetrahydrofurane, sous agitation et sous atmosphère inerte, ont été ajoutés goutte à goutte 24,3 ml de triéthylamine (2 équivalents) ; après 2 heures, 30,7 ml de chlorure de chloroacétyle (1 équivalent) on été ajoutés en 15 minutes. La suspension a été filtrée sur verre fritté et les sels minéraux ont été rincés abondamment au tetrahydrofurane. Le mélange des solutions organiques a été concentré sous vide. Le résidu a été repris dans l'acétate d'éthyle, lavé à l'eau par 6 fois 100 ml, séché sur sulfate de sodium et concentré, pour donner 23 g d'une poudre brune. Cette poudre a été lavée plusieurs fois à l'éther et séchée sous vide pour donner 18,5 g de 2-chloro-N-(3,5-dichloro-2-hydroxy-4-méthyl-phényl)-acétamide dont le point de fusion était de 149°C, (rendement 78%).

### Etape n° 2 :

4,45 ml de N-méthylimidazole (56 mmole) ont été ajoutés dans une suspension de 5 g 2-chloro-N-(3,5-dichloro-2-hydroxy-4-méthyl-phényl)-acétamide (18,6 mmole), obtenu ci-dessus à l'étape précédente, dans 5 ml d'acétate d'éthyle, conduisant à une solution limpide. Après 3 heures et 30 minutes, l'insoluble qui s'était formé a été essoré et lavé abondamment à l'acétate d'éthyle. Le nouveau précipité qui s'était formé dans les filtrats a été essoré et lavé abondamment à l'acétate d'éthyle. Les poudres blanches ainsi obtenues ont été combinées, et séchées pour donner 5 g de chlorure de 3-[(3,5-dichloro-2-hydroxy-4-méthyl-phénylcarbamoyl)-méthyl]-1-méthyl-3H-imidazol-1-ium dont le point de était de 251°C, (rendement 77%).
L'analyse élémentaire calculée pour C₁₃H₁₄Cl₃N₃O₂ était la suivante :

| % | C | H | N | O |
|---|---|---|---|---|
| Calculée | 44.05 | 3.97 | 11.82 | 9.15 |
| Trouvée | 44.53 | 4.02 | 11.98 | 9.13 |

### EXEMPLE DE PREPARATION 2 : Synthèse du chlorure de 3-[(4-acétylamino-2hydroxy-phénylcarbamoyl)-méthyl]-1-méthyl-3H-imidazol-1-ium

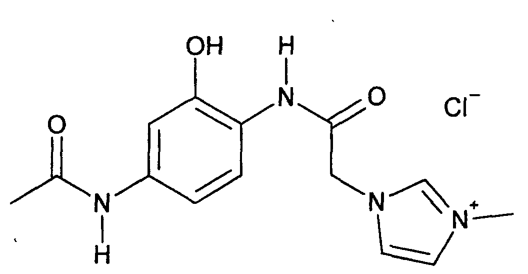

### Etape n° 1 :

A une suspension de chlorhydrate de 2-amino-5-(acétylamino)phénol (20 g, 98 mmole) dans 1 litre de tetrahydrofurane, sous agitation et sous atmosphère inerte, ont été ajoutés goutte à goutte 27,5 ml de triéthylamine (2 équivalents). Après 1 heure d'agitation, 8,26 ml de chlorure de chloroacétyle (1,05 équivalent) on été ajoutés en 15 minutes. La suspension a été filtrée sur verre fritté et les sels minéraux ont été rincés abondamment au tetrahydrofurane. Le mélange des solutions organiques a été concentré sous vide. Le résidu a été repris dans l'acétate d'éthyle, les insolubles ont été essorés et lavés à l'acétate d'éthyle pour donner 22 g d'une poudre brune. Cette poudre a été lavée plusieurs fois à l'éther et séchée sous vide pour donner 18,5 g de N-(4-acétylamino-2-hydroxy-phényl)-2-chloro-acétamide dont le point de fusion était de 218°C, (rendement 90%).

### Etape n° 2 :

9,85 ml de N-méthylimidazole (123 mmole) ont été ajoutés dans une suspension de 10 g (41 mmole) de N-(4-acétylamino-2-hydroxy-phényl)-2-chloro-acétamide obtenu ci-dessus à l'étape précédente dans 30ml d'acétate d'éthyle. Le milieu réactionnel a été chauffé au reflux pendant 6 heures, puis l'insoluble formé a été essoré et lavé abondamment à l'acétate d'éthyle. Le solide brun ainsi obtenu a été repris dans l'eau et filtré, puis le filtrat a été versé sur le dioxane. Le précipité obtenu a été essoré, lavé abondamment au dioxane puis à l'éther isopropylique et séché sous vide pour donner 10,6 g de chlorure de 3-[(4-acétylamino-2hydroxy-phénylcarbamoyl)-méthyl]-1-méthyl-3H-imidazol-1-ium sous forme d'une poudre marron-violet dont le point de fusion était de 225°C, (rendement 79%).

### EXEMPLES DE TEINTURE

### EXEMPLES 1 à 6 DE TEINTURE EN MILIEU ALCALIN

On a préparé les compositions tinctoriales suivantes (teneurs en moles) :

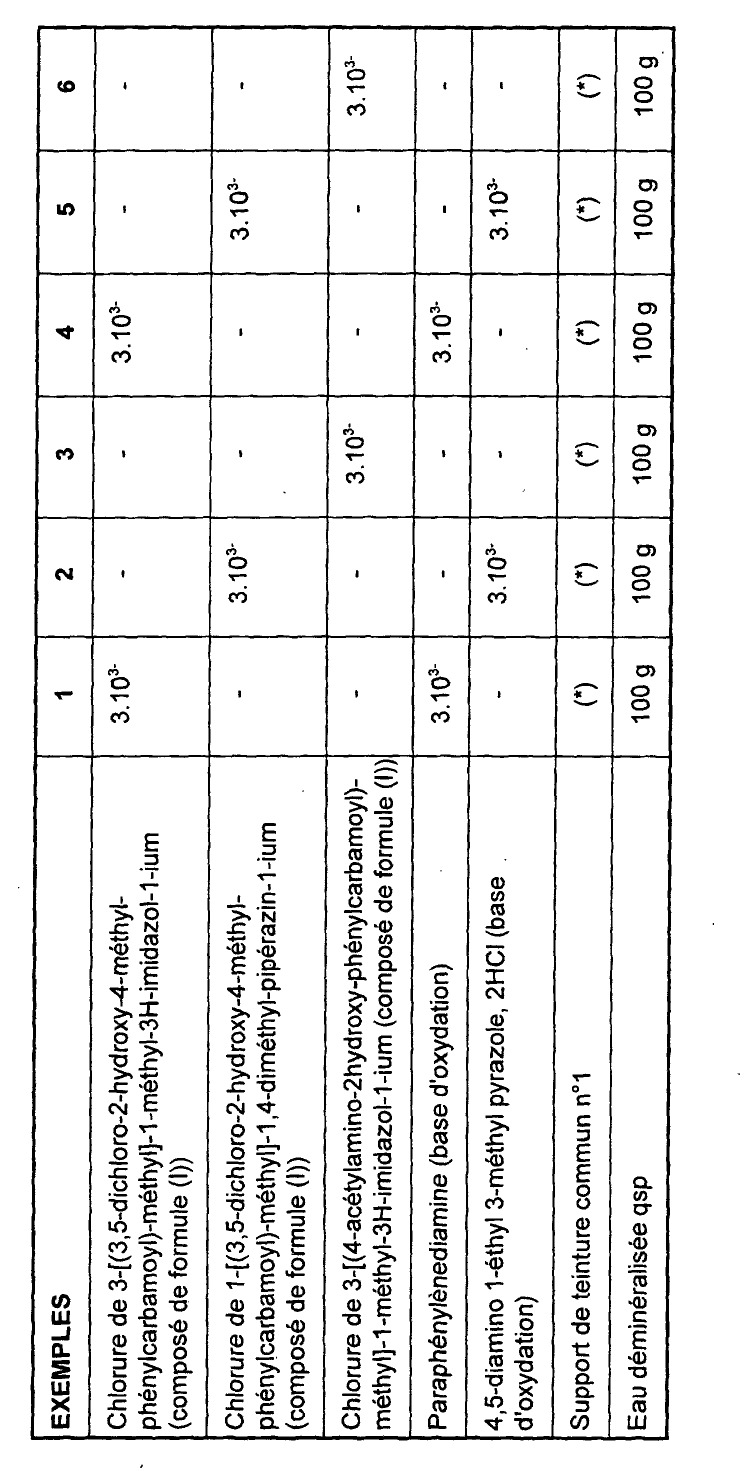

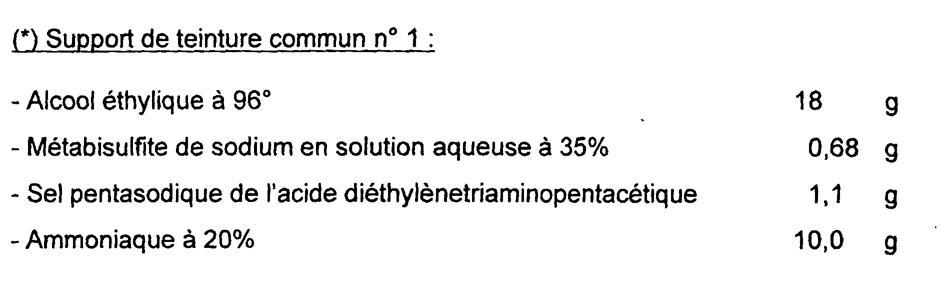

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris permanentés à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 ± 0,2 | Châtain cendré légèrement mat |
| **2** | 10 ± 0,2 | Châtain clair mat cendré |
| **3** | 10 ± 0,2 | Violet cendré légèrement bleuté |
| **4** | 10 ± 0,2 | Bleu intense |
| **5** | 10 ± 0,2 | Bleu cendré |
| **6** | 10 ± 0,2 | Châtain clair cendré violacé |

### EXEMPLES 7 à 12 DE TEINTURE EN MILIEU ALCALIN

On a préparé les compositions tinctoriales suivantes (teneurs en moles) :

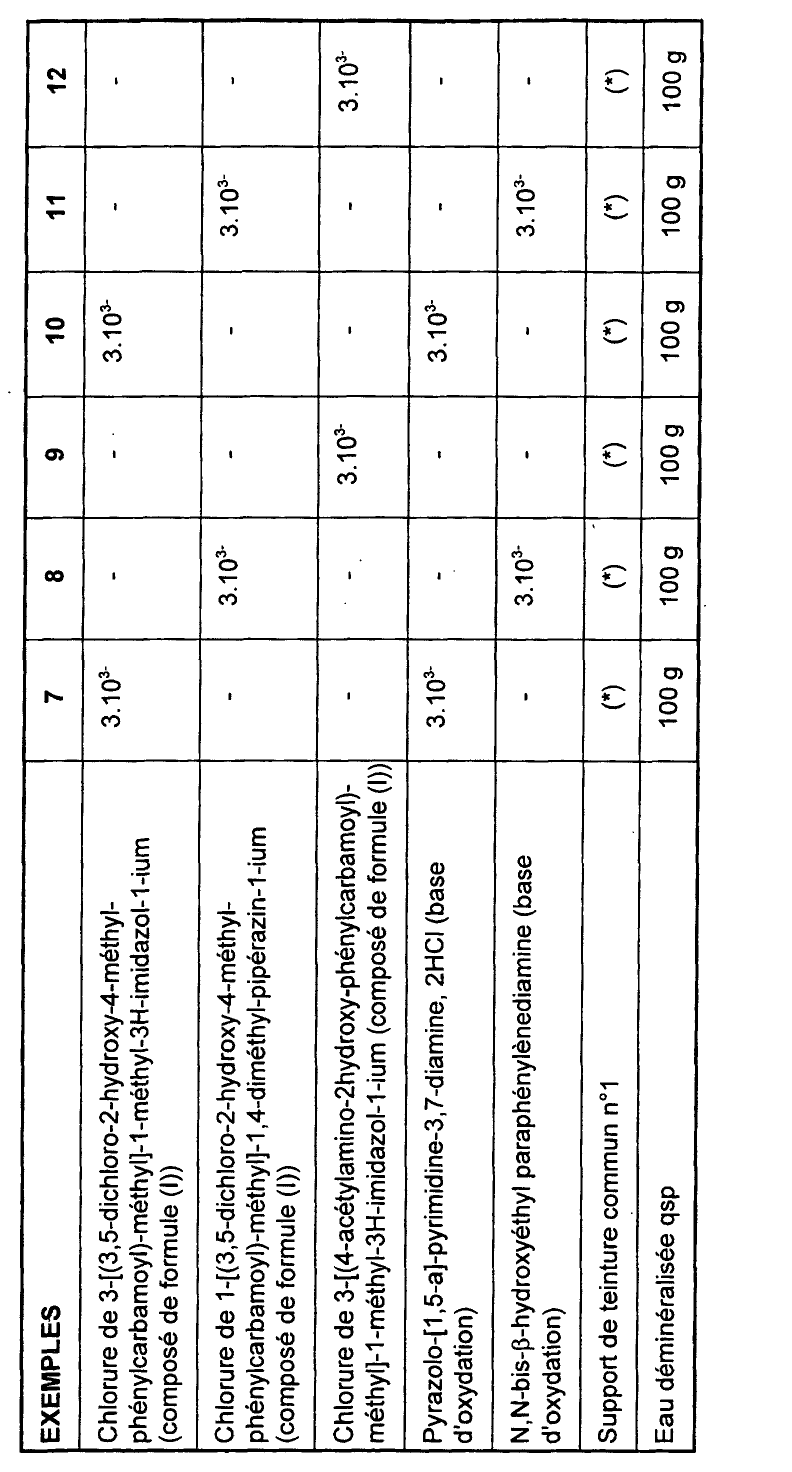

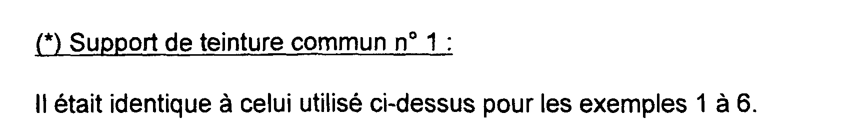

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris permanentés à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **7** | 10 ± 0,2 | Violacé irisé légèrement cendré |
| **8** | 10 ± 0,2 | Blond irisé violacé légèrement cendré |
| **9** | 10 ± 0,2 | Blond foncé irisé rouge |
| **10** | 10 ± 0,2 | Blond mat |
| **11** | 10 ± 0,2 | Blond clair mat |
| **12** | 10 ± 0,2 | Bleu |

## Revendications

1. Composition pour la teinture d'oxydation des fibres kératiniques, **caractérisée par le fait qu'**elle contient, dans un milieu approprié pour la teinture :
- au moins une base d'oxydation, et
- au moins un coupleur choisi parmi les composés de formule (I) suivante, et leurs sels d'addition avec un acide : 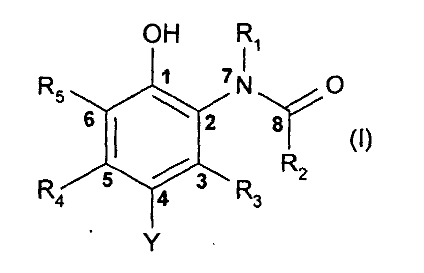
dans laquelle :
• R₁ représente un atome d'hydrogène ; un radical méthyle, éthyle, isopropyle, allyle, phényle, benzyle, fluorobenzyle, hydroxybenzyle, difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle, 2-naphtométhyl ; ou un groupement 2-hydroxyéthyle, 2-méthoxyéthyle ou 2-éthoxyéthyle;
• R₂ représente un atome d'hydrogène, un groupement Z ; un radical choisi dans le groupe **(G1)** constitué par un radical méthyle, éthyle, propyle, isopropyle, butyle, isobutyle, tert-butyle, pentyle, isopentyle, néopentyle, hexyle ; cyclopropyle, cyclobutyle, cyclopentyle, cyclopentylméthyle, 3-cyclopentyl-propyle, cyclohexyle, 2-cyclohexyl-éthyle, norbornane-2-yl, vinyle, 1-méthylvinyle, 2-méthylvinyle, 2,2-diméthylvinyle, allyle, 3-butényle ; phényle, méthylphényle, diméthylphényle, 2,4,6-triméthylphényle, 4-éthylphényle, (trifluorométhyl)phényle, hydroxyphényle, méthoxyphényle, éthoxyphényle, acétoxyphényle, (trifluorométhoxy)phényle, aminophényle, 4-diméthylamino-phényle, fluorophényle, difluorophényle, fluoro(trifluorométhyl)phényle, chlorophényle, dichlorophényle, bromophényle, napht-1-yle, napht-2-yle, (2-méthoxy)napht-1-yl, benzyle, 4'-méthoxybenzyle, 2',5'-diméthoxybenzyle, 3',4'-diméthoxybenzyle, 4'-fluorobenzyle, 4'-chlorobenzyle, phénéthyle, 2-phénylvinyle, (1-naphtyl)méthyle, (2-naphtyl)méthyle ; tétrahydrofuran-2-yl, furan-2-yl, 5-méthyl-2-(trifluorométhyl)furan-3-yl, 2-méthyl-5-phénylfuran-3-yl, thiophène-2-yl, (thiophène-2-yl)méthyle, 3-chlorothiophène-2-yl, 2,5-dichlorothiophène-3-yl, benzothiophène-2-yl, 3-chlorobenzothiophène-2-yl, isoxazole-5-yl, 5-méthylisoxazole-3-yl, 3,5-diméthylisoxazole-4-yl, 1,3-diméthylpyrazole-5-yl, 1-éthyl-3-méthylpyrazole-5-yl, 1-tertbutyl-3-méthylpyrazole-5-yl, 3-tertbutyl-1-méthylpyrazole-5-yl, 4-bromo-1-éthyl-3-méthylpyrazole-5-yl, indole-3-ylcarboxyl, pyridinyl, chloropyridinyl, dichloropyridinyl, 5-(bromo)pyridin-3-yl, pipérazin-2-yl, quinoxal-2-yl ; fluorométhyle, difluorométhyle, trifluorométhyle, 1,1,2,2-tétrafluoroéthyle, pentafluoroéthyle, heptafluoropropyle, 1,1,2,2,3,3,4,4-octafluorobutyle, nonafluorobutyle, chlorométhyle, chloroéthyle, 1,1-diméthyl-2-chloroéthyle, 1,2-dichloroéthyle, 1-chloropropyle, 3-chloropropyle,4-chlorobutyle, hydroxyméthyle, méthoxyméthyle, phénoxyméthyle, (4-chlorophénoxy)méthyle, benzyloxyméthyle, acétoxyméthyle, 1,2-dihydroxyéthyle, 1-phénoxyéthyle, 1-acétoxyéthyle, 2-(2-carboxyéthoxy)éthyle, 1-phénoxyéthyle, 1-acétoxyéthyle, méthoxycarbonyl, éthoxycarbonyl, (méthoxycarbonyl)méthyle, 2-carboxyéthyle, 2-(méthoxycarbonyl)éthyle, 2-carboxycylopropyle, 2-carboxycyclohexane; méthoxy, éthoxy, propoxy, isopropoxy, butoxy, isobutoxy, pentoxy, néopentoxy, hexyloxy, cyclopentyloxy, cyclohexyloxy, vinyloxy, allyloxy, propargyloxy, chlorométhoxy, 1-chloroéthoxy, 2-méthoxyéthoxy, 4-chlorobutoxy, phénoxy, 4-méthyphénoxy, 4-fluorophénoxy, 4-bromophénoxy, 4-chlorophénoxy, 4-méthoxyphénoxy, naphth-2-yloxy, benzyloxy; amino, méthylamino, éthylamino, propylamino, isopropylamino, butylamino, cyclohexylamino, allylamino, 2-chloroéthylamino, 3-chloropropylamino, carboxyméthylamino, phénylamino, fluorophénylamino, (trifluorométhyl)phénylamino, chlorophénylamino, bromophénylamino, 4-acétylphénylamino, méthoxyphénylamino, (trifluorométhoxy)phénylamino, naphth-1-ylamino, benzylamino, phénéthylamino, pyrid-3-ylamino, diméthylamino, et 1-pyrolidinyle, 4-morpholinyle,
• R₃ et R₄ identiques ou différents représentent un atome d'hydrogène ou d'halogène ; un groupement hydroxyle ou amino ; un groupement Z ; un groupement A₁, A₄, ou A₅ tels que définis ci dessous; un groupement A₁, A₂, A₃, A₄ ou A₅ tels que définis ci dessous et séparés du noyau phénolique de la formule (I) par un atome d'oxygène ou par un groupement -NH-, -Nalkyl(C₁₋C₃)-, -O(CO)-, -NH(CO)-, -Nalkyl(C₁-C₃)CO)-, -NH[C=NH]-, -NH(CO)NH-, -NH(CO)Nalkyl(C₁-C₃)-, -NH(CO)O-, -NHSO₂-, -NHSO₂NH-, ou -NHSO₂Nalkyl(C₁-C₃)-
- le groupement A₁ représente les radicaux alkyle en C₁-C₈, linéaire ou ramifié, pouvant porter une ou deux doubles liaisons ou une triple liaison, être substitué ou non substitué par un groupement choisi parmi un groupement A₂, A₄, et A₅ tels que définis ci-dessous, être substitué ou non substitué par un ou deux groupements, identiques ou différents, choisis parmi les groupements N-alkyl(C₁-C₃)amino, N-alkyl(C₁-C₃)-N-alkyl(C₁-C₃)amino, alcoxy(C₁-C₆), oxo, alcoxycarbonyle, acyloxy, amide, acylamino, uréyle, sulfoxy, sulfonyle, sulfonamido, sulfonylamino, bromo, cyano, carboxy, et être substitué ou non substitué par un ou plusieurs groupements hydroxyle, fluoro ou chloro ;
- le groupement A₂ représente un groupement aromatique de type phényle ou naphtyle, pouvant être substitué ou non substitué par un à trois groupements, identiques ou différents, choisis parmi les groupements méthyle, trifluorométhyle, éthyle, isopropyle, butyle, pentyle, fluoro, chloro, bromo, méthoxy, trifluorométhoxy, éthoxy, propyloxy, acétyloxy, acétyle, et cyano ;
- le groupement A₃ représente des groupements hétéroaromatiques choisis parmi les groupements furanyle, thiophényle, pyrrolyle, imidazolyle, thiazolyle, oxazolyle, 1,2,3-triazolyle, 1,2,4-triazolyle, isoxazolyle, isothiazolyle, pyrazolyle, pyrazoltriazolyle, pyrazoloimidazolyle, pyrrolotriazolyle, pyrazolopyrimidyle, pyrazolopyridyle, pyridyle, pyrimidyle, benzoimidazolyle, benzoxazolyle, benzothiazolyle, indolyle, indolidinyle, isoindolyle, indazolyle, benzotriazolyle, quinolinyle, benzoimidazolyle, benzopyrimidyle, lesdits groupements étant substitués ou non substitués par 1 à 3 radicaux choisis parmi les radicaux alkyle en C₁-C₄, linéaire ou ramifié, monohydroxyalkyle en C₁-C₄, polyhydroxyalkyle en C₂-C₄, carboxy, alkoxycarbonyle, halogène, amido, amino et hydroxy ;
- le groupement A₄ représente un radical cycloalkyle en C₃-C₇, un radical norbornanyle, portant ou non une double liaison et substitué ou non substitué par 1 ou 2 radicaux choisi parmi les radicaux alkyle en C₁-C₄, linéaire ou ramifié, monohydroxyalkyle en C₁-C₄, polyhydroxyalkyle en C₂-C₄, carboxy, alkoxycarbonyle, halogène, amido, amino et hydroxy ; ou
- le groupement A₅ représente un hétérocycle choisi parmi les cycles dihydrofuranyle, tétrahydrofuranyle, butyrolact-one-yle, dihydrothiophényle, tétrahydrothiophényle, tétrahydrothiophén-one-yle, iminothiolane, dihydropyrrolyle, pyrrolidinyle, pyrrolidin-one-yle, imidazolidin-one-yle, imidazolidinthione-yle, oxazolidinyle, oxazolidin-one-yle, oxazolanethione, thiazolidinyle, isothiazol-one-yle, mercaptothiazolinyle, pyrazolidin-one-yle, iminothiolane, dioxolanyle, pentalactone, dioxanyle, dihydropyridinyle, pipéridinyle, pentalactame, morpholinyle, pyrazoli(di)nyle, pyrimi(di)nyl, pyrazinyle, pipérazinyle et azépinyle,
• R₅ représente un atome d'hydrogène ou d'halogène ; un groupement Z ; un groupement A₁, A₄, ou A₅; un groupement A₁, A₂, A₃, A₄ ou A₅ séparés du noyau phénolique des composés de formule (I) par un atome d'oxygène, de soufre, ou par un groupement -NH-, -Nalkyl(C₁-C₃)-, -O(CO)-, -NH(CO)-,-Nalkyl(C₁-C₃)(CO)-, -NH[C=NH]-, -NH(CO)NH-, -NH(CO)Nalkyl(C₁-C₃)-, ou-NH(CO)O-,
• Y représente un atome d'hydrogène ou d'halogène ; un groupement-OCH₂COOH, -OCH₂COOCH₃, -OCH₂COOC₂H₅, -OR₆, -SR₆ ou -NH-SO₂R₆ dans lesquels R₆ représente un radical alkyle en C₁-C₆, linéaire ou ramifié (la ou les ramifications pouvant alors former un ou plusieurs cycles comportant de 3 à 6 chaînons), substitué ou non substitué par un ou plusieurs radicaux choisis dans le groupe constitué par un atome d'halogène, un radical hydroxy, alcoxy en C₁-C₄, amino et aminoalkyl en C₁-C₄ ; un radical phényle substitué ou non substitué par un ou deux radicaux choisi dans le groupe constitué par un radical alkyle en C₁-C₄, trifluorométhyle, carboxy, alcoxycarbonyle en C₁-C₄, halogène, hydroxy, alcoxy en C₁-C₄, amino, et aminoalkyl en C₁-C₄ ; ou un radical benzyle ; et étant entendu que Y ne peut représenter -NH-SO₂R₆ lorsque R₃ représente un radical hydroxyle ;
• Z est un groupement cationique représenté par la formule (II) suivante: 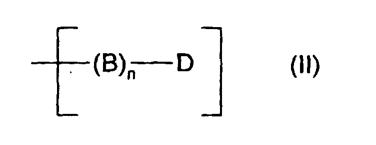
dans laquelle :
- B représente un radical comportant de 1 à 15 atomes de carbone, linéaire ou ramifié (la ou les ramifications pouvant alors former un ou plusieurs cycles comportant de 3 à 7 chaînons), pouvant contenir une ou plusieurs liaisons doubles et/ou une ou plusieurs liaisons triples, lesdites liaisons doubles conduisant éventuellement à des groupements aromatiques, et dont un ou plusieurs atomes de carbone peuvent être remplacés par un atome d'oxygène, d'azote, ou de soufre ou par un radical SO₂ ; et dont un ou plusieurs atomes de carbone peuvent, indépendamment les uns des autres, être substitués par un ou plusieurs atomes d'halogène ou par un ou plusieurs groupements Z ; ledit radical B ne comportant pas de liaison peroxyde ni de radicaux diazo, nitro ou nitroso ;
- n est égal à 0 ou 1,
- D est choisi parmi les groupements cationiques imidazolinium, thiazolinium, oxazolinium, pyrrolinium, 1,2,3-triazolinium, 1,2,4-triazolinium, isoxazolinium, ixothiazolinium, imidazolidinium, thiazolidinium, pyrazolinium, pyrazolidinium, oxazolidinium, pyrazoltriazolinium, pyrazoloimidazolinium, pyrrolotriazolinium, pyrazolopyrimidinium, pyrazolopyridinium, pyridinium, pyrimidinium, pyrazinium, triazinium, benzoimidazolinium, benzoxazolinium, benzothiazolinium, indolinium, indolidinium, isoindolinium, indazolinium, benzotriazolinium, quinolinium, tétrahydroquinolinium, benzoimidazolidinium, benzopyrimidinium, tétra-alkyl(C₁-C₄)ammonium, polyhydroxyltétra-alkyl(C₁-C₄)ammonium, dialkylpipéridinium, dialkylpyrrolidinium, dialkylmorpholinium, dialkylthiomorpholinium, dialkylpipérazinium, azépinium, et 1,4-diazabicyclo[2,2,2]octanium,
étant entendu qu'au moins un des groupements R₁ à R₅ représente un groupement Z.

2. Composition selon la revendication 1, **caractérisée par le fait que** R₂ représente un radical choisi dans le groupe **(G2)** constitué par un radical méthyle, éthyle, propyle, allyle, phényle, tétrahydrofuran-2-yl, furan-2-yl, thiophène-2-yl, pyridinyle, pipérazin-2-yl, fluorométhyle, chlorométhyle, 2-chloroéthyle, méthoxyméthyle, acétoxyméthyle, 1,2-dihydroxyéthyle, méthoxycarbonyl, 2-carboxyéthyle, méthoxy, éthoxy, propoxy, allyloxy, 2-chloroéthoxy, 2-méthoxyéthoxy, amino, éthylamino, allylamino, 2-chloroéthylamino, pyridylamino, diméthylamino, 1-pyrolidinyle, et 4-morpholinyle ; ou un groupement -D', -E-D', -O-E-D', -NH-E-D', dans lesquels -E- représente un bras -(CH₂)_{q}-, q étant un nombre entier égal à 1 ou 2, et **D'** représente un groupement choisi parmi 3-méthylimidazolidinium-1-yl, 3-(2-hydroxyéthyl)-imidazolidinium-1-yl, 1,2,4-triazolinium-1-yl, 1,2,4-triazolinium-4-yl, N-alkyl(C₁-C₄)pyridinium-2-yl, N-alkyl(C₁-C₄)pyridinium-3-yl, N-alkyl(C₁-C₄)pyridinium-4-yl, N-(2-hydroxyéthyl)pyridinium-2-yl, N-(2-hydroxyéthyl)pyridinium-3-yl, N-(2-hydroxyéthyl)pyridinium-4-yl, pyridinium-1-yl, trialkyl(C₁-C₄)ammonium-N-yl, 1-méthylpipéridinium-1-yl et 1,4-diméthylpipérazinium-1-yl.

3. Composition selon la revendication 2, **caractérisée par le fait que** R₂ représente un radical méthyle, méthoxyméthyle, 2-carboxyéthyle, méthoxy, amino, éthylamino, 1-pyrolidinyle; un groupement -D', -E-D', -O-E-D', -NH-E-D', dans lesquels -E- représente un bras -(CH₂)_{q}-, q=1 à 2.

4. Composition selon la revendication 1, **caractérisée par le fait que** R₃ représente un atome d'hydrogène ou de chlore ; un groupement Z ; un radical méthyle, hydroxyméthyle, méthoxyméthyle, 1-hydroxyéthyle, aminométhyle, méthylaminométhyle, hydroxy, méthoxy, acétoxy, amino, méthylamino, ou 2-hydroxyéthylamino ; un groupement -NH(CO)R₁₂ dans lequel R₁₂ représente un des radicaux listés dans le groupe **(G1)** ; un groupement -NHSO₂R₁₃, dans lequel R₁₃ représente un des radicaux listés dans le groupe **(G3)** constitué par les radicaux méthyle, trifluorométhyle, éthyle, 2-chloroéthyle, propyle, 3-chloropropyle, isopropyle, butyle, thiophène-2-yl, hydroxy, éthoxy et diméthylamino.

5. Composition selon la revendication 4 **caractérisée par le fait que** R₃ représente un atome d'hydrogène ; un radical méthyle, hydroxyméthyle, aminométhyle, hydroxy, méthoxy, amino, méthylamino ; un groupement méthanesulfonylamino ; éthanesulfonylamino ; diméthylamino-sulfonylamino ; un groupement -NH(CO)R₁₄ dans lequel R₁₄ représente l'un des radicaux listés dans le groupe **(G2)** ; ou un groupement -O-E-D', -NH-E-D', -CH₂O-E-D',-CH₂NH-E-D', -CH₂NH(CO)-D', -NH(CO)-D', -NH(CO)-E-D', -NH(CO)O-E-D', -NH(CO)NH-E-D', ou -NH(SO₂)-E-D'.

6. Composition selon la revendication 1, **caractérisée par le fait que** R₄ représente un atome d'hydrogène ou de chlore ; un groupement Z ; un radical méthyle, éthyle, hydroxyméthyle, méthoxyméthyle, aminométhyle, méthylaminométhyle, hydroxy, méthoxy, acétoxy, amino, méthylamino, N-pipéridino, ou N-morpholino ; un groupement -NH(CO)R₁₅ dans lequel R₁₅ représente l'un des radicaux listés dans le groupe **(G1)** ; ou un groupement -NHSO₂R₁₆ dans lequel R₁₆ représente l'un des radicaux listés dans le groupe **(G3)**.

7. Composition selon la revendication 6, **caractérisée par le fait que** R₄ représente un atome d'hydrogène ou de chlore ; un radical méthyle, hydroxyméthyle, aminométhyle, hydroxy, méthoxy, amino, ou méthylamino ; un groupement méthanesulfonylamino ; éthanesulfonylamino ; diméthylaminosulfonylamino ; un groupement -NH(CO)R₁₇ dans lequel R₁₇ représente un des radicaux listés dans le groupe **(G2)** ; ou un groupement -O-E-D', -NH-E-D', -CH₂O-E-D', -CH₂NH-E-D', -CH₂NH(CO)-D', -NH(CO)-D',-NH(CO)-E-D', -NH(CO)O-E-D', -NH(CO)NH-E-D', ou -NH(SO₂)-E-D'.

8. Composition selon la revendication 1, **caractérisée par le fait que** R₅ représente un atome d'hydrogène, de chlore, de fluor, ou de brome; un groupement Z; un radical méthyle, trifluorométhyle, allyle, hydroxyméthyle, méthoxyméthyle, 1-hydroxyéthyle, aminométhyle, méthylaminométhyle, méthoxy, acétoxy, ou méthylamino ; ou un groupement -NH(CO)R₁₈ dans lequel R₁₈ représente l'un des radicaux listés dans le groupe **(G1)**.

9. Composition selon la revendication 8, **caractérisée par le fait que** R₅ représenté un atome d'hydrogène, de chlore, ou de fluor ; un radical méthyle, hydroxyméthyle, aminométhyle, méthoxy, ou méthylamino ; un groupement -NH(CO)R₁₉ dans lesquels R₁₉ représente un des radicaux listés dans le groupe (G2) tel que défini à la revendication 7 ; ou un groupement -O-E-D', -NH-E-D', -CH₂O-E-D', -CH₂NH-E-D', -CH₂NH(GO)-D', -NH(CO)-D', -NH(CO)-E-D', -NH(CO)O-E-D', -NH(CO)NH-E-D'.

10. Composition selon l'une quelconque des revendications précédentes, **caractérisée par le fait que** Y est choisi parmi un atome d'hydrogène, de chlore, de fluor, ou de brome ; un groupement méthoxy, éthoxy, propoxy, benzyloxy, phénoxy, -OCH₂CH₂OCH₃; -OCH₂CH₂OCH₃; -OCH₂CH₂N(CH₃)₂; -OCH₂(CO)OH, -OCH₂(CO)OCH₃, -OCH₂(CO)OC₂H₅, -SCH₂CH₂CO₂H, ou -NHSO₂CH₃ ; étant entendu que Y ne peut représenter un groupement -NHSO₂CH₃ lorsque R₃ représente un radical hydroxyle.

11. Composition selon la revendication 1, **caractérisée par le fait que** D représente un groupement 3-méthylimidazolidinium-1-yl, 3-(2-hydroxyéthyl)-imidazolidinium-1-yl, 1,2,4-triazolinium-1-yl, 1,2,4-triazolinium-4-yl, N-alkyl(C₁-C₄)pyridin-2-yl, N-alkyl(C₁-C₄)pyridin-3-yl, N-alkyl(C₁-C₄)pyridin-4-yl, N-(2-hydroxyéthyl) pyridin-2-yl, N-(2-hydroxyéthyl)pyridin-3-yl, N-(2-hydroxyéthyl)pyridin-4-yl, pyridin-1-yl, trialkyl(C₁-C₄)ammonium-N-yl, 1-méthylpipéridinium-1-yl, thiazolinium3-yl ou 1,4-diméthylpipérazinium-1-yl.

12. Composition selon l'une quelconque des revendications précédentes, **caractérisée par le fait que** le ou les composés de formule (I) sont choisis parmi ceux dans lesquels :
i)
- R₁ représente un atome d'hydrogène ;
- R₂ représente un groupement -D', -E-D', -O-E-D', -NH-E-D', ou un radical choisi dans le groupe **(G4)** constitué par un radical méthyle, méthoxyméthyle, 2-carboxyéthyle, méthoxy, amino, éthylamino, 1-pyrolidinyle ;
- R₃ représente un radical hydroxy, amino, ou méthylamino ; un groupement -NH(CO)R₂₀ dans lequel R₂₀ représente l'un des radicaux listés dans le groupe **(G4)** défini ci-dessus ; un groupement méthanesulfonylamino, éthanesulfonylamino, ou diméthylaminosulfonylamino ; un groupement -O-E-D', -NH-E-D', -NH(CO)-D', -NH(CO)-E-D', -NH(CO)O-E-D', -NH(CO)NH-E-D', ou -NH(SO₂)-E-D';
- R₄ représente un atome d'hydrogène ou de chlore, ou un groupement méthyle ;
- R₅ représente un atome d'hydrogène, de chlore ou de fluor, ou un groupement méthyle ;
- Y représente un atome d'hydrogène ou de chlore ; un groupement méthoxy, ou -OCH₂(CO)OCH₃ ; étant entendu qu'au moins un des groupements R₂ et R₃ contient un groupement Z ;
ii)
- R₁ représente un atome d'hydrogène ;
- R₂ représente un groupement -D₁, -E-D', -O-E-D', ou -NH-E-D', ou l'un des radicaux listés dans le groupe **(G4)**;
- R₃ représente un atome d'hydrogène ; ou un radical méthyle ;
- R₄ représente un radical hydroxy, amino, méthylamino, méthanesulfonylamino, éthanesulfonylamino, ou diméthylamino-sulfonylamino ; un groupement -NH(CO)R₂₁ dans lequel R₂₁ représente un des radicaux listés dans le groupe **(G4)** ; ou un groupement -O-E-D', -NH-E-D', -NH(CO)-D', -NH(CO)-E-D'; -NH(CO)O-E-D', -NH(CO)NH-E-D', ou -NH(SO₂)-E-D';
- R₅ représente un atome d'hydrogène, de chlore, ou de fluor ; ou un groupement méthyle, méthoxy, ou méthylamino ;
- Y représente un atome d'hydrogène ou de chlore ; un groupement méthoxy, ou -OCH₂(CO)OCH₃ ; étant entendu qu'au moins un des groupements R₂ et R₄ contient un groupement Z ;
iii)
- R₁ représente un atome d'hydrogène ;
- R₂ représente un des radicaux listés dans le groupe **(G4)** défini précédemment ; ou un groupement -D', -E-D', -O-E-D', -NH-E-D',
- R₃ représente un atome d'hydrogène ; ou un radical méthyle ;
- R₄ représente un atome d'hydrogène ou de chlore ; un radical méthyle ; ou un groupement méthoxy, ou méthylamino ;
- R₅ représente un groupement -NH(CO)R₂₂ dans lequel R₂₂ représente l'un des radicaux listés dans le groupe **(G4)**; ou un groupement -O-E-D', -NH-E-D', -NH(CO)-D', -NH(CO)-E-D', -NH(CO)O-E-D', ou -NH(CO)NH-E-D',
- Y représente un atome d'hydrogène ou de chlore ; ou un groupement méthoxy, ou -OCH₂(CO)OCH₃ ; étant entendu qu'au moins un des groupements R₂ et R₅ contient un groupement Z ;
iv)
- R₁ représente un atome d'hydrogène ;
- R₂ représente un groupement -D', -E-D', -O-E-D', ou -NH-E-D',
- R₃ représente un atome d'hydrogène ; ou un radical méthyle ;
- R₄ représente un atome d'hydrogène ou de chlore ; ou un radical méthyle ;
- R₅ représente un atome d'hydrogène, de chlore ou de fluor ; ou un groupement méthyle ;
- Y représente un atome d'hydrogène, ou de chlore ; ou un groupement méthoxy, ou -OCH₂(CO)OCH₃.

13. Composition selon l'une quelconque des revendications précédentes, **caractérisée par le fait que** le ou les composés de formule (I) sont choisis parmi
- le chlorure de 3-[(2-hydroxy-phénylcarbamoyl)-méthyl]-1-méthyl-3H-imidazol-1-ium ;
- le dichlorure de 3-[(2-hydroxy-3-(2-(3-méthyl-1H-imidazol-3-ium-1-yl)-acétylamino)-phénylcarbamoyl)-méthyl]-1-méthyl-3H-imidazol-1-ium ;
- le dichlorure de 3-[(2-hydroxy-4-(2-(3-méthyl-1H-imidazol-3-ium-1-yl)-acétylamino)-phénylcarbamoyl)-méthyl]-1-méthyl-3H-imidazol-1-ium ;
- le chlorure de 3-[(2-hydroxy-4-méthyl-phénylcarbamoyl)-méthyl]-1-méthyl-3H-imidazol-1-ium ;
- le chlorure de 3-[(2-hydroxy-4-amino-phénylcarbamoyl)-méthyl]-1-méthyl-3H-imidazol-1-ium ;
- le chlorure de 3-[(2-hydroxy-4-acétylamino-phénylcarbamoyl)-méthyl]-1-méthyl-3H-imidazol-1-ium ;
- le chlorure de 3-[(2-hydroxy-4-méthoxycarbonylamino-phénylcarbamoyl)-méthyl]-1-méthyl-3H-imidazol-1-ium ;
- le chlorure de 3-[(3-hydroxy-4-acétylamino-phénylcarbamoyl)-méthyl]-1-méthyl-3H-imidazol-1-ium ;
- le chlorure de 3-[(3-hydroxy-4-méthoxycarbonylamino-phénylcarbamoyl)-méthyl]-1-méthyl-3H-imidazol-1-ium ;
- le chlorure de 3-[(2-hydroxy-5-chloro-phénylcarbamoyl)-méthyl]-1-méthyl-3H-imidazol-1-ium ;
- le chlorure de 3-[(2-hydroxy-4-méthyl-5-chloro-phénylcarbamoyl)-méthyl]-1-méthyl-3H-imidazol-1-ium ;
- le chlorure de 3-[(2-hydroxy-4-amino-5-chloro-phénylcarbamoyl)-méthyl]-1-méthyl-3H-imidazol-1-ium ;
- le chlorure de 3-[(2-hydroxy-4-acétylamino-5-chloro-phénylcarbamoyl)-méthyl]-1-méthyl-3H-imidazol-1-ium ;
- le chlorure de 3-[(2-hydroxy-4-méthoxycarbonylamino-5-chloro-phénylcarbamoyl)-méthyl]-1-méthyl-3H-imidazol-1-ium ;
- le chlorure de 3-[(3-hydroxy-4-acétylamino-5-chloro-phénylcarbamoyl)-méthyl]-1-méthyl-3H-imidazol-1-ium ;
- le chlorure de 3-[(3-hydroxy-4-méthoxycarbonylamino-6-chloro-phénylcarbamoyl)-méthyl]-1-méthyl-3H-imidazol-1-ium ;
- le chlorure de 3-[(2-hydroxy-5-méthoxy-phénylcarbamoyl)-méthyl]-1-méthyl-3H-imidazol-1-ium ;
- le chlorure de 3-[(2-hydroxy-4-méthyl-5-méthoxy-phénylcarbamoyl)-méthyl]-1-méthyl-3H-imidazol-1-ium ;
- le chlorure de 3-[(2-hydroxy-4-amino-5-méthoxy-phénylcarbamoyl)-méthyl]-1-méthyl-3H-imidazol-1-ium ;
- le chlorure de 3-[(2-hydroxy-4-acétylamino-5-méthoxy-phénylcarbamoyl)-méthyl]-1-méthyl-3H-imidazol-1-ium ;
- le chlorure de 3-[(2-hydroxy-4-méthoxycarbonylamino-5-méthoxy-phénylcarbamoyl)-méthyl]-1-méthyl-3H-imidazol-1-ium ;
- le chlorure de 3-[(3-hydroxy-4-acétylamino-6-méthoxy-phénylcarbamoyl)-méthyl]-1-méthyl-3H-imidazol-1-ium ;
- le chlorure de 3-[(3-hydroxy-4-méthoxycarbonylamino-6-méthoxy-phénylcarbamoyl)-méthyl]-1-méthyl-3H-imidazol-1-ium ;
- le chlorure de 3-[(2-hydroxy-6-amino-phénylcarbamoyl)-méthyl]-1-méthyl-3H-imidazol-1-ium ;
- le chlorure de 3-[(2-hydroxy-6-acétylamino-phénylcarbamoyl)-méthyl]-1-méthyl-3H-imidazol-1-ium ;
- le chlorure de 3-[(2-hydroxy-4,6-diamino-phénylcarbamoyl)-méthyl]-1-méthyl-3H-imidazol-1-ium ;
- le chlorure de 3-[(2-hydroxy-4-acétylamino-6-amino-phénylcarbamoyl)-méthyl]-1-méthyl-3H-imidazol-1-ium ;
- le chlorure de 3-[(2-hydroxy-3,5-dichloro-4-méthyl-phénylcarbamoyl)-méthyl]-1-méthyl-3H-imidazol-1-ium ;
- le chlorure de 3-[(2-hydroxy-3,5-dichloro-4-amino-phénylcarbamoyl)-méthyl]-1-méthyl-3H-imidazol-1-ium ;
- le chlorure de 3-[(2-hydroxy-3,5-dichloro-4-acétylamino-phénylcarbamoyl)-méthyl]-1-méthyl-3H-imidazol-1-ium ;
- le chlorure de 3-[(2-hydroxy-3,5-dichloro-4-méthoxycarbonylamino-phénylcarbamoyl)-méthyl]-1-méthyl-3H-imidazol-1-ium ;
- le chlorure de 3-[(2-hydroxy-3-acétylamino-phénylcarbamoyl)-méthyl]-1-méthyl-3H-imidazol-1-ium ;
- le chlorure de 1-[(2-hydroxy-phénylcarbamoyl)-méthyl]-pyridinium ;
- le dichlorure de 1-[(2-hydroxy-3-(2-(pyridinium-1-yl)acétylamino)-phénylcarbamoyl)-méthyl]-pyridinium ;
- le dichlorure de 1-[(2-hydroxy-4-(2-(pyridinium-1-yl)acétylamino)-phénylcarbamoyl)-méthyl]-pyridinium ;
- le chlorure de 1-[(2-hydroxy-4-méthyl-phénylcarbamoyl)-méthyl]-pyridinium ;
- le chlorure de 1-[(2-hydroxy-4-amino-phénylcarbamoyl)-méthyl]-pyridinium ;
- le chlorure de 1-[(2-hydroxy-4-acétylamino-phénylcarbamoyl)-méthyl]-pyridinium ;
- le chlorure de 1-[(2-hydroxy-4-méthoxycarbonylamino-phénylcarbamoyl)-méthyl]-pyridinium ;
- le chlorure de 1-[(3-hydroxy-4-acétylamino-phénylcarbamoyl)-méthyl]-pyridinium ;
- le chlorure de 1-[(3-hydroxy-4-méthoxycarbonylamino-phénylcarbamoyl)-méthyl]-pyridinium ;
- le chlorure de 1-[(2-hydroxy-5-chloro-phénylcarbamoyl)-méthyl]-pyridinium ;
- le chlorure de 1-[(2-hydroxy-4-méthyl-5-chloro-phénylcarbamoyl)-méthyl]-pyridinium ;
- le chlorure de 1-[(2-hydroxy-4-amino-5-chloro-phénylcarbamoyl)-méthyl]-pyridinium ;
- le chlorure de 1-[(2-hydroxy-4-acétylamino-5-chloro-phénylcarbamoyl)-méthyl]-pyridinium ;
- le chlorure de 1-[(2-hydroxy-4-méthoxycarbonylamino-5-chloro-phénylcarbamoyl)-méthyl]-pyridinium ;
- le chlorure de 1-[(3-hydroxy-4-acétylamino-6-chloro-phénylcarbamoyl)-méthyl]-pyridinium ;
- le chlorure de 1-[(3-hydroxy-4-méthoxycarbonylamino-6-chloro-phénylcarbamoyl)-méthyl]-pyridinium ;
- le chlorure de 1-[(2-hydroxy-5-méthoxy-phénylcarbamoyl)-méthyl]-pyridinium ;
- le chlorure de 1-[(2-hydroxy-4-méthyl-5-méthoxy-phénylcarbamoyl)-méthyl]-pyridinium ;
- le chlorure de 1-[(2-hydroxy-4-amino-5-méthoxy-phénylcarbamoyl)-méthyl]-pyridinium ;
- le chlorure de 1-[(2-hydroxy-4-acétylamino-5-méthoxy-phénylcarbamoyl)-méthyl]-pyridinium ;
- le chlorure de 1-[(2-hydroxy-4-méthoxycarbonylamino-5-méthoxy-phénylcarbamoyl)-méthyl]-pyridinium ;
- le chlorure de 1-[(3-hydroxy-4-acétylamino-6-méthoxy-phénylcarbamoyl)-méthyl]-pyridinium ;
- le chlorure de 1-[(3-hydroxy-4-méthoxycarbonylamino-6-méthoxyphénylcarbamoyl)-méthyl]-pyridinium ;
- le chlorure de 1-[(2-hydroxy-6-amino-phénylcarbamoyl)-méthyl]-pyridinium ;
- le chlorure de 1-[(2-hydroxy-6-acétylamino-phénylcarbamoyl)-méthyl]-pyridinium ;
- le chlorure de 1-[(2-hydroxy-4,6-diamino-phénylcarbamoyl)-méthyl]-pyridinium ;
- le chlorure de 1-[(2-hydroxy-4-acétylamino-6-amino-phénylcarbamoyl)-méthyl]-pyridinium ;
- le chlorure de 1-[(2-hydroxy-3,5-dichloro-4-méthyl-phénylcarbamoyl)-méthyl]-pyridinium ;
- le chlorure de 1-[(2-hydroxy-3,5-dichloro-4-amino-phénylcarbamoyl)-méthyl]-pyridinium ;
- le chlorure de 1-[(2-hydroxy-3,5-dichloro-4-acétylamino-phénylcarbamoyl)-méthyl]-pyridinium ;
- le chlorure de 1-[(2-hydroxy-3,5-dichloro-4-méthoxycarbonylamino-phényl-carbamoyl)-méthyl]-pyridinium ;
- le chlorure de 1-[(2-hydroxy-3-acétylamino-phénylcarbamoyl)-méthyl]-pyridinium ;
- le chlorure de 1-[(2-hydroxy-phénylcarbamoyl)-méthyl]-1,4-diméthyl-pipérazin-1-ium ;
- le dichlorure de 1-[(2-hydroxy-3-(2-(1,4-diméthyl-pipérazin-1-ium-1-yl)-acétyl)amino-phénylcarbamoyl)-méthyl]-1,4-diméthyl-pipérazin-1-ium ;
- le dichlorure de 1-[(2-hydroxy-4-(2-(1,4-diméthyl-pipérazin-1-ium-1-yl) acétyl)amino-phényl-carbamoyl)-méthyl]-1,4-diméthyl-pipérazin-1-ium ;
- le chlorure de 1-[(2-hydroxy-4-méthyl-phénylcarbamoyl)-méthyl]-1,4-diméthyl-pipérazin-1-ium ;
- le chlorure de 1-[(2-hydroxy-4-amino-phénylcarbamoyl)-méthyl]-1,4-diméthyl-pipérazin-1-ium ;
- le chlorure de 1-[(2-hydroxy-4-acétylamino-phénylcarbamoyl)-méthyl]-1,4-diméthyl-pipérazin-1-ium ;
- le chlorure de 1-[(2-hydroxy-4-méthoxycarbonylamino-phénylcarbamoyl)-méthyl]-1,4-diméthyl-pipérazin-1-ium ;
- le chlorure de 1-[(3-hydroxy-4-acétylamino-phénylcarbamoyl)-méthyl]-1,4-diméthyl-pipérazin-1-ium ;
- le chlorure de 1-[(3-hydroxy-4-méthoxycarbonylamino-phénylcarbamoyl)-méthyl]-1,4-diméthyl-pipérazin-1-ium ;
- le chlorure de 1-[(2-hydroxy-5-chloro-phénylcarbamoyl)-méthyl]-1,4-diméthyl-pipérazin-1-ium ;
- le chlorure de 1-[(2-hydroxy-4-méthyl-5-chloro-phénylcarbamoyl)-méthyl]-1,4-diméthyl-pipérazin-1-ium ;
- le chlorure de 1-[(2-hydroxy-4-amino-5-chloro-phénylcarbamoyl)-méthyl]-1,4-diméthyl-pipérazin-1-ium ;
- le chlorure de 1-[(2-hydroxy-4-acétylamino-5-chloro-phénylcarbamoyl)-méthyl]-1,4-diméthyl-pipérazin-1-ium ;
- le chlorure de 1-[(2-hydroxy-4-méthoxycarbonylamino-5-chloro-phénylcarbamoyl)-méthyl]-1,4-diméthyl-pipérazin-1-ium ;
- le chlorure de 1-[(3-hydroxy-4-acétylamino-6-chloro-phénylcarbamoyl)-méthyl]-1,4-diméthyl-pipérazin-1-ium ;
- le chlorure de 1-[(3-hydroxy-4-méthoxycarbonylamino-6-chloro-phénylcarbamoyl)-méthyl]-1,4-diméthyl-pipérazin-1-ium ;
- le chlorure de 1-[(2-hydroxy-5-méthoxy-phénylcarbamoyl)-méthyl]-1,4-diméthyl-pipérazin-1-ium ;
- le chlorure de 1-[(2-hydroxy-4-méthyl-5-méthoxy-phénylcarbamoyl)-méthyl]-1,4-diméthyl-pipérazin-1-ium ;
- le chlorure de 1-[(2-hydroxy-4-amino-5-méthoxy-phénylcarbamoyl)-méthyl]-1,4-diméthyl-pipérazin-1-ium ;
- le chlorure de 1-[(2-hydroxy-4-acétylamino-5-méthoxy-phénylcarbamoyl)-méthyl]-1,4-diméthyl-pipérazin-1-ium ;
- le chlorure de 1-[(2-hydroxy-4-méthoxycarbonylamino-5-méthoxy-phénylcarbamoyl)-méthyl]-1,4-diméthyl-pipérazin-1-ium ;
- le chlorure de 1-[(3-hydroxy-4-acétylamino-6-méthoxy-phénylcarbamoyl)-méthyl]-1,4-diméthyl-pipérazin-1-ium ;
- le chlorure de 1-[(3-hydroxy-4-méthoxycarbonylamino-6-méthoxy-phénylcarbamoyl)-méthyl]-1,4-diméthyl-pipérazin-1-ium ;
- le chlorure de 1-[(2-hydroxy-6-amino-phénylcarbamoyl)-méthyl]-1,4-diméthyl-pipérazin-1-ium ;
- le chlorure de 1-[(2-hydroxy-6-acétylamino-phénylcarbamoyl)-méthyl]-1,4-diméthyl-pipérazin-1-ium ;
- le chlorure de 1-[(2-hydroxy-4,6-diamino-phénylcarbamoyl)-méthyl]-1,4-diméthyl-pipérazin-1-ium ;
- le chlorure de 1-[(2-hydroxy-4-acétylamino-6-amino-phénylcarbamoyl)-méthyl]-1,4-diméthyl-pipérazin-1-ium;
- le chlorure de 1-[(2-hydroxy-3,5-dichloro-4-méthyl-phénylcarbamoyl)-méthyl]-1,4-diméthyl-pipérazin-1-ium ;
- le chlorure de 1-[(2-hydroxy-3,5-dichloro-4-amino-phénylcarbamoyl)-méthyl]-1,4-diméthyl-pipérazin-1-ium ;
- le chlorure de 1-[(2-hydroxy-3,5-dichloro-4-acétylamino-phénylcarbamoyl)-méthyl]-1,4-diméthyl-pipérazin-1-ium ;
- le chlorure de 1-[(2-hydroxy-3,5-dichloro-4-méthoxycarbonylamino-phénylcarbamoyl)-méthyl]-1,4-diméthyl-pipérazin-1-ium ;
- le chlorure de 1-[(2-hydroxy-3-acétylamino-phénylcarbamoyl)-méthyl]-1,4-diméthyl-pipérazin-1-ium ;
et leurs sels d'addition avec un acide.

14. Composition selon l'une quelconque des revendications précédentes, **caractérisée par le fait que** le ou les composés de formule (I) et/ou le ou leurs sels d'addition avec un acide représentent de 0,0005 à 12 % en poids du poids total de la composition tinctoriale.

15. Composition selon l'une quelconque des revendications précédentes, **caractérisée par le fait que** la ou les bases d'oxydation sont choisies parmi les paraphénylènediamines, les bis-phénylalkylènediamines, les para-aminophénols, les ortho-aminophénols et les bases hétérocycliques.

16. Composition selon l'une quelconque des revendications précédentes, **caractérisée par le fait qu'**elle renferme un ou plusieurs coupleurs additionnels choisis parmi les métaphénylènediamines, les méta-aminophénols, les métadiphénols et les coupleurs hétérocycliques, et leurs sels d'addition avec un acide, et/ou un ou plusieurs colorants directs.

17. Composition selon l'une quelconque des revendications précédentes, **caractérisée par le fait que** les sels d'addition avec un acide sont choisis parmi les chlorhydrates, les bromhydrates, les sulfates, les citrates, les succinates, les tartrates, les lactates et les acétates.

18. Procédé de teinture d'oxydation des fibres kératiniques, **caractérisé par le fait que** l'on applique sur ces fibres au moins une composition tinctoriale telle que définie à l'une quelconque des revendications 1 à 17, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

19. Procédé selon la revendication 18, **caractérisé par le fait que** l'agent oxydant est choisi parmi le peroxyde d'hydrogène, le peroxyde d'urée, les bromates de métaux alcalins, les persels, et les enzymes.

20. Dispositif à plusieurs compartiments, ou "kit" de teinture à plusieurs compartiments, dont un premier compartiment renferme une composition tinctoriale telle que définie à l'une quelconque des revendications 1 à 17 et un second compartiment renferme une composition oxydante.

21. Composés de formule (I') suivante, et leurs sels d'addition avec un acide : 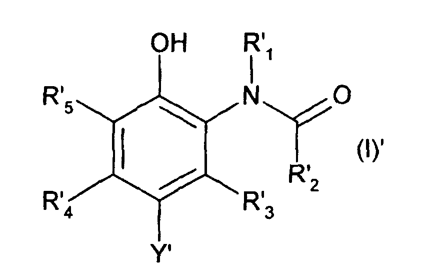 dans laquelle R'₁, R'₂, R'₃, R'₄, R'₅ et Y peuvent prendre les mêmes significations que celles indiquées à l'une quelconque des revendications 1 à 20 pour R₁, R₂, R₃, R₄, R₅ et Y, dans lesquels au moins un des groupements R'₁ à R'₅ représente un groupement Z' pouvant prendre les mêmes significations que celles indiquées pour le groupement Z à la revendication 1 ; à l'exclusion des composés suivants : 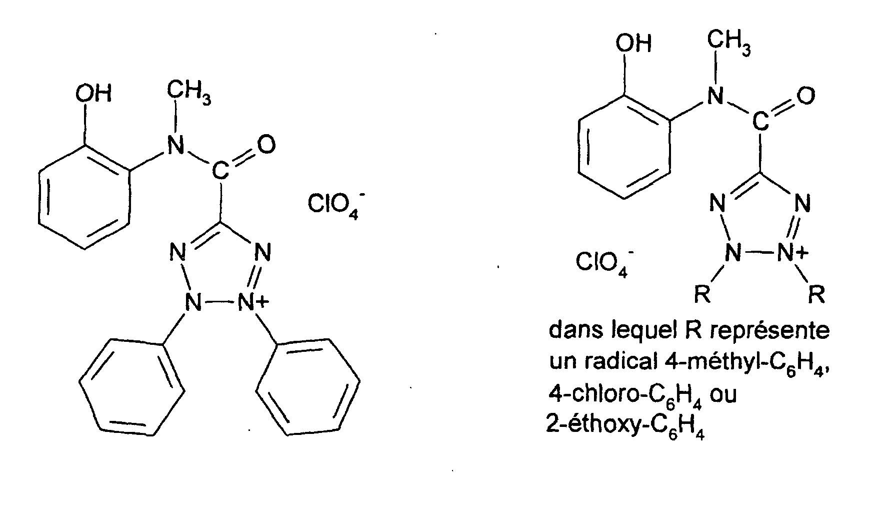 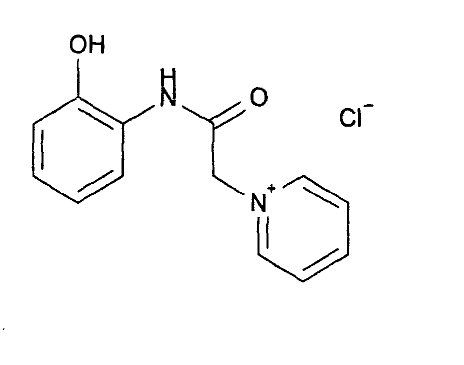 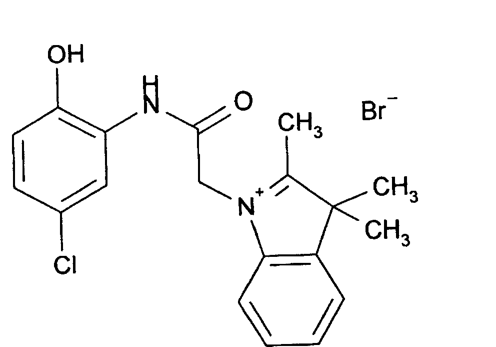 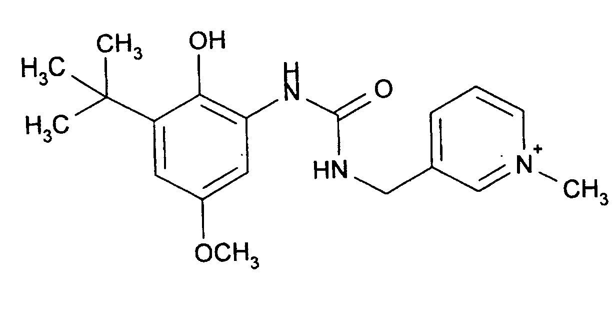 et

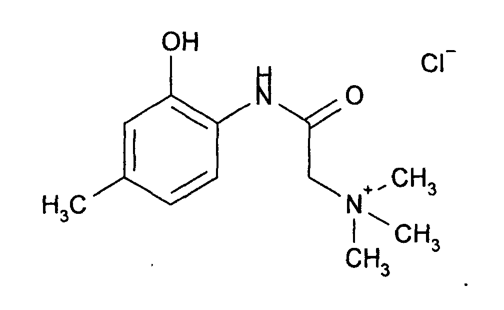

22. Composés selon la revendication 21, **caractérisés par le fait qu'**ils sont choisis parmi :
- le chlorure de 3-[(2-hydroxy-phénylcarbamoyl)-méthyl]-1-méthyl-3H-imidazol-1-ium ;
- le dichlorure de 3-[(2-hydroxy-3-(2-(3-méthyl-1H-imidazol-3-ium-1-yl)-acétylamino)-phénylcarbamoyl)-méthyl]-1-méthyl-3H-imidazol-1-ium ;
- le dichlorure de 3-[(2-hydroxy-4-(2-(3-méthyl-1H-imidazol-3-ium-1-yl)-acétylamino)-phénylcarbamoyl)-méthyl]-1-méthyl-3H-imidazol-1-ium ;
- le chlorure de 3-[(2-hydroxy-4-méthyl-phénylcarbamoyl)-méthyl]-1-méthyl-3H-imidazol-1-ium ;
- le chlorure de 3-[(2-hydroxy-4-amino-phénylcarbamoyl)-méthyl]-1-méthyl-3H-imidazol-1-ium ;
- le chlorure de 3-[(2-hydroxy-4-acétylamino-phénylcarbamoyl)-méthyl]-1-méthyl-3H-imidazol-1-ium ;
- le chlorure de 3-[(2-hydroxy-4-méthoxycarbonylamino-phénylcarbamoyl)-méthyl]-1-méthyl-3H-imidazol-1-ium ;
- le chlorure de 3-[(3-hydroxy-4-acétylamino-phénylcarbamoyl)-méthyl]-1-méthyl-3H-imidazol-1-ium ;
- le chlorure de 3-[(3-hydroxy-4-méthoxycarbonylamino-phénylcarbamoyl)-méthyl]-1-méthyl-3H-imidazol-1-ium ;
- le chlorure de 3-[(2-hydroxy-5-chloro-phénylcarbamoyl)-méthyl]-1-méthyl-3H-imidazol-1-ium ;
- le chlorure de 3-[(2-hydroxy-4-méthyl-5-chloro-phénylcarbamoyl)-méthyl]-1-méthyl-3H-imidazol-1-ium ;
- le chlorure de 3-[(2-hydroxy-4-amino-5-chloro-phénylcarbamoyl)-méthyl]-1-méthyl-3H-imidazol-1-ium ;
- le chlorure de 3-[(2-hydroxy-4-acétylamino-5-chloro-phénylcarbamoyl)-méthyl]-1-méthyl-3H-imidazol-1-ium ;
- le chlorure de 3-[(2-hydroxy-4-méthoxycarbonylamino-5-chloro-phénylcarbamoyl)-méthyl]-1-méthyl-3H-imidazol-1-ium ;
- le chlorure de 3-[(3-hydroxy-4-acétylamino-5-chloro-phénylcarbamoyl)-méthyl]-1-méthyl-3H-imidazol-1-ium ;
- le chlorure de 3-[(3-hydroxy-4-méthoxycarbonylamino-6-chloro-phénylcarbamoyl)-méthyl]-1-méthyl-3H-imidazol-1-ium ;
- le chlorure de 3-[(2-hydroxy-5-méthoxy-phénylcarbamoyl)-méthyl]-1-méthyl-3H-imidazol-1-ium ;
- le chlorure de 3-[(2-hydroxy-4-méthyl-5-méthoxy-phénylcarbamoyl)-méthyl]-1-méthyl-3H-imidazol-1-ium ;
- le chlorure de 3-[(2-hydroxy-4-amino-5-méthoxy-phénylcarbamoyl)-méthyl]-1-méthyl-3H-imidazol-1-ium ;
- le chlorure de 3-[(2-hydroxy-4-acétylamino-5-méthoxy-phénylcarbamoyl)-méthyl]-1-méthyl-3H-imidazol-1-ium ;
- le chlorure de 3-[(2-hydroxy-4-méthoxycarbonylamino-5-méthoxy-phénylcarbamoyl)-méthyl]-1-méthyl-3H-imidazol-1-ium ;
- le chlorure de 3-[(3-hydroxy-4-acétylamino-6-méthoxy-phénylcarbamoyl)-méthyl]-1-méthyl-3H-imidazol-1-ium ;
- le chlorure de 3-[(3-hydroxy-4-méthoxycarbonylamino-6-méthoxy-phénylcarbamoyl)-méthyl]-1-méthyl-3H-imidazol-1-ium ;
- le chlorure de 3-[(2-hydroxy-6-amino-phénylcarbamoyl)-méthyl]-1-méthyl-3H-imidazol-1-ium ;
- le chlorure de 3-[(2-hydroxy-6-acétylamino-phénylcarbamoyl)-méthyl]-1-méthyl-3H-imidazol-1-ium ;
- le chlorure de 3-[(2-hydroxy-4,6-diamino-phénylcarbamoyl)-méthyl]-1-méthyl-3H-imidazol-1-ium ;
- le chlorure de 3-[(2-hydroxy-4-acétylamino-6-amino-phénylcarbamoyl)-méthyl]-1-méthyl-3H-imidazol-1-ium ;
- le chlorure de 3-[(2-hydroxy-3,5-dichloro-4-méthyl-phénylcarbamoyl)-méthyl]-1-méthyl-3H-imidazol-1-ium ;
- le chlorure de 3-[(2-hydroxy-3,5-dichloro-4-amino-phénylcarbamoyl)-méthyl]-1-méthyl-3H-imidazol-1-ium ;
- le chlorure de 3-[(2-hydroxy-3,5-dichloro-4-acétylamino-phénylcarbamoyl)-méthyl]-1-méthyl-3H-imidazol-1-ium ;
- le chlorure de 3-[(2-hydroxy-3,5-dichloro-4-méthoxycarbonylamino-phénylcarbamoyl)-méthyl]-1-méthyl-3H-imidazol-1-ium ;
- le chlorure de 3-[(2-hydroxy-3-acétylamino-phénylcarbamoyl)-méthyl]-1-méthyl-3H-imidazol-1-ium ;
- le chlorure de 1-[(2-hydroxy-phénylcarbamoyl)-méthyl]-pyridinium ;
- le dichlorure de 1-[(2-hydroxy-3-(2-(pyridinium-1-yl)acétylamino)-phénylcarbamoyl)-méthyl]-pyridiniunn ;
- le dichlorure de 1-[(2-hydroxy-4-(2-(pyridinium-1-yl)acétylamino)-phénylcarbamoyl)-méthyl]-pyridinium ;
- le chlorure de 1-[(2-hydroxy-4-méthyl-phénylcarbamoyl)-méthyl]-pyridinium ;
- le chlorure de 1-[(2-hydroxy-4-amino-phénylcarbamoyl)-méthyl]-pyridinium ;
- le chlorure de 1-[(2-hydroxy-4-acétylamino-phénylcarbamoyl)-méthyl]-pyridinium ;
- le chlorure de 1-[(2-hydroxy-4-méthoxycarbonylamino-phénylcarbamoyl)-méthyl]-pyridinium ;
- le chlorure de 1-[(3-hydroxy-4-acétylamino-phénylcarbamoyl)-méthyl]-pyridinium ;
- le chlorure de 1-[(3-hydroxy-4-méthoxycarbonylamino-phénylcarbamoyl)-méthyl]-pyridinium ;
- le chlorure de 1-[(2-hydroxy-5-chloro-phénylcarbamoyl)-méthyl]-pyridinium ;
- le chlorure de 1-[(2-hydroxy-4-méthyl-5-chloro-phénylcarbamoyl)-méthyl]-pyridinium ;
- le chlorure de 1-[(2-hydroxy-4-amino-5-chloro-phénylcarbamoyl)-méthyl]-pyridinium ;
- le chlorure de 1-[(2-hydroxy-4-acétylamino-5-chloro-phénylcarbamoyl)-méthyl]-pyridinium ;
- le chlorure de 1-[(2-hydroxy-4-méthoxycarbonylamino-5-chloro-phénylcarbamoyl)-méthyl]-pyridinium ;
- le chlorure de 1-[(3-hydroxy-4-acétylamino-6-chloro-phénylcarbamoyl)-méthyl]-pyridinium ;
- le chlorure de 1-[(3-hydroxy-4-méthoxycarbonylamino-6-chloro-phénylcarbamoyl)-méthyl]-pyridinium ;
- le chlorure de 1-[(2-hydroxy-5-méthoxy-phénylcarbamoyl)-méthyl]-pyridinium ;
- le chlorure de 1-[(2-hydroxy-4-méthyl-5-méthoxy-phénylcarbamoyl)-méthyl]-pyridinium ;
- le chlorure de 1-[(2-hydroxy-4-amino-5-méthoxy-phénylcarbamoyl)-méthyl]-pyridinium;
- le chlorure de 1-[(2-hydroxy-4-acétylamino-5-méthoxy-phénylcarbamoyl)-méthyl]-pyridinium ;
- le chlorure de 1-[(2-hydroxy-4-méthoxycarbonylamino-5-méthoxy-phénylcarbamoyl)-méthyl]-pyridinium ;
- le chlorure de 1-[(3-hydroxy-4-acétylamino-6-méthoxy-phénylcarbamoyl)-méthyl]-pyridinium ;
- le chlorure de 1-[(3-hydroxy-4-méthoxycarbonylamino-6-méthoxyphénylcarbamoyl)-méthyl]-pyridinium ;
- le chlorure de 1-[(2-hydroxy-6-amino-phénylcarbamoyl)-méthyl]-pyridinium ;
- le chlorure de 1-[(2-hydroxy-6-acétylamino-phénylcarbamoyl)-méthyl]-pyridinium ;
- le chlorure de 1-[(2-hdroxy-4,6-diamino-phénylcarbamoyl)-méthyl]-pyridinium ;
- le chlorure de 1-[(2-hydroxy-4-acétylamino-6-amino-phénylcarbamoyl)-méthyl]-pyridinium ;
- le chlorure de 1-[(2-hydroxy-3,5-dichloro-4-méthyl-phénylcarbamoyl)-méthyl]-pyridinium ;
- le chlorure de 1-[(2-hydroxy-3,5-dichloro-4-amino-phénylcarbamoyl)-méthyl]-pyridinium ;
- le chlorure de 1-[(2-hydroxy-3,5-dichloro-4-acétylamino-phénylcarbamoyl)-méthyl]-pyridinium ;
- le chlorure de 1-[(2-hydroxy-3,5-dichloro-4-méthoxycarbonylamino-phényl-carbamoyl)-méthyl]-pyridinium ;
- le chlorure de 1-[(2-hydroxy-3-acétylamino-phénylcarbamoyl)-méthyl]-pyridinium ;
- le chlorure de 1-[(2-hydroxy-phénylcarbamoyl)-méthyl]-1,4-diméthyl-pipérazin-1-ium ;
- le dichlorure de 1-[(2-hydroxy-3-(2-(1,4-diméthyl-pipérazin-1-ium-1-yl)-acétyl)amino-phénylcarbamoyl)-méthyl]-1,4-diméthyl-pipérazin-1-ium ;
- le dichlorure de 1-[(2-hydroxy-4-(2-(1,4-diméthyl-pipérazin-1-ium-1-yl) acétyl)amino-phényl-carbamoyl)-méthyl]-1,4-diméthyl-pipérazin-1-ium ;
- le chlorure de 1-[(2-hydroxy-4-méthyl-phénylcarbamoyl)-méthyl]-1,4-diméthyl-pipérazin-1-ium ;
- le chlorure de 1-[(2-hydroxy-4-amino-phénylcarbamoyl)-méthyl]-1,4-diméthyl-pipérazin-1-ium ;
- le chlorure de 1-[(2-hydroxy-4-acétylamino-phénylcarbamoyl)-méthyl]-1,4-diméthyl-pipérazin-1-ium ;
- le chlorure de 1-[(2-hydroxy-4-méthoxycarbonylamino-phénylcarbamoyl)-méthyl]-1,4-diméthyl-pipérazin-1-ium ;
- le chlorure de 1-[(3-hydroxy-4-acétylamino-phénylcarbamoyl)-méthyl]-1,4-diméthyl-pipérazin-1-ium ;
- le chlorure de 1-[(3-hydroxy-4-méthoxycarbonylamino-phénylcarbamoyl)-méthyl]-1,4-diméthyl-pipérazin-1-ium ;
- le chlorure de 1-[(2-hydroxy-5-chloro-phénylcarbamoyl)-méthyl]-1,4-diméthyl-pipérazin-1-ium ;
- le chlorure de 1-[(2-hydroxy-4-méthyl-5-chloro-phénylcarbamoyl)-méthyl]-1,4-diméthyl-pipérazin-1-ium ;
- le chlorure de 1-[(2-hydroxy-4-amino-5-chloro-phénylcarbamoyl)-méthyl]-1,4-diméthyl-pipérazin-1-ium ;
- le chlorure de 1-[(2-hydroxy-4-acétylamino-5-chloro-phénylcarbamoyl)-méthyl]-1,4-diméthyl-pipérazin-1-ium ;
- le chlorure de 1-[(2-hydroxy-4-méthoxycarbonylamino-5-chloro-phénylcarbamoyl)-méthyl]-1,4-diméthyl-pipérazin-1-ium ;
- le chlorure de 1-[(3-hydroxy-4-acétylamino-6-chloro-phénylcarbamoyl)-méthyl]-1,4-diméthyl-pipérazin-1-ium ;
- le chlorure de 1-[(3-hydroxy-4-méthoxycarbonylamino-6-chloro-phénylcarbamoyl)-méthyl]-1,4-diméthyl-pipérazin-1-ium ;
- le chlorure de 1-[(2-hydroxy-5-méthoxy-phénylcarbamoyl)-méthyl]-1,4-diméthyl-pipérazin-1-ium ;
- le chlorure de 1-[(2-hydroxy-4-méthyl-5-méthoxy-phénylcarbamoyl)-méthyl]-1,4-diméthyl-pipérazin-1-ium ;
- le chlorure de 1-[(2-hydroxy-4-amino-5-méthoxy-phénylcarbamoyl)-méthyl]-1,4-diméthyl-pipérazin-1-ium ;
- le chlorure de 1-[(2-hydroxy-4-acétylamino-5-méthoxy-phénylcarbamoyl)-méthyl]-1,4-diméthyl-pipérazin-1-ium ;
- le chlorure de 1-[(2-hydroxy-4-méthoxycarbonylamino-5-méthoxy-phénylcarbamoyl)-méthyl]-1,4-diméthyl-pipérazin-1-ium ;
- le chlorure de 1-[(3-hydroxy-4-acétylamino-6-méthoxy-phénylcarbamoyl)-méthyl]-1,4-diméthyl-pipérazin-1-ium ;
- le chlorure de 1-[(3-hydroxy-4-méthoxycarbonylamino-6-méthoxy-phénylcarbamoyl)-méthyl]-1,4-diméthyl-pipérazin-1-ium ;
- le chlorure de 1-[(2-hydroxy-6-amino-phénylcarbamoyl)-méthyl]-1,4-diméthyl-pipérazin-1-ium ;
- le chlorure de 1-[(2-hydroxy-6-acétylamino-phénylcarbamoyl)-méthyl]-1,4-diméthyl-pipérazin-1-ium ;
- le chlorure de 1-[(2-hydroxy-4,6-diamino-phénylcarbamoyl)-méthyl]-1,4-diméthyl-pipérazin-1-ium ;
- le chlorure de 1-[(2-hydroxy-4-acétylamino-6-amino-phénylcarbamoyl)-méthyl]-1,4-diméthyl-pipérazin-1-ium;
- le chlorure de 1-[(2-hydroxy-3,5-dichloro-4-méthyl-phénylcarbamoyl)-méthyl]-1,4-diméthyl-pipérazin-1-ium ;
- le chlorure de 1-[(2-hydroxy-3,5-dichloro-4-amino-phénylcarbamoyl)-méthyl]-1,4-diméthyl-pipérazin-1-ium ;
- le chlorure de 1-[(2-hydroxy-3,5-dichloro-4-acétylamino-phénylcarbamoyl)-méthyl]-1,4-diméthyl-pipérazin-1-ium ;
- le chlorure de 1-[(2-hydroxy-3,5-dichloro-4-méthoxycarbonylamino-phénylcarbamoyl)-méthyl]-1,4-diméthyl-pipérazin-1-ium ;
- le chlorure de 1-[(2-hydroxy-3-acétylamino-phénylcarbamoyl)-méthyl]-1,4-diméthyl-pipérazin-1-ium ;
et leurs sels d'addition avec un acide.

23. Utilisation des composés de formule (I') tels que définis à l'une quelconque des revendications 21 à 22 à titre de coupleur pour la teinture d'oxydation des fibres kératiniques.

## Patentansprüche

1. Zusammensetzung zum oxidativen Färben von Keratinfasern, **dadurch gekennzeichnet, dass** sie in einem zum Färben geeigneten Medium enthält:
- mindestens eine Oxidationsbase, und
- mindestens einen Kuppler, der unter den folgenden Verbindungen der Formel (I) und deren Additionssalzen mit einer Säure ausgewählt ist: 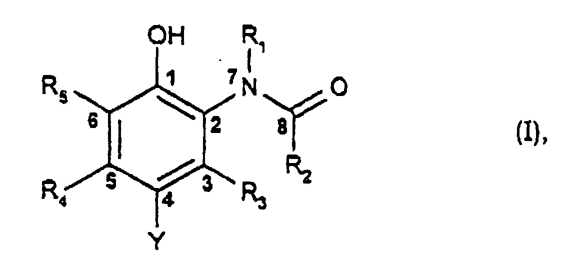
worin bedeuten:
· R₁ Wasserstoff; Methyl, Ethyl, Isopropyl, Allyl, Phenyl, Benzyl, Fluorbenzyl, Hydroxybenzyl, Difluorbenzyl, Trifluorbenzyl, Chlorbenzyl, Brombenzyl, Methoxybenzyl, Dimethoxybenzyl, (Trifluormethoxy)benzyl, 3,4-Methylendioxybenzyl, 6-Chlorpiperonyl, 4-Methylthiobenzyl, 4-Methylsulfonylbenzyl, 4-Acetylaminobenzyl, 4-Carboxybenzyl, 1-Naphtomethyl, 2-Naphthomethyl, 2-Hydroxyethyl, 2-Methoxyethyl oder 2-Ethoxyethyl;
· R₂ Wasserstoff, eine Gruppe Z; eine Gruppe, die aus der Gruppe (G1) ausgewählt ist, die aus den folgenden Gruppen besteht: Methyl, Ethyl, Propyl, Isopropyl, Butyl, Isobutyl, *t*-Butyl, Pentyl, Isopentyl, Neopentyl, Hexyl; Cyclopropyl, Cyclobutyl, Cyclopentyl, Cyclopentylmethyl, 3-Cyclopentylpropyl, Cyclohexyl, 2-Cyclohexylethyl, Norbornan-2-yl, Vinyl, 1-Methylvinyl, 2-Methylvinyl, 2,2-Dimethylvinyl, Allyl, 3-Butenyl; Phenyl, Methylphenyl, Dimethylphenyl, 2,4,6-Trimethylphenyl, 4-Ethylphenyl, (Trifluormethyl)phenyl, Hydroxyphenyl, Methoxyphenyl, Ethoxyphenyl, Acetoxyphenyl, (Trifluormethoxy)phenyl, Aminophenyl, 4-Dimethylaminophenyl, Fluorphenyl, Difluorphenyl, Fluor(trifluormethyl)phenyl, Chlorphenyl, Dichlorphenyl, Bromphenyl, Naphth-1-yl, Naphth-2-yl, (2-Methoxy)naphth-1-yl, Benzyl, 4'-Methoxybenzyl, 2',5'-Dimethoxybenzyl, 3',4'-Dimethoxybenzyl, 4'-Fluorbenzyl, 4'-Chlorbenzyl, Phenethyl, 2-Phenylvinyl, (1-Naphthyl)methyl, (2-Naphthyl)methyl; Tetrahydrofuran-2-yl, Furan-2-yl, 5-Methyl-2-(trifluormethyl)furan-3-yl, 2-Methyl-5-phenylfuran-3-yl, Thiophen-2-yl, (Thiophen-2-yl)methyl, 3-Chlorthiophen-2-yl, 2,5-Dichlorthiophen-3-yl, Benzothiophen-2-yl, 3-Chlorbenzothiophen-2-yl, Isoxazol-5-yl, 5-Methylisoxazol-3-yl, 3,5-Dimethylisoxazol-4-yl, 1,3-Dimethylpyrazol-5-yl, 1-Ethyl-3-methylpyrazol-5-yl, 1-*t*-Butyl-3-methylpyrazol-5-yl, 3-*t*-Butyl-1-methylpyrazol-5-yl, 4-Brom-1-ethyl-3-methylpyrazol-5-yl, Indol-3-ylcarboxy, Pyridinyl, Chlorpyridinyl, Dichlorpyridinyl, 5-(Brom)pyridin-3-yl, Piperazin-2-yl, Chinoxal-2-yl; Fluormethyl, Difluormethyl, Trifluormethyl, 1,1,2,2-Tetrafluorethyl, Pentafluorethyl, Heptafluorpropyl, 1,1,2,2,3,3,4,4-Octafluorbutyl, Nonafluorbutyl, Chlormethyl, Chlorethyl, 1,1-Dimethyl-2-chlorethyl, 1,2-Dichlorethyl, 1-Chlorpropyl, 3-Chlorpropyl, 4-Chlorbutyl, Hydroxymethyl, Methoxymethyl, Phenoxymethyl, (4-Chlorphenoxy)methyl, Benzyloxymethyl, Acetoxymethyl, 1,2-Dihydroxyethyl, 1-Phenoxyethyl, 1-Acetoxyethyl, 2-(2-Carboxyethoxy)ethyl, 1-Fhenoxyethyl, 1-Acetoxyethyl, Methoxycarbonyl, Ethoxycarbonyl, (Methoxycarbonyl)methyl, 2-Carboxyethyl, 2-(Methoxycarbonyl)-ethyl, 2-Carboxycyclopropyl, 2-Carboxycyclohexan; Methoxy, Ethoxy, Propoxy, Isopropoxy, Butoxy, Isobutoxy, Pentoxy, Neopentoxy, Hexyloxy, Cyclopentyloxy, Cyclohexyloxy, Vinyloxy, Allyloxy, Propargyloxy, Chlormethoxy, 1-Chlorethoxy, 2-Methoxyethoxy, 4-Chlorbutoxy, Phenoxy, 4-Methylphenoxy, 4-Fluorphenoxy, 4-Bromphenoxy, 4-Chlorphenoxy, 4-Methoxyphenoxy, Naphth-2-yloxy, Benzyloxy; Amino, Methylamino, Ethylamino, Propylamino, Isopropylamino, Butylamino, Cyclohexylamino, Allylamino, 2-Chlorethylamino, 3-Chlorpropylamino, Carboxymethylamino, Phenylamino, Fluorphenylamino, (Trifluormethyl)phenylamino, Chlorphenylamino, Bromphenylamino, 4-Acetylphenylamino, Methoxyphenylamino, (Trifluormethoxy)phenylamino, Naphth-1-ylamino, Benzylamino, Phenetylamino, Pyrid-3-ylamino, Dimethylamino, 1-Pyrolidinyl und 4-Morpholinyl;
· R₃ und R₄, die gleich oder verschieden sind, Wasserstoff, Halogen; Hydroxy, Amino; eine Gruppe Z; eine nachstehend definierte Gruppe A₁, A₄ oder A₅; oder eine nachstehend definierte Gruppe A₁, A₂, A₃, A₄ oder A₅, die an den Phenolring der Formel (I) über ein Sauerstoffatom oder eine Gruppe -NH-, -Nalkyl(C₁₋₃)-, -O(CO)-, -NH(CO)-, -Nalkyl(C₁₋₃)CO-, -NH[C=NH]-, -NH(CO)NH-, -NH(CO)Nalkyl(C₁₋₃)-, -NH(CO)O-, -NHSO₂-, -NHSO₂NH- oder -NHSO₂Nalkyl(C₁₋₃)- gebunden ist;
- die Gruppe A₁ bedeutet; eine geradkettige oder verzweigte C₁₋₃-Alkylgruppe, die eine oder zwei Doppelbindungen oder eine Dreifachbindung enthalten kann und die unsubstituiert vorliegt oder mit einer Gruppe substituiert ist, die unter den nachfolgend definierten Gruppen A₂, A₄ und A₅ ausgewählt ist, die unsubstituiert vorliegt oder mit einer oder zwei identischen oder voneinander verschiedenen Gruppen substituiert ist, die unter den Gruppen N-Alkyl(C₁₋₃)amino, N-Alkyl(C₁₋₃)-N-alkyl(C₁₋₃)amino, Alkoxy(C₁₋₆), Oxo, Alkoxycarbonyl, Acyloxy, Amid, Acylamino, Ureyl, Sulfoxy, Sulfonyl, Sulfonamido, Sulfonylamino, Brom, Cyano und Carboxy ausgewählt sein können, und die unsubstituiert vorliegt oder mit einer oder mehreren Gruppen Hydroxy, Fluor oder Chlor substituiert sein kann;
- die Gruppe A₂ bedeutet: eine aromatische Gruppe vom Typ Phenyl oder Naphthyl, die gegebenenfalls mit 1 bis 3 Gruppen substituiert sein kann, die identisch oder voneinander verschieden sind und unter den Gruppen Methyl, Trifluormethyl, Ethyl, Isopropyl, Butyl, Pentyl, Fluor, Chlor, Brom, Methoxy, Trifluormethoxy, Ethoxy, Propyloxy, Acetyloxy, Acetyl und Cyano ausgewählt sind;
- die Gruppe A₃ bedeutet: eine heteroaromatische Gruppe, die unter den folgenden Gruppen ausgewählt ist: Furanyl, Thiophenyl, Pyrrolyl, Imidazolyl, Thiazolyl, Oxazolyl, 1,2,3-Triazolyl, 1,2,4-Triazolyl, Isoxazolyl, Isothiazolyl, Pyrazolyl, Pyrazolotriazolyl, Pyrazoloimidazolyl, Pyrrolotriazolyl, Pyrazolopyrimidyl, Pyrazolopyridyl, Pyridyl, Pyrimidyl, Benzoimidazolyl, Benzoxazolyl, Benzothiazolyl, Indolyl, Indolidinyl, Isoindolyl, Indazolyl, Benzotriazolyl, Chinolinyl, Benzoimidazolyl und Benzopyrimidyl, wobei diese Gruppen gegebenenfalls mit 1 bis 3 Gruppen substituiert sein können, die unter den geradkettigen oder verzweigten C₁₋₄-Alkylgruppen, C₁₋₄-Monohydroxyalkyl, C₂₋₄-Polyhydroxyalkyl, Carboxy, Alkoxycarbonyl, Halogen, Amido, Amino und Hydroxy ausgewählt sind;
- die Gruppe A₄ bedeutet: C₃₋₇-Cycloalkyl, Norbornanyl, wobei sie gegebenenfalls eine Doppelbindung aufweist und gegebenenfalls mit 1 oder 2 Gruppen substituiert ist, die unter den geradkettigen oder verzweigten C₁₋₄-Alkylgruppen, C₁₋₄-Monohydroxyalkyl, C₂₋₄-Polyhydroxyalkyl, Carboxy, Alkoxycarbonyl, Halogen, Amido, Amino und Hydroxy ausgewählt sind; und
- die Gruppe A₅ bedeutet: einen Heterocyclus, der unter den folgenden Gruppen ausgewählt ist: Dihydrofuranyl, Tetrahydrofuranyl, Butyrolact-on-yl, Dihydrothiophenyl, Tetrahydrothiophenyl, Tetrahydrothiophen-on-yl, Iminothiolan, Dihydropyrrolyl, Pyrrolidinyl, Pyrrolidin-on-yl, Imidazolidinon-yl, Imidazolidinthionyl, Oxazolidinyl, Oxazolidin-on-yl, Oxazolanthion, Thiazolidinyl, Isothiazol-on-yl, Mercaptothiazolinyl, Pyrazolidin-on-yl, Iminothiolan, Dioxolanyl, Pentalacton, Dioxanyl, Dihydropyridinyl, Piperidinyl, Pentalactam, Morpholinyl, Pyrazoli(di)nyl, Pyrimi(di)nyl, Pyrazinyl, Piperazinyl und Azepinyl;
· R₅ Wasserstoff, Halogen; eine Gruppe Z; eine Gruppe A₁, A₄ oder A₅; eine Gruppe A₁, A₂, A₃, A₄ oder A₅, die an den Phenolring der Verbindungen der Formel (I) über ein Sauerstoffatom, ein Schwefelatom oder eine der folgenden Gruppen gebunden ist: -NH-, -Nalkyl(C₁₋₃)-, -O(CO)-, -NH(CO)-, -Nalkyl(C₁₋₃)(CO)-, -NH[C=NH]-, -NH(CO)NH-, -NH(CO)Nalkyl(C₁₋₃)- oder -NH(CO)O-;
· Y Wasserstoff, Halogen; eine Gruppe -OCH₂COOH, -OCH₂COOCH₃, -OCH₂COOC₂H₅, -OR₆, -SR₆ oder -NH-SO₂R₆, worin R₆ eine geradkettige oder verzweigte C₁₋₆-Alkylgruppe bedeutet (wobei der Zweig oder die Zweige einen oder mehrere 3- bis 6-gliedrige Ringe bilden können), die unsubstituiert vorliegt oder mit einer oder mehreren Gruppen substituiert ist, die unter Halogen, Hydroxy, C₁₋₄-Alkoxy, Amino und C₁₋₄-Aminoalkyl ausgewählt sind; eine Phenylgruppe, die gegebenenfalls mit einer oder zwei Gruppen substituiert ist, die unter C₁₋₄-Alkyl, Trifluormethyl, Carboxy, C₁₋₄-Alkoxycarbonyl, Halogen, Hydroxy, C₁₋₄-Alkoxy, Amino und C₁₋₄-Aminoalkyl ausgewählt sind; oder eine Benzylgruppe; mit der Maßgabe, dass Y nicht -NH-SO₂R₆ bedeuten kann, wenn R₃ eine Hydroxygruppe ist;
· Z eine kationische Gruppe der folgenden Formel (II): 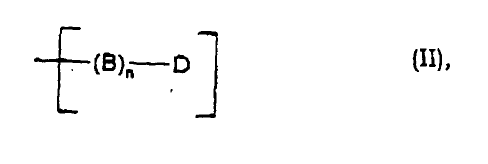 worin bedeuten:
- B eine geradkettige oder verzweigte (wobei der Zweig oder die Zweige einen oder mehrere 3- bis 7-gliedrige Ringe bilden können) Gruppe mit 1 bis 15 Kohlenstoffatomen, die eine oder mehrere Doppelbindungen und/oder eine oder mehrere Dreifachbindungen enthalten kann, wobei die Doppelbindungen gegebenenfalls zu aromatischen Gruppen führen, und in der ein oder mehrere Kohlenstoffatome durch ein Sauerstoffatom, Stickstoffatom, Schwefelatom oder eine Gruppe -SO₂ ersetzt sein können; und in der ein oder mehrere Kohlenstoffatome unabhängig voneinander mit einem oder mehreren Halogenatomen oder einer oder mehreren Gruppen Z substituiert sein können; wobei die Gruppe B keine Peroxidbindung und keine Diazogruppe, Nitrogruppe oder Nitrosogruppe enthält;
- n 0 oder 1,
- D eine Gruppe, die unter den folgenden kationischen Gruppen ausgewählt ist: Imidazolinium, Thiazolinium, Oxazolinium, Pyrrolinium, 1,2,3-Triazolinium, 1,2,4-Triazolinium, Isoxazolinium, Isothiazolinium, Imidazolinium, Thiazolidinium, Pyrazolinium, Pyrazolidinium, Oxazolidinium, Pyrazolotriazolinium, Pyrazoloimidazolinium, Pyrrolotriazolinium, Pyrazolopyrimidinium, Pyrazolopyridinium, Pyridinium, Pyrimidinium, Pyrazinium, Triazinium, Benzimidazolinium, Benzoxazolinium, Benzothiazolinium, Indolinium, Indolidinium, Isoindolinium, Indazolinium, Benzotriazolinium, Chinolinium, Tetrahydrochinolinium, Benzoimidazolidinium, Benzopyrimidinium, Tetra-alkyl(C₁₋₄)-ammonium, Polyhydroxytetra-alkyl(C₁₋₄)ammonium, Dialkylpiperidinium, Dialkylpyrrolidinium, Dialkylmorpholinium, Dialkylthiomorpholinium, Dialkylpiperazinium, Azepinium und 1,4-Diazabicyclo-[2,2,2]octanium, mit der Maßgabe, dass mindestens eine der Gruppen R₁ bis R₅ eine Gruppe Z bedeutet.

2. Zusammensetzung nach Anspruch 1, **dadurch gekennzeichnet, dass** die Gruppe R₂ bedeutet: eine Gruppe, die aus der Gruppe (G2) ausgewählt ist, die aus Methyl, Ethyl, Propyl, Allyl, Phenyl, Tetrahydrofuran-2-yl, Furan-2-yl, Thiophen-2-yl, Pyridinyl, Piperazin-2-yl, Fluormethyl, Chlormethyl, 2-Chlorethyl, Methoxymethyl, Acetoxymethyl, 1,2-Dihydroxyethyl, Methoxycarbonyl, 2-Carboxyethyl, Methoxy, Ethoxy, Propoxy, Allyloxy, 2-Chlorethoxy, 2-Methoxyethoxy, Amino, Ethylaxnino, Allylamino, 2-Chlorethylamino, Pyridylamino, Dimethylamino, 1-Pyrolidinyl und 4-Morpholinyl besteht; oder eine Gruppe -D', -E-D', -O-E-D', -NH-E-D', wobei -E- eine Kette -(CH₂)_{q}- bedeutet, worin q 1 oder 2 ist, und D' eine Gruppe bedeutet, die unter 3-Methylimidazolidinium-1-yl, 3-(2-Hydroxyethyl)-imidazolidinium-1-yl, 1,2,4-Triazolinium-1-yl, 1,2,4-Triazolinium-4-yl, N-Alkyl(C₁₋₄)-pyridinium-2-yl, N-Alkyl(C₁₋₄)pyridinium-3-yl, N-Alkyl(C₁₋₄)-pyridinium-4-yl, N-(2-Hydroxyethyl)pyridinium-2-yl, N-(2-Hydroxyethyl)pyridinium-3-yl, N-(2-Hydroxyethyl)pyridinium-4-yl, Pyridinium-1-yl, Trialkyl(C₁₋₄)ammonium-N-yl, 1-Methylpiperidinium-1-yl und 1,4-Dimethylpiperazinium-1-yl ausgewählt ist.

3. Zusammensetzung nach Anspruch 2, **dadurch gekennzeichnet, dass** die Gruppe R₂ Methyl, Methoxymethyl, 2-Carboxyethyl, Methoxy, Amino, Ethylamino, 1-Pyrolidinyl; eine Gruppe -D', -E-D', -O-E-D' oder -NH-E-D' bedeutet, worin -E- eine Kette -(CH₂)_{q} mit q = 1 oder 2 bedeutet.

4. Zusammensetzung nach Anspruch 1, **dadurch gekennzeichnet, dass** die Gruppe R₃ Wasserstoff oder Chlor; eine Gruppe Z; Methyl, Hydroxymethyl, Methoxymethyl, 1-Hydroxyethyl, Aminomethyl, Methylaminomethyl, Hydroxy, Methoxy, Acetoxy, Amino, Methylamino oder 2-Hydroxyethylamino; eine Gruppe -NH(CO)R₁₂, wobei R₁₂ eine Gruppe aus der Gruppe (G1) bedeutet; eine Gruppe -NHSO₂R₁₃, wobei R₁₃ eine Gruppe aus der Gruppe (G3) bedeutet, die aus Methyl, Trifluormethyl, Ethyl, 2-Chlorethyl, Propyl, 3-Chlorpropyl, Isopropyl, Butyl, Thiophen-2-yl, Hydroxy, Ethoxy und Dimethylamino besteht.

5. Zusammensetzung nach Anspruch 4, **dadurch gekennzeichnet, dass** R₃ Wasserstoff; Methyl, Hydroxymethyl, Aminomethyl, Hydroxy, Methoxy, Amino, Methylamino; eine Gruppe Methansulfonylamino; Ethansulfonylamin; Dimethylamino-sulfonylamino; eine Gruppe -NH(CO)R₁₄, wobei R₁₄ eine Gruppe aus der Gruppe (G2) bedeutet; oder eine Gruppe -O-E-D', -NH-E-D', -CH₂O-E-D', -CH₂NH-E-D', -CH₂NH(CO)-D', -NH(CO)-D', -NH(CO)-E-D', -NH(CO)O-E-D', -NH(CO)NH-E-D' oder -NH(SO₂)-E-D' bedeutet.

6. Zusammensetzung nach Anspruch 1, **dadurch gekennzeichnet, dass** die Gruppe R₄ Wasserstoff oder Chlor; eine Gruppe Z; Methyl, Ethyl, Hydroxymethyl, Methoxymethyl, Aminomethyl, Methylaminomethyl, Hydroxy, Methoxy, Acetoxy, Amino, Methylamino, N-Piperidino oder N-Morpholino; eine Gruppe -NH(CO)R₁₅, wobei R₁₅ eine Gruppe aus der Gruppe (G1) bedeutet; oder eine Gruppe -NHSO₂R₁₆ bedeutet, wobei R₁₆ eine Gruppe aus der Gruppe (G3) ist.

7. Zusammensetzung nach Anspruch 6, **dadurch gekennzeichnet, dass** die Gruppe R₄ Wasserstoff oder Chlor; Methyl, Hydroxymethyl, Aminomethyl, Hydroxy, Methoxy, Amino oder Methylamino; eine Gruppe Methansulfonylamino; Ethansulfonylamino; Dimethylaminosulfonylamin; eine Gruppe -NH(CO)R₁₇, wobei R₁₇ eine Gruppe aus (G2) bedeutet; oder eine Gruppe -O-E-D', -NH-E-D', -CH₂O-E-D', -CH₂NH-E-D', -CH₂NH(CO)-D', -NH(CO)-D', -NH(CO)-E-D', -NH(CO)O-E-D', -NH(CO)NH-E-D' oder -NH(SO₂)-E-D' bedeutet.

8. Zusammensetzung nach Anspruch 1, **dadurch gekennzeichnet, dass** die Gruppe R₅ Wasserstoff, Chlor, Fluor oder Brom; eine Gruppe Z; Methyl, Trifluormethyl, Allyl, Hydroxymethyl, Methoxymethyl, 1-Hydroxyethyl, Aminomethyl, Methylaminomethyl, Methoxy, Acetoxy oder Methylamino; oder eine Gruppe NH(CO)R₁₈ bedeutet, wobei R₁₈ eine Gruppe aus der Gruppe (G1) ist.

9. Zusammensetzung nach Anspruch 8, **dadurch gekennzeichnet, dass** R₅ Wasserstoff, Chlor oder Fluor; Methyl, Hydroxymethyl, Aminomethyl, Methoxy oder Methylamino; eine Gruppe -NH(CO)R₁₉, wobei R₁₉ eine Gruppe aus der in Anspruch 7 definierten Gruppe (G2) ist; oder eine Gruppe -O-E-D', -NH-E-D',-CH₂O-E-D', -CH₂NH-E-D', -CH₂NH(CO)-D', -NH(CO)-D', -NH(CO)-E-D', -NH(CO)O-E-D' oder -NH(CO)NH-E-D' bedeutet.

10. Zusammensetzung nach einem der vorhergehenden Anspräche, **dadurch gekennzeichnet, dass** Y ausgewählt ist unter Wasserstoff, Chlor, Fluor oder Brom; Methoxy, Ethoxy, Propoxy, Benzyloxy, Phenoxy, -OCH₂CH₂OCH₃; -OCH₂CH₂OCH₃; -OCH₂CH₂N(CH₃)₂; -OCH₂(CO)OH, -OCH₂(CO)OCH₃, -OCH₂(CO)OC₂H₅, -SCH₂CH₂CO₂H oder -NHSO₂CH₃, mit der Maßgabe, dass Y keine Gruppe -NHSO₂CH₃ bedeuten kann, wenn R₃ Hydroxy bedeutet.

11. Zusammensetzung nach Anspruch 1, **dadurch gekennzeichnet, dass** D 3-Methylimidazolidinium-1-yl, 3-(2-Hydroxyethyl)-imidazolidinium-1-yl, 1,2,4-Triazolinium-1-yl, 1,2,4-Triazolinium-4-yl, N-Alkyl(C₁₋₄)pyridin-2-yl, N-Alkyl(C₁₋₄)pyridin-3-yl, N-Alkyl(C₁₋₄)pyridin-4-yl, N-(2-Hydroxyethyl)pyridin-2-yl, N-(2-Hydroxyethyl)pyridin-3-yl, N-(2-Hydroxyethyl)pyridin-4-yl, Pyridin-1-yl, Trialkyl(C₁₋₄)ammonium-N-yl, 1-Methylpiperidinium-1-yl, Thiazolinium-3-yl oder 1,4-Dimethylpiperazinium-1-yl bedeutet.

12. Zusammensetzung nach einem der vorhergehenden Ansprüche, **dadurch gekennzeichnet, dass** die Verbindung(en) der Formel (I) unter den Verbindungen ausgewählt sind, worin bedeuten;
i)
- R₁ Wasserstoff;
- R₂ -D', -E-D', -O-E-D', -NH-E-D' (wie oben definiert); oder eine Gruppe, die aus der Gruppe (G4) ausgewählt ist, welche aus Methyl, Methoxymethyl, 2-Carboxyethyl, Methoxy, Amino, Ethylamino und 1-Pyrolidinyl besteht;
- R₃ Hydroxy, Amino oder Methylamino; eine Gruppe -NH(CO)R₂₀, wobei R₂₀ eine Gruppe bedeutet, die aus der oben definierten Gruppe (G4) ausgewählt ist; Methansulfonylamino, Ethansulfonylamino oder Dimethylaminosulfonylamino; eine Gruppe -O-E-D', -NH-E-D',-NH(CO)-D', -NH(CO)-E-D', -NH(CO)O-E-D', -NH(CO)NH-E-D' oder -NH(SO₂)-E-D' (wie oben definiert);
- R₄ Wasserstoff, Chlor oder Methyl;
- R₅ Wasserstoff, Chlor, Fluor oder Methyl;
- Y Wasserstoff, Chlor; Methoxy oder -OCH₂(CO)OCH₃; mit der Maßgabe, dass mindestens eine der Gruppen R₂ und R₃ eine Gruppe Z aufweist;
ii)
- R₁ Wasserstoff;
- R₂ eine Gruppe -D', -E-D', -O-E-D' oder -NH-E-D' (wie oben definiert); oder eine Gruppe, die zur oben definierten Gruppe (G4) gehört;
- R₃ Wasserstoff oder Methyl;
- R₄ Hydroxy, Amino, Methylamino, Methansulfonylamino, Ethansulfonylamino oder Dimethylaminosulfonylamino; eine Gruppe -NH(CO)R₂₁, wobei R₂₁ eine Gruppe bedeutet, die zur oben definierten Gruppe (G4) gehört; oder eine Gruppe -O-E-D', -NH-E-D', -NH(CO)-D', -NH(CO)-E-D', -NH(CO)O-E-D', -NH(CO)NH-E-D' oder -NH(SO₂)-E-D' (wie oben definiert);
- R₅ Wasserstoff, Chlor, Fluor; Methyl, Methoxy oder Methylamino;
- Y Wasserstoff, Chlor, Methoxy oder -OCH₂(CO)OCH₃; mit der Maßgabe, dass mindestens eine der Gruppen R₂ und R₄ eine Gruppe Z aufweist;
iii)
- R₁ Wasserstoff;
- R₂ eine Gruppe, die zur oben definierten Gruppe (G4) gehört; oder eine Gruppe -D', -E-D', -O-E-D', -NH-E-D' (wie oben definiert);
- R₃ Wasserstoff oder Methyl;
- R₄ Wasserstoff oder Chlor; Methyl; Methoxy oder Methylamino;
- R₅ -NH(CO)R₂₂, wobei R₂₂ eine Gruppe bedeutet, die zur oben definierten Gruppe (G4) gehört; oder eine Gruppe -O-E-D', -NH-E-D', -NH(CO)-D', -NH(CO)-E-D', -NH(CO)O-E-D' oder -NH(CO)NH-E-D' (wie oben definiert);
- Y Wasserstoff oder Chlor; oder eine Gruppe Methoxy oder -OCH₂(CO)OCH₃; mit der Maßgabe, dass mindestens eine der Gruppen R₂ und R₅ eine Gruppe Z aufweist;
iv)
- R₁ Wasserstoff;
- R₂ eine Gruppe -D', -E-D', -O-E-D' oder -NH-E-D' (wie oben definiert);
- R₃ Wasserstoff oder Methyl;
- R₄ Wasserstoff, Chlor oder Methyl;
- R₅ Wasserstoff, Chlor oder Fluor; oder eine Gruppe Methyl;
- Y Wasserstoff oder Chlor; oder eine Gruppe Methoxy oder -OCH₂(CO)OCH₃.

13. Zusammensetzung nach einem der vorhergehenden Ansprüche, **dadurch gekennzeichnet, dass** die Verbindung(en) der Formel (I) unter den folgenden Verbindungen ausgewählt sind:
- 3-[(2-Hydroxy-phenylcarbamoyl)-methyl]-1-methyl-3H-imidazol-1-iumchlorid;
- 3-[(2-Hydroxy-3-(2-(3-methyl-1H-imidazol-3-ium-1-yl)-acetylamino)-phenylcarbamoyl)-methyl]-1-methyl-3H-imidazol-1-iumdichlorid;
- 3-[(2-Hydroxy-4-(2-(3-methyl-1H-imidazol-3-ium-1-yl)-acetylamino)-phenylcarbamoyl)-methyl]-1-methyl-3H-imidazol-1-iumdichlorid;
- 3-[(2-Hydroxy-4-methyl-phenylcarbamoyl)-methyl]-1-methyl-3H-imidazol-1-iumchlorid;
- 3-[(2-Hydroxy-4-amino-phenylcarbamoyl)-methyl]-1-methyl-3H-imidazol-1-iumchlorid;
- 3-[(2-Hydroxy-4-acetylamino-phenylcarbamoyl)-methyl]-1-methyl-3H-imidazol-1-iumchlorid;
- 3-[(2-Hydroxy-4-methoxycarbonylamino-phenylcarbamoyl)-methyl)-1-methyl-3H-imidazol-1-iumchlorid;
- 3-[(3-Hydroxy-4-acetylamino-phenylcarbamoyl)-methyl]-1-methyl-3H-imidazol-1-iumchlorid;
- 3-[(3-Hydroxy-4-methoxycarbonylamino-phenylcarbamoyl)-methyl)-1-methyl-3H-imidaxol-1-iumchlorid;
- 3-[(2-Hydroxy-5-chlor-phenylcarbamoyl)-methyl]-1-methyl-3H-imidazol-1-iumchlorid;
- 3-[(2-Hydroxy-4-methyl-5-chlor-phenylcarbamoyl)-methyl]-1-methyl-3H-imidazol-1-iumchlorid;
- 3-[(2-Hydroxy-4-amino-5-Chlor-phenylcarbamoyl)-methyl]-1-methyl-3H-imidazol-1-iumchlorid;
- 3-[(2-Hydroxy-4-acetylamino-5-chlor-phenylcarbamoyl)-methyl]-1-methyl-3H-imidazol-1-iumchlorid;
- 3-[(2-Hydroxy-4-methoxycarbonylamino-5-Chlor-phenylcarbamoyl)-methyl]-1-methyl-3H-imidazol-1-iumchlorid;
- 3-[(3-Hydroxy-4-acetylamino-5-chlor-phenylcarbamoyl)-methyl]-1-methyl-3H-imidazol-1-iumchlorid;
- 3-[(3-Hydroxy-4-methoxycarbonylamino-6-chlor-phenylcarbamoyl)-methyl]-1-methyl-3H-imidazol-1-iumchlorid;
- 3-[(2-Hydroxy-5-methoxy-phenylcarbamoyl)-methyl]-1-methyl-3H-imidazol-1-iumchlorid;
- 3-[(2-Hydroxy-4-methyl-5-methoxy-phenylcarbamoyl)-methyl]-1-methyl-3H-imidazol-1-iumchlorid;
- 3-[(2-Hydroxy-4-amino-5-methoxy-phenylcarbamoyl)-methyl]-1-methyl-3H-imidazol-1-iumchlorid;
- 3-[(2-Hydroxy-4-acetylamino-5-methoxy-phenylcarbamoyl)-methyl]-1-methyl-3H-imidazol-1-iumchlorid;
- 3-[(2-Hydroxy-4-methoxycarbonylamino-5-methoxy-phenylcarbamoyl)-methyl]-1-methyl-3H-imidazol-1-iumchlorid;
- 3-[(3-Hydroxy-4-acetylamino-6-methoxy-phenylcarbamoyl)-methyl]-1-methyl-3H-imidazol-1-iumchlorid;
- 3-[(3-Hydroxy-4-methoxycarbonylamino-6-methoxy-phenylcarbamoyl)-methyl]-1-methyl-3H-imidazol-1-iumchlorid;
- 3-[(2-Hydroxy-6-amino-phenylcarbamoyl)-methyl]-1-methyl-3H-imidazol-1-iumchlorid;
- 3-[(2-Hydroxy-6-acetylamino-phenylcarbamoyl)-methyl]-1-methyl-3H-imidazol-1-iumchlorid;
- 3-[(2-Hydroxy-4,6-diamino-phenylcarbamoyl)-methyl]-1-methyl-3H-imidazol-1-iumchlorid;
- 3-[(2-Hydroxy-4-acetylamino-6-amino-phenylcarbamoyl)-methyl]-1-methyl-3H-imidazol-1-iumchlorid;
- 3-[(2-Hydroxy-3,5-dichlor-4-methyl-phenylcarbamoyl)-methyl]-1-methyl-3H-imidazol-1-iumchlorid;
- 3-[(2-Hydroxy-3,5-dichlor-4-amino-phenylcarbamoyl)-methyl]-1-methyl-3H-imidazol-1-iumchlorid;
- 3-[(2-Hydroxy-3,5-dichlor-4-acetylamino-phenylcarbamoyl)-methyl]-1-methyl-3H-imidazol-1-iumchlorid;
- 3-[(2-Hydroxy-3,5-dichlor-4-methoxycarbonylamino-phenylcarbamoyl)-methyl]-1-methyl-3H-imidazol-1-iumchlorid;
- 3-[(2-Hydroxy-3-acetylamino-phenylcarbamoyl)-methyl]-1-methyl-3H-imidazol-1-iumchlorid;
- 1-[(2-Hydroxy-phenylcarbamoyl)-methyl]-pyridiniumchlorid;
- 1-[(2-Hydroxy-3-(2-(pyridinium-1-yl)acetylamino)-phenylcarbamoyl)-methyl)-pyridiniumdichlorid;
- 1-[(2-Hydroxy-4-(2-(pyridinium-1-yl)acetylamino)-phenylcarbarnoyl)-methyl]-pyridiniumdichlorid;
- 1-[(2-Hydroxy-4-methyl-phenylcarbamoyl)-methyl]-pyridiniumchlorid;
- 1-[(2-Hydroxy-4-amino-phenylcarbamoyl)-methyl]-pyridiniumchlorid;
- 1-[(2-Hydroxy-4-acetylamino-phenylcarbamoyl)-methyl]-pyridiniumchlorid;
- 1-[(2-Hydroxy-4-methoxycarbonylamino-phenylcarbamoyl)-methyl]-pyridiniumchlorid;
- 1-[(3-Hydroxy-4-acetylamino-phenylcarbamoyl)-methyl]-pyridiniumchlorid;
- 1-[(3-Hydroxy-4-methoxycarbonylamino-phenylcarbamoyl)-methyl]-pyridiniumchlorid;
- 1-[(2-Hydroxy-5-chlor-phenylcarbamoyl)-methyl]-pyridiniumchlorid;
- 1-[(2-Hydroxy-4-methyl-5-chlor-phenylcarbamoyl)-methyl]-pyridiniumchlorid;
- 1-[(2-Hydroxy-4-amino-5-Chlor-phenylcarbamoyl)-methyl]-pyridiniumchlorid;
- 1-[(2-Hydroxy-4-acetylamino-5-chlor-phenylcarbamoyl)-methyl]-pyridiniumchlorid;
- 1-[(2-Hydroxy-4-methoxycarbonylamino-5-chlor-phenylcarbamoyl)-methyl]-pyridiniumchlorid;
- 1-[(3-Hydroxy-4-acetylamino-6-Chlor-phenylcarbamoyl)-methyl]-pyridiniumchlorid;
- 1-[(3-Hydroxy-4-methoxycarbonyl-6-chlor-phenylcarbamoyl)-methyl]-pyridiniumchlorid;
- 1-[(2-Hydroxy-5-methoxy-phenylcarbamoyl)-methyl]-pyridiniumchlorid;
- 1-[(2-Hydroxy-4-methyl-5-methoxy-phenylcarbamoyl)-methyl]-pyridiniumchlorid;
- 1-[(2-Hydroxy-4-amino-5-methoxy-phenylcarbamoyl)-methyl]pyridiniumchlorid;
- 1-[(2-Hydroxy-4-acetylamino-5-methoxy-phenylcarbamoyl)-methyl]pyridiniumchlorid;
- 1-[(2-Hydroxy-4-methoxycaxbonylamino-5-methoxy-phenylcarbamoyl)-methyl]pyridiniumchlorid;
- 1-[(3-Hydroxy-4-acetylamino-6-methoxy-phenylcarbamoyl)-methyl]pyridiniumchlorid;
- 1-[(3-Hydroxy-4-methoxycarbonylamino-6-methoxyphenylcarbamoyl)-methyl]pyridiniumchlorid;
- 1-[(2-Hydroxy-6-amino-phenylcarbamoyl)-methyl]pyridiniumchlorid;
- 1-[(2-Hydroxy-6-acetylamino-phenylcarbamoyl)-methyl]pyridiniumchlorid;
- 1-[(2-Hydroxy-4,6-diamino-phenylcarbamoyl)-methyl]pyridiniumchlorid;
- 1-[(2-Hydroxy-4-acetylamino-6-amino-phenylcarbamoyl)-methyl]pyridiniumchlorid;
- 1-[(2-Hydroxy-3,5-dichlor-4-methyl-phenylcarbamoyl)-methyl]pyridiniumchlorid;
- 1-[(2-Hydroxy-3,5-dichlor-4-amino-phenylcarbamoyl)-methyl]pyridiniumchlorid;
- 1-[(2-Hydroxy-3,5-dichlor-4-acetylamino-phenylcarbamoyl)-methyl]pyridiniumchlorid;
- 1-[(2-Hydroxy-3,5-dichlor-4-methoxycarbonylamino-phenylcarbamoyl)-methyl]pyridiniumchlorid;
- 1-[(2-Hydroxy-3-acetylamino-phenylcarbamoyl)-methyl]pyridiniumchlorid;
- 1-[(2-Hydroxy-phenylcarbamoyl)-methyl]-1,4-dimethylpiperazin-1-iumchlorid;
- 1-[(2-Hydroxy-3-(2-(1,4-dimethyl-piperazin-1-ium-1-yl)-acetyl)amino-phenylcarbamoyl)-methyl]-1,4-dimethylpiperazin-1-iumdichlorid;
- 1-[(2-Hydroxy-4-(2-(1,4-dimethyl-piperazin-1-ium-1-yl)-acetyl)amino-phenylcarbamoyl)-methyl]-1,4-dimethylpiperazin-1-iumdichlorid;
- 1-[(2-Hydroxy-4-methyl-phenylcarbamoyl)-methyl]-1,4-dimethyl-piperazin-1-iumchlorid;
- 1-[(2-Hydroxy-4-amino-phenylcarbamoyl)-methyl]-1,4-dimethyl-piperazin-1-iumchlorid;
- 1-[(2-Hydroxy-4-acetylamino-phenylcarbamoyl)-methyl]-1,4-dimethyl-piperazin-1-iumchlorid;
- 1-[(2-Hydroxy-4-methoxycarbonylamino-phenylcarbamoyl)-methyl]-1,4-dimethyl-piperazin-1-iumchlorid;
- 1-[(3-Hydroxy-4-acetylamino-phenylcarbamoyl)-methyl]-1,4-dimethyl-piperazin-1-iumchlorid;
- 1-[(3-Hydroxy-4-methoxycarbonylamino-phenylcarbamoyl)-methyl]-1,4-dimethyl-piperazin-1-iumchlorid;
- 1-[(2-Hydroxy-5-chlor-phenylcarbamoyl)-methyl]-1,4-dimethyl-piperazin-1-iumchlorid;
- 1-[(2-Hydroxy-4-methyl-5-chlor-phenylcarbamoyl)-methyl]-1,4-dimethyl-piperazin-1-iumchlorid;
- 1-[(2-Hydroxy-4-amino-5-Chlor-phenylcarbamoyl)-methyl]-1,4-dimethyl-piperazin-1-iumchlorid;
- 1-[(2-Hydroxy-4-acetylamino-5-chlor-phenylcarbamoyl)-methyl]-1,4-dimethyl-piperazin-1-iumchlorid;
- 1-[(2-Hydroxy-4-methoxycarbonylamino-5-chlor-phenylcarbamoyl)-methyl]-1,4-dimethyl-piperazin-1-iumchlorid;
- 1-[(3-Hydroxy-4-acetylamino-6-chlor-phenylcarbamoyl)-methyl]-1,4-dimethyl-piperazin-1-iumchlorid;
- 1-[(3-Hydroxy-4-methoxycarbonylamino-6-chlor-phenylcarbamoyl)-methyl]-1,4-dimethyl-piperazin-1-iumchlorid;
- 1-[(2-Hydroxy-5-methoxy-phenylcarbamoyl)-methyl]-1,4-dimethyl-piperazin-1-iumchlorid;
- 1-[(2-Hydroxy-4-methyl-5-methoxy-phenylcarbamoyl)-methyl]-1,4-dimethyl-piperazin-1-iumchlorid;
- 1-[(2-Hydroxy-4-amino-5-methoxy-phenylcarbamoyl)-methyl]-1,4-dimethyl-piperazin-1-iumchlorid;
- 1-[(2-Hydroxy-4-acetylamino-5-methoxy-phenylcarbamoyl)-methyl]-1,4-dimethyl-piperazin-1-iumchlorid;
- 1-[(2-Hydroxy-4-methoxycarbonylamino-5-methoxy-phenylcarbamoyl)-methyl]-1,4-dimethyl-piperazin-1-iumchlorid;
- 1-[(3-Hydroxy-4-acetylamino-6-methoxy-phenylcarbamoyl)-methyl]-1,4-dimethyl-piperazin-1-iumchlorid;
- 1-[(3-Hydroxy-4-methoxycarbonylamino-6-methoxy-phenylcarbamoyl)-methyl]-1,4-dimethyl-piperazin-1-iumchlorid;
- 1-[(2-Hydroxy-6-amino-phenylcarbamoyl)-methyl]-1,4-dimethyl-piperazin-1-iumchlorid;
- 1-[(2-Hydroxy-6-acetylamino-phenylcarbamoyl)-methyl]-1,4-dimethyl-piperazin-1-iumchlorid;
- 1-[(2-Hydroxy-4,6-diamino-phenylcarbamoyl)-methyl]-1,4-dimethyl-piperazin-1-iumchlorid;
- 1-[(2-Hydroxy-4-acetylamino-6-amino-phenylcarbamoyl)-methyl]-1,4-dimethyl-piperazin-1-iumchlorid;
- 1-[(2-Hydroxy-3,5-dichlor-4-methyl-phenylcarbamoyl)-methyl]-1,4-dimethyl-piperazin-1-iumchlorid;
- 1-[(2-Hydroxy-3,5-dichlor-4-amino-phenylcarbamoyl)-methyl]-1,4-dimethyl-piperazin-1-iumchlorid;
- 1-[(2-Hydroxy-3,5-dichlor-4-acetylamino-phenylcarbamoyl)-methyl]-1,4-dimethyl-piperazin-1-iumchlorid;
- 1-[(2-Hydroxy-3,5-dichlor-4-methoxycarbonylamino-phenylcarbamoyl)-methyl]-1,4-dimethyl-piperazin-1-iumchlorid;
- 1-[(2-Hydroxy-3-acetylamino-phenylcarbamoyl)-methyl]-1,4-dimethyl-piperazin-1-iumchlorid;
und deren Additionssalze mit einer Säure.

14. Zusammensetzung nach einem der vorhergehenden Ansprüche, **dadurch gekennzeichnet, dass** die Verbindung(en) der Formel (I) und/oder ihr(e) Additionssalz(e) mit einer Säure 0,0005 bis 12 Gew.-% des Gesamtgewichts der Farbmittelzusammensetzung ausmachen.

15. Zusammensetzung nach einem der vorhergehenden Ansprüche, **dadurch gekennzeichnet, dass** die Oxidationsbase(n) unter den p-Phenylendiaminen, Bisphenylalkylendiaminen, p-Aminophenolen, o-Aminophenolen und den heterocyclischen Basen ausgewählt sind.

16. Zusammensetzung nach einem der vorhergehenden Ansprüche, **dadurch gekennzeichnet, dass** sie einen oder mehrere zusätzliche Kuppler, die unter den m-Phenylendiaminen, m-Aminophenolen, m-Dihydroxybenzolen und den heterocyclischen Kupplern ausgewählt sind, und ihre Additionssalze mit einer Säure und/oder einen oder mehrere Direktfarbstoffe enthält.

17. Zusammensetzung nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

18. Verfahren zum oxidativen Färben von Keratinfasern, **dadurch gekennzeichnet, dass** auf die Fasern mindestens eine Farbmittelzusammensetzung nach einem der Ansprüche 1 bis 17 aufgetragen wird und die Farbe bei einem sauren, neutralen oder alkatischen pH-Wert mit einem Oxidationsmittel entwickelt wird, das kurz vor der Anwendung zu der Farbmittelzusammensetzung gegeben wird oder das in einer oxidierenden Zusammensetzung vorliegt, die gleichzeitig oder getrennt davon anschließend aufgetragen wird.

19. Verfahren nach Anspruch 18, **dadurch gekennzeichnet, dass** das Oxidationsmittel unter Wasserstoffperoxid, Harnstoffperoxid, Alkalimetallbromaten, Salzen von Persäuren und Enzymen ausgewählt ist.

20. Vorrichtung mit mehreren Abteilungen oder "Kit" zum Färben mit mehreren Abteilungen, wobei eine erste Abteilung eine Farbmittelzusammensetzung nach einem der Ansprüche 1 bis 17 und eine zweite Abteilung eine oxidierende Zusammensetzung enthält.

21. Verbindungen der folgenden Formel (I') und deren Additionssalze mit einer Säure: 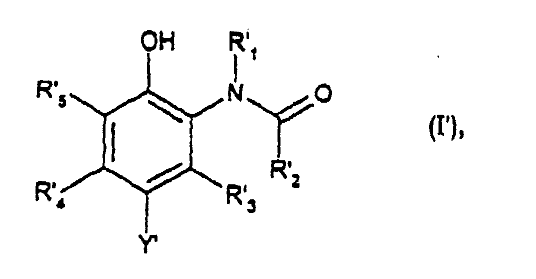 worin die Gruppen R'₁, R'₂, R'₃, R'₄, R'₅ und Y die in einem der Ansprüche 1 bis 20 für die Gruppen R₁, R₂, R₃, R₄, R₅ und Y angegebenen Bedeutungen aufweisen können, wobei mindestens eine der Gruppen R'₁ bis R'₅ eine Gruppe Z' bedeutet, die die für die Gruppe Z in Anspruch 1 angegebenen Bedeutungen aufweisen kann; wobei die folgenden Verbindungen ausgenommen sind: 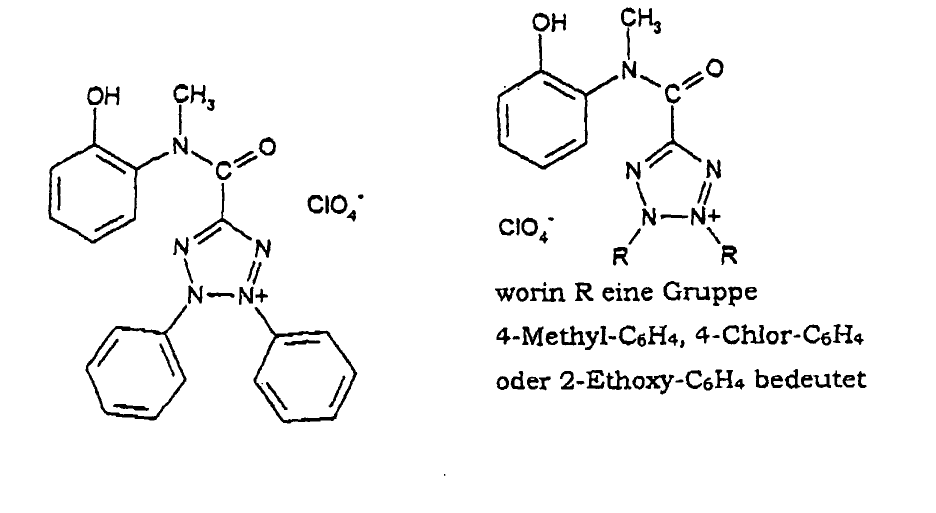 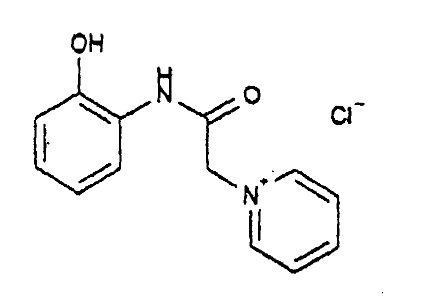 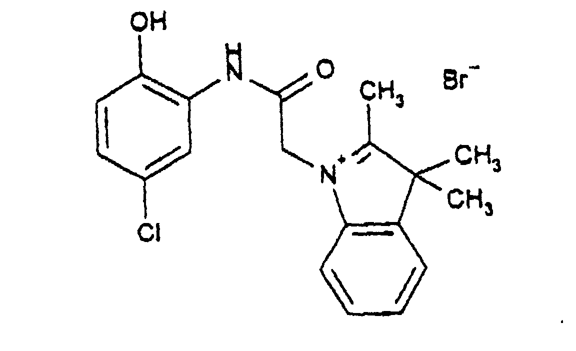 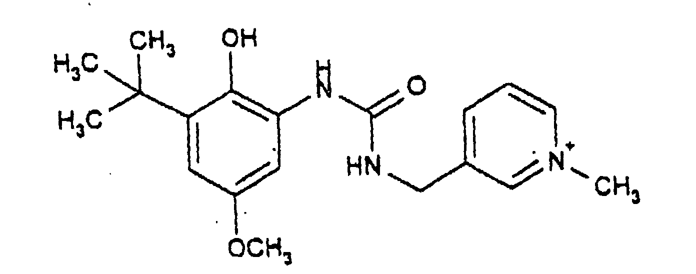 und

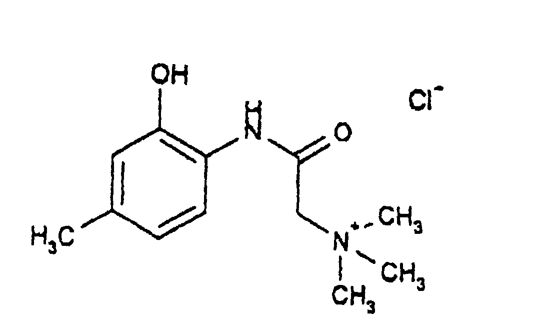

22. Verbindungen nach Anspruch 21, **dadurch gekennzeichnet, dass** sie unter den folgenden Verbindungen ausgewählt sind:
- 3-[(2-Hydroxy-phenylcarbamoyl)-methyl]-1-methyl-3Himidazol-1-iumchlorid;
- 3-[(2-Hydroxy-3-(2-(3-methyl-1H-imidazol-3-ium-1-yl)-acetylamino)-phenylcarbamoyl)-methyl]-1-methyl-3H-imidazol-1-iumdichlorid;
- 3-[(2-Hydroxy-4-(2-(3-methyl-1H-imidazol-3-ium-1-yl)-acetylamino)-phenylcarbamoyl)-methyl]-1-methyl-3H-imidazol-1-iumdichlorid;
- 3-[(2-Hydroxy-4-methyl-phenylcarbamoyl)-methyl]-1-methyl-3H-imidazol-1-iumchlorid;
- 3-[(2-Hydroxy-4-amino-phenylcarbamoyl)-methyl]-1-methyl-3H-imidazol-1-iumchlorid;
- 3-[(2-Hydroxy-4-acetylamino-phenylcarbamoyl)-methyl]-1-methyl-3H-imidazol-1-iumchlorid;
- 3-[(2-Hydroxy-4-methoxycarbonylamino-phenylcarbamoyl)-methyl]-1-methyl-3H-imidazol-1-iumchlorid;
- 3-[(3-Hydroxy-4-acetylamino-phenylcarbamoyl)-methyl]-1-methyl-3H-imidazol-1-iumchlorid;
- 3-[(3-Hydroxy-4-methoxycarbonylamino-phenylcarbamoyl)-methyl]-1-methyl-3H-imidazol-1-iumchlorid;
- 3-[(2-Hydroxy-5-chlor-phenylcarbamoyl)-methyl]-1-methyl-3H-imidazol-1-iumchlorid;
- 3-[(2-Hydroxy-4-methyl-5-chlor-phenylcarbamoyl)-methyl]-1-methyl-3H-imidazol-1-iumchlorid;
- 3-[(2-Hydroxy-4-amino-5-chlor-phenylcarbamoyl)-methyl]-1-methyl-3H-imidazol-1-iumchlorid;
- 3-[(2-Hydroxy-4-acetylamino-5-Chlor-phenylcarbamoyl)-methyl]-1-methyl-3H-imidazol-1-iumchlorid;
- 3-[(2-Hydroxy-4-methoxycarbonylamino-5-chlor-phenylcarbamoyl)-methyl]-1-methyl-3H-imidazol-1-iumchlorid;
- 3-[(3-Hydroxy-4-acetylamino-5-chlor-phenylcarbamoyl)-methyl]-1-methyl-3H-imidazol-1-iumchlorid;
- 3-[(3-Hydroxy-4-methoxycarbonylamino-6-Chlor-phenylcarbamoyl)-methyl]-1-methyl-3H-imidazol-1-iumchlorid;
- 3-[(2-Hydroxy-5-methoxy-phenylcarbamoyl)-methyl]-1-methyl-3H-imidazol-1-iumchlorid;
- 3-[(2-Hydroxy-4-methyl-5-methoxy-phenylcarbamoyl]-methyl]-1-methyl-3H-imidazol-1-iumchlorid;
- 3-[(2-Hydroxy-4-amino-5-methoxy-phenylcarbamoyl)-methyl]-1-methyl-3H-imidazol-1-iumchlorid;
- 3-[(2-Hydroxy-4-acetylamino-5-methoxy-phenylcarbamoyl)-methyl]-1-methyl-3H-imidazol-1-iumchlorid;
- 3-[(2-Hydroxy-4-methoxycarbonylamino-5-methoxy-phenylcarbamoyl)-methyl]-1-methyl-3H-imidazol-1-iumchlorid;
- 3-[(3-Hydroxy-4-acetylamino-6-methoxy-phenylcarbamoyl)-methyl]-1-methyl-3H-imidazol-1-iumchlorid;
- 3-[(3-Hydroxy-4-methoxycarbonylamino-6-methoxy-phenylcarbamoyl)-methyl]-1-methyl-3H-imidazol-1-iumchlorid;
- 3-[(2-Hydroxy-6-amino-phenylcarbamoyl)-methyl]-1-methyl-3H-imidazol-1-iumchlorid;
- 3-[(2-Hydroxy-6-acetylamino-phenylcarbamoyl)-methyl]-1-methyl-3H-imidazol-1-iumchlorid;
- 3-[(2-Hydroxy-4,6-diamino-phenylcarbamoyl)-methyl]-1-methyl-3H-imidazol-1-iumchlorid;
- 3-[(2-Hydroxy-4-acetylamino-6-amino-phenylcarbamoyl)-methyl]-1-methyl-3H-imidazol-1-iumchlorid;
- 3-[(2-Hydroxy-3,5-dichlor-4-methyl-phenylcarbamoyl)-methyl)-1-methyl-3H-imidazol-1-iumchlorid;
- 3-[(2-Hydroxy-3,5-dichlor-4-amino-phenylcarbamoyl)-methyl]-1-methyl-3H-imidazol-1-iumchlorid;
- 3-[(2-Hydroxy-3,5-dichlor-4-acetylamino-phenylcarbamoyl)-methyl]-1-methyl-3H-imidazol-1-iumchlorid;
- 3-[(2-Hydroxy-3,5-dichlor-4-methoxycarbonylamino-phenylcarbamoyl)-methyl]-1-methyl-3H-imidazol-1-iumchlorid;
- 3-[(2-Hydroxy-3-acetylamino-phenylcarbamoyl)-methyl]-1-methyl-3H-imidazol- 1-iumchlorid;
- 1-[(2-Hydroxy-phenylcarbamoyl)-methyl]-pyridiniumchlorid;
- 1-[(2-Hydroxy-3-(2-(pyridinium-1-yl)acetylamino)-phenylcarbamoyl)-methyl]-pyridiniumdichlorid;
- 1-[(2-Hydroxy-4-(2-(pyridinium-1-yl)acetylamino)-phenylcarbamoyl)-methyl]-pyridiniumdichlorid;
- 1-[(2-Hydroxy-4-methyl-phenylcarbamoyl)-methyl]-pyridiniumchlorid;
- 1-[(2-Hydroxy-4-amino-phenylcarbamoyl)-methyl]-pyridiniumchlorid;
- 1-[(2-Hydroxy-4-acetylamino-phenylcarbamoyl)-methyl]-pyridiniumchlorid;
- 1-[(2-Hydroxy-4-methoxycarbonylamino-phenylcarbamoyl)-methyl]-pyridiniumchlorid;
- 1-[(3-Hydroxy-4-acetylammino-phenylcarbamoyl)-methyl]-pyridiniumchlorid;
- 1-[(3-Hydroxy-4-methoxycarbonylamino-phenylcarbamoyl)-methyl]-pyridiniumchlorid;
- 1-[(2-Hydroxy-5-chlor-phenylcarbamoyl)-methyl]-pyridiniumchlorid;
- 1-[(2-Hydroxy-4-methyl-5-chlor-phenylcarbamoyl)-methyl]-pyridiniumchlorid;
- 1-[(2-Hydroxy-4-amino-5-chlor-phenylcarbamoyl)-methyl]-pyridiniumchlorid;
- 1-[(2-Hydroxy-4-acetylamino-5-chlor-phenylcarbamoyl)-methyl]-pyridiniumchlorid;
- 1-[(2-Hydroxy-4-methoxycarbonylamino-5-chlor-phenylcarbamoyl)-methyl]-pyridiniumchlorid;
- 1-[(3-Hydroxy-4-acetylamino-6-chlor-phenylcarbamoyl)-methyl]-pyridiniumchlorid;
- 1-[(3-Hydroxy-4-methoxycarbonyl-6-chlor-phenylcarbamoyl)-methyl]-pyridiniumchlorid;
- 1-[(2-Hydroxy-5-methoxy-phenylcarbamoyl)-methyl]-pyridiniumchlorid;
- 1-[(2-Hydroxy-4-methyl-5-methoxy-phenylcarbamoyl)-methyl]-pyridiniumchlorid;
- 1-[(2-Hydroxy-4-amino-5-methoxy-phenylcarbamoyl)-methyl]pyridiniumchlorid;
- 1-[(2-Hydroxy-4-acetylamino-5-methoxy-phenylcarbamoyl)-methyl]pyridiniumchlorid;
- 1-[(2-Hydroxy-4-methoxycarbonylamino-5-methoxy-phenylcarbamoyl)-methyl]pyridiniumchlorid;
- 1-[(3-Hydroxy-4-acetylamino-6-methoxy-phenylcarbamoyl)-methyl]pyridiniumchlorid;
- 1-[(3-Hydroxy-4-methoxycarbonylamino-6-methoxyphenylcarbamoyl)-methyl]pyridiniumchlorid;
- 1-[(2-Hydroxy-6-amino-phenylcarbamoyl)-methyl]pyridiniumchlorid;
- 1-[(2-Hydroxy-6-acetylamino-phenylcarbamoyl)-methyl]pyridiniumchlorid;
- 1-[(2-Hydroxy-4,6-diamino-phenylcarbamoyl)-methyl]pyridiniumchlorid;
- 1-[(2-Hydroxy-4-acetylamino-6-amino-phenylcarbamoyl)-methyl]pyridiniumchlorid;
- 1-[(2-Hydroxy-3,5-dichlor-4-methyl-phenylcarbamoyl)-methyl]pyridiniumchlorid;
- 1-[(2-Hydroxy-3,5-dichlor-4-amino-phenylcarbamoyl)-methyl]pyridiniumchlorid;
- 1-[(2-Hydroxy-3,5-dichlor-4-acetylamino-phenylcarbamoyl)-methyl]pyridiniumchlorid;
- 1-[(2-Hydroxy-3,5-dichlor-4-methoxycarbonylamino-phenylcarbamoyl)-methyl]pyridiniumchlorid;
- 1-[(2-Hydroxy-3-acetylamino-phenylcarbamoyl)-methyl]pyridiniumchlorid;
- 1-[(2-Hydroxy-phenylcarbamoyl)-methyl]-1,4-dimethylpiperazin-1-iumchlorid;
- 1-[(2-Hydroxy-3-(2-(1,4-dimethyl-piperazin-1-ium-1-yl)-acetyl)amino-phenylcarbamoyl)-methyl]-1,4-dimethylpiperazin-1-iumdichlorid;
- 1-[(2-Hydroxy-4-(2-(1,4-dimethyl-piperazin-1-ium-1-yl)-acetyl)amino-phenylcarbamoyl)-methyl]-1,4-dimethylpiperazin-1-iumdichlorid;
- 1-[(2-Hydroxy-4-methyl-phenylcarbamoyl)-methyl]-1,4-dimethyl-piperazin-1-iumchlorid;
- 1-[(2-Hydroxy-4-amino-phenylcarbamoyl)-methyl]-1,4-dimethyl-piperazin-1-iumchlorid;
- 1-[(2-Hydroxy-4-acetylamino-phenylcarbamoyl)-methyl]-1,4-dimethyl-piperazin-1-iumchlorid;
- 1-[(2-Hydroxy-4-methoxycarbonylamino-phenylcarbamoyl)-methyl]-1,4-dimethyl-piperazin-1-iumchlorid;
- 1-[(3-Hydroxy-4-acetylamino-phenylcarbamoyl)-methyl]-1,4-dimethyl-piperazin-1-iumchlorid;
- 1-[(3-Hydroxy-4-methoxycarbonylamino-phenylcarbamoyl)-methyl]-1,4-dimethyl-piperazin-1-iumchlorid;
- 1-[(2-Hydroxy-5-chlor-phenylcarbamoyl)-methyl]-1,4-dimethyl-piperazin-1-iumchlorid;
- 1-[(2-Hydroxy-4-methyl-5-Chlor-phenylcarbamoyl)-methyl]-1,4-dimethyl-piperazin-1-iumchlorid;
- 1-[(2-Hydroxy-4-amino-5-chlor-phenylcarbamoyl)-methyl]-1,4-dimethyl-piperazin-1-iumchlorid;
- 1-[(2-Hydroxy-4-acetylamino-5-chlor-phenylcarbamoyl)-methyl]-1,4-dimethyl-piperazin-1-iumchlorid;
- 1-[(2-Hydroxy-4-methoxycarbonylamino-5-Chlor-phenylcarbamoyl)-methyl]-1,4-dimethyl-piperazin-1-iumchlorid;
- 1-[(3-Hydroxy-4-acetylamino-6-chlor-phenylcarbamoyl)-methyl]-1,4-dimethyl-piperazin-1-iumchlorid;
- 1-[(3-Hydroxy-4-methoxycarbonylamino-6-chlor-phenylcarbamoyl)-methyl]-1,4-dimethyl-piperazin-1-iumchlorid;
- 1-[(2-Hydroxy-5-methoxy-phenylcarbamoyl)-methyl]-1,4-dimethyl-piperazin-1-iumchlorid;
- 1-[(2-Hydroxy-4-methyl-5-methoxy-phenylcarbamoyl)-methyl]-1,4-dimethyl-piperazin-1-iumchlorid;
- 1-[(2-Hydroxy-4-amino-5-methoxy-phenylcarbamoyl)-methyl]-1,4-dimethyl-piperazin-1-iumchlorid;
- 1-[(2-Hydroxy-4-acetylamino-5-methoxy-phenylcarbamoyl)-methyl]-1,4-dimethyl-piperazin-1-iumchlorid;
- 1-[(2-Hydroxy-4-methoxycarbonylamino-5-methoxy-phenylcarbamoyl)-methyl]-1,4-dimethyl-piperazin-1-iumchlorid;
- 1-[(3-Hydroxy-4-acetylamino-6-methoxy-phenylcarbamoyl)-methyl]-1,4-dimethyl-piperazin-1-iumchlorid;
- 1-[(3-Hydroxy-4-methoxycarbonylamino-6-methoxy-phenylcarbamoyl)-methyl]-1,4-dimethyl-piperazin-1-iumchlorid;
- 1-[(2-Hydroxy-6-amino-phenylcarbamoyl)-methyl]-1,4-dimethyl-piperazin-1-iumchlorid;
- 1-[(2-Hydroxy-6-acetylamino-phenylcarbamoyl)-methyl]-1,4-dimethyl-piperazin-1-iumchlorid;
- 1-[(2-Hydroxy-4,6-diamino-phenylcarbamoyl)-methyl]-1,4-dimethyl-piperazin-1-iumchlorid;
- 1-[(2-Hydroxy-4-acetylamino-6-amino-phenylcarbamoyl)-methyl]-1,4-dimethyl-piperazin-1-iumchlorid;
- 1-[(2-Hydroxy-3,5-dichlor-4-methyl-phenylcarbamoyl)-methyl]-1,4-dimethyl-piperazin-1-iumchlorid;
- 1-[(2-Hydroxy-3,5-dichlor-4-amino-phenylcarbamoyl)-methyl]-1,4-dimethyl-piperazin-1-iumchlorid;
- 1-[(2-Hydroxy-3,5-dichlor-4-acetylamino-phenylcarbamoyl)-methyl]-1,4-dimethyl-piperazin-1-iumchlorid;
- 1-[(2-Hydroxy-3,5-dichlor-4-methoxycarbonylamino-phenylcarbamoyl)-methyl]-1,4-dimethyl-piperazin-1-iumchlorid;
- 1-[(2-Hydroxy-3-acetylamino-phenylcarbamoyl)-methyl]-1,4-dimethyl-piperazin-1-iumchlorid;
und deren Additionssalze mit einer Säure.

23. Verwendung der Verbindungen der Formel (I') nach einem der Ansprüche 21 bis 22 als Kuppler zum oxidativen Färben von Keratinfasern.

## Claims

1. Composition for the oxidation dyeing of keratinous fibres, **characterized in that** it contains, in a medium appropriate for dyeing:
- at least one oxidation base, and
- at least one coupler chosen from the compounds of the following formula (I) and their addition salts with an acid: 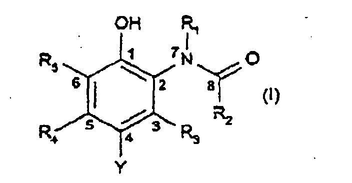 in which:
• R₁ represents a hydrogen atom; a methyl, ethyl, isopropyl, allyl, phenyl, benzyl, fluorobenzyl, hydroxybenzyl, difluorobenzyl, trifluorobenzyl, chlorobenzyl, bromobenzyl, methoxybenzyl, dimethoxybenzyl, (trifluoromethoxy)benzyl, 3,4-methylenedioxybenzyl, 6-chloropiperonyl, 4-methylthiobenzyl, 4-methylsulphonylbenzyl, 4-acetylaminobenzyl, 4-carboxybenzyl, 1-naphthomethyl or 2-naphthomethyl radical; or a 2-hydroxyethyl, 2-methoxyethyl or 2-ethoxyethyl group;
• R₂ represents a hydrogen atom; a group Z; a radical chosen from the group **(G1)** consisting of a methyl, ethyl, propyl, isopropyl, butyl, isobutyl, tert-butyl, pentyl, isopentyl, neopentyl, hexyl; cyclopropyl, cyclobutyl, cyclopentyl, cyclopentylmethyl, 3-cyclopentylpropyl, cyclohexyl, 2-cyclohexylethyl, norborn-2-yl, vinyl, 1-methylvinyl, 2-methylvinyl, 2,2-dimethylvinyl, allyl, 3-butenyl; phenyl, methylphenyl, dimethylphenyl, 2,4,6-trimethylphenyl, 4-ethylphenyl, (trifluoromethyl)phenyl, hydroxyphenyl, methoxyphenyl, ethoxyphenyl, acetoxyphenyl, (trifluoromethoxy)phenyl, aminophenyl, 4-dimethylaminophenyl, fluorophenyl, difluorophenyl, fluoro(trifluoromethyl)phenyl, chlorophenyl, dichlorophenyl, bromophenyl, naphth-1-yl, naphth-2-yl, (2-methoxy)naphth-1-yl, benzyl, 4'-methoxybenzyl, 2',5'-dimethoxybenzyl, 3',4'-dimethoxybenzyl, 4'-fluorobenzyl, 4'-chlorobenzyl, phenethyl, 2-phenylvinyl, (1-naphthyl)methyl, (2-naphthyl)methyl; tetrahydrofuran-2-yl, furan-2-yl, 5-methyl-2-(trifluoromethyl)furan-3-yl, 2-methyl-5-phenylfuran-3-yl, thiophen-2-yl, (thiophen-2-yl)methyl, 3-chlorothiophen-2-yl, 2,5-dichlorothiophen-3-yl, benxothiophen-2-yl, 3-chlorobenzothiophen-2-yl, isoxazol-5-yl, 5-methylisoxazol-3-yl, 3,5-dimethylisoxazol-4-yl, 1,3-dimethylpyrazol-5-yl, 1-ethyl-3-methylpyrazol-5-yl, 1-tert-butyl-3-methylpyrazolyl, 3-tert-butyl-1-methylpyrazol-5-yl, 4-bromo-1-ethyl-3-methylpyrazol-5-yl, indol-3-ylcarboxyl, pyridinyl, chloropyridinyl, dichloropyridinyl, 5-(bromo)pyridin-3-yl, piperazin-2-yl, quinoxal-2-yl; fluoromethyl, difluoromethyl, trifluoromethyl, 1,1,2,2-tetrafluoroethyl, pentafluoroethyl, heptafluoropropyl, 1,1,2,2,3,3,4,4-octafluorobutyl, nonafluorobutyl, chloromethyl, chloroethyl, 1,1-dimethyl-2-chloroethyl, 1,2-dichloroethyl, 1-chloropropyl, 3-chloropropyl, 4-chlorobutyl, hydroxymethyl, methoxymethyl, phenoxymethyl, (9-chlorophenoxy)methyl, benzyloxymethyl, acetoxymethyl, 1,2-dihydroxyethyl, 1-phenoxyethyl, 1-acetoxyethyl, 2-(2-carboxyethoxy)ethyl, 1-phenoxyethyl, 1-acetoxyethyl, methoxycarbonyl, ethoxycarbonyl, (methoxycarbonyl)methyl, 2-carboxyethyl, 2-(methoxycarbonyl)ethyl, 2-carboxycyclopropyl, 2-carboxycyclohexane; methoxy, ethoxy, propoxy, isopropoxy, butoxy, isobutoxy, pentoxy, neopentoxy, hexyloxy, cyclopentyloxy, cyclohexyloxy, vinyloxy, allyloxy, propargyloxy, chloromethoxy, 1-chloroethoxy, 2-methoxyethoxy, 4-chlorobutoxy, phenoxy, 4-methylphenoxy, 4-fluorophenoxy, 4-bromophenoxy, 4-chlorophenoxy, 4-methoxyphenoxy, naphth-2-yloxy, benzyloxy; amino, methylamino, ethylamino, propylamino, isopropylamino, butylamino, cyclohexylamino, allylamino, 2-chloroethylamino, 3-chloropropylamino, carboxymethylamino, phenylamino, fluorophenylamino, (trifluoromethyl)-phenylamino, chlorophenylamino, bromophenylamino, 4-acetylphenylamino, methoxyphenylamino, (trifluoromethoxy)phenylamino, naphth-1-ylamino, benzylamino, phenethylamino, pyrid-3-ylamino, dimethylamino, 1-pyrrolidinyl and 4-morpholinyl radical;
• R₃ and R₄, which are identical or different, represent a hydrogen or halogen atom; a hydroxyl or amino group; a group Z; a group A₁, A₄ or A₅ as defined below; a group A₁, A₂, A₃, A₄ or A₅ as defined below and separated from the phenolic ring of the formula (I) by an oxygen atom or by a group -NH-, -N(C₁-C₃)alkyl-, -O(CO)-, -NH(CO)-, -N(C₁-C₃)alkyl(CO)-, -NH[C=NH]-, -NH(CO)NH-, -NH(CO)N(C₁-C₃)alkyl-, -NH(CO)O-, -NHSO₂-, -NHSO₂NH-or -NHSO₂N(C₁-C₃)alkyl-
- the group A₁ represents linear or branched C₁-C₈ alkyl radicals, which may carry one or two double bonds or one triple bond, which may be unsubstituted or substituted with a group chosen from a group A₂, a group A₄, and a group A₅ as defined below, which may be unsubstituted or substituted with one or two groups, which are identical or different, chosen from the N-(C₁-C₃)alkylamino, N-(C₁-C₃)alkyl-N-(C₁-C₃)alkylamino, (C₁-C₆)alkoxy, oxo, alkoxycarbonyl, acyloxy, amide, acylamino, ureyl, sulphoxy, sulphonyl, sulphonamido, sulphonylamino, bromo, cyano and carboxyl groups, and which may be unsubstituted or substituted with one or more hydroxyl, fluoro or chloro groups;
- the group A₂ represents an aromatic group of the phenyl or naphthyl type, which may be unsubstituted or substituted with one to three groups, which are identical or different, chosen from the methyl, trifluoromethyl, ethyl, isopropyl, butyl, pentyl, fluoro, chloro, bromo, methoxy, trifluoromethoxy, ethoxy, propyloxy, acetyloxy, acetyl and cyano groups;
- the group A₃ represents heteroaromatic groups chosen from the furanyl, thiophenyl, pyrrolyl, imidazolyl, thiazolyl, oxazolyl, 1,2,3-triazolyl, 1,2,4-triazolyl, isoxazolyl, isothiazolyl, pyrazolyl, pyrazolotriazolyl, pyrazoloimidazolyl, pyrrolotriazolyl, pyrazolopyrimidyl, pyrazolopyridyl, pyridyl, pyrimidyl, benzoimidazolyl, benzoxazolyl, benzothiazolyl, indolyl, indolidinyl, isoindolyl, indazolyl, benzotriazolyl, quinolinyl, benzoimidazolyl and benzopyrimidyl groups, the said groups being unsubstituted or substituted with 1 to 3 radicals chosen from a linear or branched C₁-C₄ alkyl radical, a C₁-C₄ monohydroxyalkyl radical, a C₂-C₄ polyhydroxyalkyl radical, a carboxyl radical, an alkoxycarbonyl radical, a halogen atom, an amido radical, an amino radical and a hydroxyl radical;
- the group A₄ represents a C₃-C₇ cycloalkyl radical, a norbornyl radical, carrying or otherwise a double bond and unsubstituted or substituted with 1 or 2 radicals chosen from a linear or branched C₁-C₄ alkyl radical, a C₁-C₄ monohydroxyalkyl radical, a C₂-C₄ polyhydroxyalkyl radical, a carboxyl radical, an alkoxycarbonyl radical, a halogen atom, an amido radical, an amino radical and a hydroxyl radical; or
- the group A₅ represents a heterocycle chosen from the dihydrofuranyl, tetrahydrofuranyl, butyrolactonyl, dihydxothiophenyl, tetrahydrothiophenyl, tetrahydrothiophenonyl, iminothiolane, dihydropyrrolyl, pyrrolidinyl, pyrrolidinonyl, imidazolidinonyl, imidazolidinethionyl, oxazolidinyl, oxazolidinonyl, oxazolanethione, thiazolidinyl, isothiazolonyl, mercaptothiazolinyl, pyrazolidinonyl, iminathiolane, dioxolanyl, pentalactone, dioxanyl, dihydropyridinyl, piperidinyl, pentalactam, morpholinyl, pyrazoli(di)nyl, pyximi(di)nyl, pyrazinyl, piperazinyl and azepinyl rings,
• R₅ represents a hydrogen or halogen atom; a group Z; a group A₁, A₄ or A₅; a group A₁, A₂, A₃, A₄ or A₅ separated from the phenolic ring of the compounds of formula (I) by an oxygen or sulphur atom or by a group -NH-, -N(C₁-C₃)alkyl-, -O(CO)-, -NH(CO)-, -N(C₁-C₃)alkyl(CO)-, -NH[C=NH]-, -NH(CO)NH-, -NH(CO)N(C₁-C₃)alkyl-, or -NH(CO)O-,
• Y represents a hydrogen or halogen atom; a group -OCH₂COOH, -OCH₂COOCH₃, -OCH₂COOC₂H₅, -OR₆, -SR₆ or -NH-SO₂R₆ in which R₆ represents a C₁-C₆ alkyl radical, linear or branched (it being possible for the branch(es) to then form one or more rings comprising from 3 to 6 members), unsubstituted or substituted with one or more radicals chosen from the group consisting of a halogen atom, a hydroxyl, C₁-C₄ alkoxy, amino and C₁-C₄ aminoalkyl radical; a phenyl radical which is unsubstituted or substituted with one or two radicals chosen from the group consisting of a C₁-C₄ alkyl, trifluoromethyl, carboxyl, C₁-C₄ alkoxycarbonyl, halogen, hydroxyl, C₁-C₄ alkoxy, amino and C₁-C₄ aminoalkyl radical; or a benzyl radical; and it being understood that Y cannot represent -NH-SO₂R₆ when R₃ represents a hydroxyl radical;
• Z is a cationic group represented by the following formula (II): 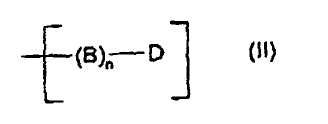
in which:
- B represents a radical comprising from 1 to 15 carbon atoms, linear or branched (it being possible for the branch(es) to then form one or more rings comprising from 3 to 7 members), which may contain one or more double bonds and/or one or more triple bonds, the said double bonds possibly leading to aromatic groups, and in which one or more carbon atoms may be replaced with an oxygen, nitrogen or sulphur atom or with an SO₂ radical; and in which one or more carbon atoms may, independently of each other, be substituted with one or more halogen atoms or with one or more groups Z; the said radical B containing no peroxide bond or diazo, nitro or nitroso radicals;
- n is equal to 0 or 1,
- D is chosen from the cationic imidazolinium, thiazolinium, oxazoliniurn, pyrrolinium, 1,2,3-triazolinium, 1,2,4-triazolinium, isoxazolinium, isothiazolinium, imidazolidinium, thiazolidinium, pyrazolinium, pyrazolidinium, oxazolidinium, pyrazoltriazolinium, pyrazoloimidazolinium, pyrrolotriazolinium, pyrazolopyrimidinium, pyrazolopyridinium, pyridinium, pyrimidinium, pyrazinium, triazinium, benzoimidazolinium, benzoxazolinium, benzothiazolinium, indolinium, indolidinium, isoindolinium, indazolinium, benzotriazolinium, quinolinium, tetrahydroquinolinium, benzoimidazolidinium, benzopyrimidinium, tetra(C₁-C₄)alkylammonium, polyhydroxytetra(C₁-C₄)alkylammonium, dialkylpiperidinium, dialkylpyrrolidinium, dialkylmorpholinium, dialkylthiomorpholinium, dialkylpiperazinium, azepinium and 1,4-diazabicyclo[2.2.2]octanium groups,
it being understood that at least one of the groups R₁ to R₅ represents a group Z.

2. Composition according to Claim 1, **characterized in that** R₂ represents a radical chosen from the group **(G2)** consisting of a methyl, ethyl, propyl, allyl, phenyl, tetrahydrofuran-2-yl, furan-2-yl, thiophen-2-yl, pyridinyl, piperazin-2-yl, fluoromethyl chloromethyl, 2-chloroethyl, methoxymethyl, acetoxymethyl, 1,2-dihydroxyethyl, methoxycarbonyl, 2-carboxyethyl, methoxy, ethoxy, propoxy, allyloxy, 2-chloroethoxy, 2-methoxyethoxy, amino, ethylamino, allylamino, 2-chloroethylamino, pyridylamino, dimethylamino, 1-pyrrolidinyl or 4-morpholinyl radical; or a group -D', -E-D', -O-E-D', -NH-E-D', in which -E- represents a -(CH₂)_{q}- arm, q being an integer equal to 1 or 2, and **D'** represents a group chosen from 3-methylimidazolidinium-1-yl, 3-(2-hydroryethyl)imidazolidinium-1-yl, 1,2,4-triazolinium-1-yl, 1,2,4-triazolinium-4-yl, N-(C₁-C₄)alkylpyridinium-2-yl, N-(C₁-C₄)alkylpyridinium-3-yl, N-(C₁-C₄)alkylpyridinium-4-yl, N-(2-hydroxyethyl)-pyridinium-2-yl, N-(2-hydroxyethyl)pyridinium-3-yl, N-(2-hydroxyethyl)pyridinium-4-yl, pyridinium-1-yl, tri(C₁-C₄)alkylammonium-N-yl, 1-methylpiperidinium-1-yl and 1,4-dimethylpiperazinium-1-yl.

3. Composition according to Claim 2, **characterized in that** R₂ represents a methyl, methoxymethyl, 2-carboxyethyl, methoxy, amino, ethylamino or 1-pyrrolidinyl radical; a group -D', -E-D', -O-E-D' or -NH-E-D', in which -E- represents a -(CH₂)_{q}- arm, q = 1 to 2.

4. Composition according to Claim 1, **characterized in that** R₃ represents a hydrogen or chlorine atom; a group Z; a methyl, hydroxymethyl, methoxymethyl, 1-hydroxyethyl, aminomethyl, methylaminomethyl, hydroxyl, methoxy, acetoxy, amino, methylamino or 2-hydroxyethylamino radical; a group -NH(CO)R₁₂ in which R₁₂ represents one of the radicals listed in the group **(G1)**; a group -NHSO₂R₁₃, in which R₁₃ represents one of the radicals listed in the group **(G3)** consisting of the methyl, trifluoromethyl, ethyl, 2-chloroethyl, propyl, 3-chloropropyl, isopropyl, butyl, thiophen-2-yl, hydroxyl, ethoxy and dimethylamino radicals.

5. Composition according to Claim 4, **characterized in that** R₃ represents a hydrogen atom; a methyl, hydroxymethyl, aminomethyl, hydroxyl, methoxy, amino or methylamino radical; a methanesulphonylamino group; ethanesulphonylamino; dimethylaminosulphonylamino; a group -NH(CO)R₁₄ in which R₁₄ represents one of the radicals listed in the group **(G2)**; or a group -O-E-D', -NH-E-D', -CH₂O-E-D', -CH₂NH-E-D', -CH₂NH(CO)-D', -NH(CO)-D', -NH(CO)-E-D', -NH(CO)O-E-D', -NH(CO)NH-E-D' or -NH(SO₂)-E-D'.

6. Composition according to Claim 1, **characterized in that** R₄ represents a hydrogen or chlorine atom; a group Z; a methyl, ethyl, hydroxymethyl, methoxymethyl, aminomethyl, methylaminomethyl, hydroxyl, methoxy, acetoxy, amino, methylamino, N-piperidino or N-morpholino radical; a group -NH(CO)R₁₅ in which R₁₅ represents one of the radicals listed in the group **(G1)**; or a group -NHSO₂R₁₆ in which R₁₆ represents one of the radicals listed in the group **(G3).**

7. Composition according to Claim 6, **characterized in that** R₄ represents a hydrogen or chlorine atom; a methyl, hydroxymethyl, aminomethyl, hydroxyl, methoxy, amino or methylamino radical; a methanesulphonylamino group; ethanesulphonylamino; dimethylaminosulphonylamino; a group -NH(CO)R₁₇ in which R₁₇ represents one of the radicals listed in the group **(G2);** or a group -O-E-D', -NH-E-D', -CH₂O-E-D', -CH₂NH-E-D', -CH₂NH(CO)-D', -NH(CO)-D', -NH(CO)-E-D', -NH(CO)O-E-D', -NH(CO)NH-E-D' or -NH(SO₂)-E-D'.

8. Composition according to Claim 1, **characterized in that** R₅ represents a hydrogen, chlorine, fluorine or bromine atom; a group z; a methyl, trifluoromethyl, allyl, hydroxymethyl, methoxymethyl, 1-hydroxyethyl, aminomethyl, methylaminomethyl, methoxy, acetoxy or methylamino radical; or a group -NH(CO)R₁₈ in which R₁₈ represents one of the radicals listed in the group **(G1)**.

9. Composition according to Claim 8, **characterized in that** R₅ represents a hydrogen, chlorine or fluorine atom; a methyl, hydroxymethyl, aminomethyl, methoxy or methylamino radical; a group -NH(CO)R₁₉ in which R₁₉ represents one of the radicals listed in the group (G2) *as* defined in Claim 7; or a group -O-E-D', -NH-E-D', -CH₂O-E-D', -CH₂NH-E-D', -CH₂NH(CO)-D', -NH(CO)-D', -NH(CO)-E-D', -NH(CO)O-E-D', or -NH(CO)NH-E-D'.

10. Composition according to any one of the preceding claims, **characterized in that** Y is chosen from a hydrogen, chlorine, fluorine or bromine atom; a methoxy, ethoxy, propoxy, benzyloxy, phenoxy, -OCH₂CH₂OCH₃; -OCH₂CH₂N(CH₃)₂; -OCH₂(CO)OH, -OCH₂(CO)OCH₃, -OCH₂(CO)OC₂H₅, -SCH₂CH₂CO₂H or -NHSO₂CH₃ group; it being understood that Y cannot represent a group -NHSO₂CH₃ when R₃ represents a hydroxyl radical.

11. Composition according to Claim 1, **characterized in that** D represents a 3-methylimidazolidinium-1-yl, 3-(2-hydroxyethyl)-imidazolidinium-1-yl, 1,2,4-triazolinium-1-yl, 1,2,4-triazolinium-4-yl, N-(C₁-C₄)alkylpyridin-2-yl, N-(C₁-C₄)alkylpyridin-3-yl, N-(C₁-C₄)alkylpyridin-4-yl, N-(2-hydroxyethyl)pyridin-2-yl, N-(2-hydroxyethyl)pyridin-3-yl, N-(2-hydroxyethyl)pyridin-4-yl, pyridin-1-yl, tri(C₁-C₄)alkylammonium-N-yl, 1-methylpiperidinium-1-yl, thiazolinium-3-yl or 1,4-dimethylpiperazinium-1-yl group.

12. Composition according to any one of the preceding claims, **characterized in that** the compounds of formula (I) are chosen from those in which:
i)
- R₁ represents a hydrogen atom;
- R₂ represents a group -D', -E-D', -O-E-D', -NH-E-D', or a radical chosen from the group **(G4)** consisting of a methyl, methoxymethyl, 2-carboxyethyl, methoxy, amino, ethylamino or 1-pyrrolidinyl radical;
- R₃ represents a hydroxyl, amino or methylamino radical; a group -NH(CO)R₂₀ in which R₂₀ represents one of the radicals listed in the group **(G4)** defined above; a methanesulphonylamino, ethanesulphonylamino or dimethylaminosulphonylamino group; a group -O-E-D', -NH-E-D', -NH(CO)-D', -NH(CO)-E-D', -NH(CO)O-E-D', -NH(CO)NH-E-D' or -NH(SO₂)-E-D';
- R₄ represents a hydrogen or chlorine atom, or a methyl group;
- R₅ represents a hydrogen, chlorine or fluorine atom, or a methyl group;
- Y represents a hydrogen or chlorine atom; a methoxy or -OCH₂(CO)OCH₃ group; it being understood that at least one of the groups R₂ and R₃ contains a group Z;
ii)
- R₁ represents a hydrogen atom;
- R₂ represents a group -D', -E-D', -O-E-D' or -NH-E-D, or one of the radicals listed in the group **(G4)**;
- R₃ represents a hydrogen atom; or a methyl radical;
- R₄ represents a hydroxyl, amino, methylamino, methanesulphonylamino, ethanesulphonylamino or dimethylaminosulphonylamina radical; a group -NH(CO)R₂₁ in which R₂₁ represents one of the radicals listed in the group **(G4);** or a group -O-E-D', -NH-E-D', -NH(CO)-D', -NH(CO)-E-D', -NH(CO)O-E-D', -NH(CO)NH-E-D' or -NH(SO₂)-E-D';
- R₅ represents a hydrogen, chlorine or fluorine atom; or a methyl, methoxy or methylamino group;
- Y represents a hydrogen or chlorine atom; a methoxy or -OCH₂(CO)OCH₃ group; it being understood that at least one of the groups R₂ and R₄ contains a group Z;
iii)
- R₁ represents a hydrogen atom;
- R₂ represents one of the radicals listed in the group **(G4)** defined above; or a group -D', -E-D', -O-E-D', -NH-E-D';
- R₃ represents a hydrogen atom; or a methyl radical;
- R₄ represents a hydrogen or chlorine atom; a methyl radical; or a methoxy or methylamino group;
- R₅ represents a group -NH(CO)R₂₂ in which R₂₂ represents one of the radicals listed in the group **(G4)**; or a group -O-E-D', -NH-E-D', -NH(CO)-D', -NH(CO)-E-D', -NH(CO)O-E-D' or -NH(CO)NH-E-D',
- Y represents a hydrogen or chlorine atom; or a mathoxy or -OCH₂(CO)OCH₃ group; it being understood that at least one of the groups R₂ and R₅ contains a group Z;
iv)
- R₁ represents a hydrogen atom;
- R₂ represents a group -D', -E-D', -O-E-D' or -NH-E-D',
- R₃ represents a hydrogen atom; or a methyl radical;
- R₄ represents a hydrogen or chlorine atom; or a methyl radical;
- R₅ represents a hydrogen, chlorine or fluorine atom; or a methyl group;
- Y represents a hydrogen or chlorine atom; or a methoxy or -OCH₂(CO)OCH₃ group.

13. Composition according to any one of the preceding claims, **characterized in that** the compound(s) of formula (I) are chosen from
- 3-[(2-Hydroxyphenylcarbamoyl)methyl]-1-methyl-3H-imidazol-1-ium chloride;
- 3-[(2-Hydroxy-3-(2-(3-methyl-1H-imidazol-3-ium-1-yl)acetylamino)phenylcarbamoyl)methyl]-1-methyl-3H-imidazol-1-ium dichloride;
- 3-[(2-Hydroxy-4-(2-(3-methyl-1H-imidazol-3-ium-1-yl)acetylamino)phenylcarbamoyl)methyl]-1-methyl-3H-imidazol-1-ium dichloride;
- 3-[(2-Hydroxy-4-methylphenylcarbamoyl)methyl]-1-methyl-3H-imidazol-1-ium chloride;
- 3-[(2-Hydroxy-4-aminophenylcarbamoyl)methyl]-1-methyl-3H-imidazol-1-ium chloride;
- 3-[(2-Hydroxy-4-acetylaminophenylcarbamoyl)methyl]-1-methyl-3H-imidazol-1-ium chloride;
- 3- [(2-Hydroxy-4-methoxycarbonylaminophenylcarbamoyl)methyl]-1-methyl-3H-imidazol-1-ium chloride;
- 3-[(3-Hydroxy-4-acetylaminophenylcarbamoyl)methyl]-1-methyl-3H-imidazol-1-ium chloride;
- 3-[(3-Hydroxy-4-methoxycarbonylaminophenylcarbamoyl)methyl]-2-methyl-3H-imidazol-1-ium chloride;
- 3-[(2-Hydroxy-5-chlorophenylcarbamoyl)methyl]-1-methyl-3H-imidazol-1-ium chloride;
- 3-[(2-Hydroxy-4-methyl-5-chlorophenylcarbamoyl)methyl]-1-methyl-3H-imidazol-1-ium chloride;
- 3-[(2-Hydroxy-4-amino-5-chlorophenylcarbamoyl)methyl]-1-methyl-3H-imidazol-1-ium chloride;
- 3-[(2-Hydroxy-4-acetylamino-5-chlorophenylcarbamoyl)methyl]-1-methyl-3H-imidazol-1-ium chloride;
- 3-[(2-Hydroxy-4-methoxycarbonylamino-5-chlorophenylcarbamoyl)methyl]-1-methyl-3H-imidazol-1-ium chloride;
- 3-[(3-Hydroxy-4-acetylamino-5-chlorophenylcarbamoyl)methyl)-1-methyl-3H-imidazol-1-ium chloride;
- 3-[(3-Hydroxy-4-methoxycarbonylamino-6-chlorophenylcarbamoyl)methyl]-1-methyl-3H-imidazol-1-ium chloride;
- 3-[(2-Hydroxy-5-methoxyphenylcarbamoyl)methyl]-1-methyl-3H-imidazol-1-ium chloride;
- 3-[(2-Hydroxy-4-methyl-5-methoxyphenylcarbamoyl)methyl]-1-methyl-3H-imidazol-1-ium chloride;
- 3-[(2-Hydroxy-4-amino-5-methoxyphenylcarbamoyl)methyl)-1-methyl-3H-imidazol-1-ium chloride;
- 3-[(2-Hydroxy-4-acetylamino-5-methoxyphenylcarbamoyl)methyl]-1-methyl-3H-imidazol-1-ium chloride;
- 3-[(2-Hydroxy-4-methoxycarbonylamino-5-methoxyphenylcarbamoyl)methyl]-1-methyl-3H-imidazol-1-ium chloride;
- 3-[(3-Hydroxy-4-acetylamino-6-methoxyphenylcarbamoyl)methyl]-1-methyl-3H-imidazol-1-ium chloride;
- 3-[(3-Hydroxy-4-methoxycarbonylamino-6-methoxyphenylcarbamoyl)methyl]-1-methyl-3H-imidazol-1-ium chloride;
- 3-[(2-Hydroxy-6-aminophenylcarbamoyl)methyl]-1-methyl-3H-imidazol-1-ium chloride;
- 3-[(2-Hydroxy-6-acetylaminophenylcarbamoyl)methyl]-1-methyl-3H-imidazol-1-ium chloride;
- 3-[(2-Hydroxy-4,6-diaminophenylcarbamoyl)methyl]-1-methyl-3H-imidazol-1-ium chloride;
- 3-[(2-Hydroxy-4-acetylamino-6-aminophenylcarbamoyl)methyl]-1-methyl-3H-imidazol-1-ium chloride;
- 3-[(2-Hydroxy-3,5-dichloro-4-methylphenylcarbamoyl)methyl]-1-methyl-3H-imidazol-1-ium chloride;
- 3-[(2-Hydroxy-3,5-dichloro-9-aminophenylcarbamoyl)methyl]-1-methyl-3H-imidazol-1-ium chloride;
- 3-[(2-Hydroxy-3,5-dichloro-4-acetylaminophenylcarbamoyl)methyl]-1-methyl-3H-imidazol-1-ium chloride;
- 3-[(2-Hydroxy-3,5-dichloro-4-methoxycarbonylaminophenylcarbamoyl)methyl]-1-methyl-3H-imidazol-1-ium chloride;
- 3-[(2-Hydroxy-3-acetylaminophenylcarbamoyl)methyl]-1-methyl-3H-imidazol-1-ium chloride;
- 1-[(2-Hydroxyphenylcarbamoyl)methyl]pyridinium chloride;
- 1-[(2-Hydroxy-3-(2-(pyridinium-1-yl)acetylamino)phenylcarbamoyl)methyl]pyridinium dichloride;
- 1-[(2-Hydroxy-4-(2-(pyridinium-1-yl)acetylamino)phenylcarbamoyl)methyl]pyridinium dichloride;
- 1-[(2-Hydroxy-4-methylphenylcarbamoyl)methyl]pyridinium chloride;
- 1-[(2-Hydroxy-4-aminophenylcarbamoyl)methyl]pyridinium chloride;
- 1-[(2-Hydxoxy-4-acetylaminophenylcarbamoyl)methyl]pyridinium chloride;
- 1-[(2-Hydroxy-4-methoxycarbonylaminophenylcarbamoyl)methyl]pyridinium chloride;
- 1-[(3-Hydroxy-4-acetylaminophenylcarbamoyl)methyl]pyridinium chloride;
- 1-[(3-Hydroxy-4-methoxycarbonylaminophenylcarbamoyl)methyl]pyridinium chloride;
- 1-[(2-Hydroxy-5-chlorophenylcarbamoyl)methyl]pyridinium chloride;
- 1-[(2-Hydroxy-4-methyl-5-chlorophenylcarbamoyl)methyl]pyridinium chloride;
- 1-[(2-Hydxoxy-4-amino-5-chlorophenylcarbamoyl)methyl]pyridinium chloride;
- 1-[(2-Hydroxy-4-acetylamino-5-chlorophenylcarbamoyl)methyl]pyridinium chloride;
- 1-[(2-Hydroxy-4-methoxycarbonylamino-5-chlorophenylcarbamoyl)methyl]pyridinium chloride;
- 1-[(3-Hydroxy-4-acetylamino-6-chlorophenylcarbamoyl)methyl]pyridinium chloride;
- 1-[(3-Hydroxy-4-methoxycarbonylamino-6-chlorophenylcarbamoyl)methyl]pyridinium chloride;
- 1-[(2-Hydroxy-5-methoxyphenylcarbamoyl)methyl]pyridinium chloride;
- 1-[(2-Hydroxy-4-methyl-5-methoxyphenylcarbamoyl)methyl]pyridinium chloride;
- 1-[(2-Hydroxy-4-amino-5-methoxyphenylcarbamoyl)methyl]pyridinium chloride;
- 1-[(2-Hydroxy-9-acetylamino-5-methoxyphenylcarbamoyl)methyl]pyridinium chloride;
- 1-[(2-Hydroxy-4-methoxycarbonylamino-5-methoxyphenylcarbamoyl)methyl]pyridinium chloride;
- 1-[(3-Hydroxy-4-acetylamino-6-methoxyphenylcarbamoyl)methyl]pyridinium chloride;
- 1-[(3-Hydroxy-4-methoxycarbonylamino-6-methoxyphenylcarbamoyl)methyl]pyridinium chloride;
- 1-[(2-Hydroxy-6-aminophenylcarbamoyl)methyl]pyridinium chloride;
- 1-[(2-Hydroxy-6-acetylaminophenylcarbamoyl)methyl]pyridinium chloride;
- 1-[(2-Hydroxy-4,6-diaminophenylcarbamoyl)methyl]pyridinium chloride;
- 1-[(2-Hydroxy-4-acetylamino-6-aminophenylcarbamoyl)methyl]pyridinium chloride;
- 1-[(2-Hydroxy-3,5-dichloro-4-methylphenylcarbamoyl)methyl]pyridinium chloride;
- 1-[(2-Hydroxy-3,5-dichloro-4-aminophenylcarbamoyl)methyl]pyridinium chloride;
- 1-[(2-Hydroxy-3,5-dichloro-4-acetylaminophenylcarbamoyl)methyl]pyridinium chloride;
- 1-[(2-Hydroxy-3,5-dichloro-4-methoxycarbonylaminophenylcarbamoyl)methyl]pyridinium chloride;
- 1-[(2-Hydroxy-3-acetylaminophenylcarbamoyl)methyl]pyridinium chloride;
- 1-[(2-Hydroxyphenylcarbamoyl)methyl]-1,4-dimethylpiperazin-1-ium chloride;
- 1-[(2-Hydroxy-3-(2-(1,4-dimethylpiperazin-1-ium-1-yl)acetyl)aminophenylcarbamoyl)methyl]-1,4-dimethylpiperazin-1-ium dichloride;
- 1-[(2-Hydroxy-4-(2-(1,4-dimethylpiperazin-1-ium-1-yl)acetyl)aminophenylcarbamoyl)methyl]-1,4-dimethylpiperazin-1-ium dichloride;
- 1-[(2-Hydroxy-4-methylphenylcarbamoyl)methyl]-1,4-dimethylpiperazin-1-ium chloride;
- 1-[(2-Hydroxy-4-aminophenylcarbamoyl)methyl]-1,4-dimethylpiperazin-1-ium chloride;
- 1-[(2-Hydroxy-4-acetylaminophenylcarbamoyl)methyl]1,4-dimethylpiperazin-1-ium chloride;
- 1-[(2-Hydroxy-4-methoxycarbonylaminophenylcarbamoyl)methyl]-1,4-dimethylpiperazin-1-ium chloride;
- 1-[(3-Hydroxy-4-acetylaminophenylcarbamoyl)methyl]1,4-dimethylpiperazin-1-ium chloride;
- 1-[(3-Hydroxy-4-methoxycarbonylaminophenylcarbamoyl)methyl]-1,4-dimethylpiperazin-1-ium chloride;
- 1-[(2-Hydroxy-5-chlorophenylcarbamoyl)methyl]-1,4-dimethylpiperazin-1-ium chloride;
- 1-[(2-Hydroxy-4-methyl-5-chlorophenylcarbamoyl)methyl]-1,4-dimethylpiperazin-1-ium chloride;
- 1-[(2-Hydroxy-4-amino-5-chlorophenylcarbamoyl)methyl]-1,4-dimethylpiperazin-1-ium chloride;
- 1-[(2-Hydxoxy-4-acetylamino-5-chlorophenylcarbamoyl)methyl]-1,4-dimethylpiperazin-1-ium chloride;
- 1-[(2-Hydroxy-4-methoxycarbonylamino-5-chlorophenyl-carbamoyl)methyl]-1,4-dimethylpiperazin-1-ium chloride;
- 1-[(3-Hydroxy-4-acetylamino-6-chlorophenylcarbamoyl)methyl]-1,4-dimethylpiperazin-1-ium chloride;
- 1-[(3-Hydroxy-4-methoxycarbonylamino-6-chlorophenylcarbamoyl)methyl]-1,4-dimethylpiperazin-1-ium chloride;
- 1-[(2-Hydroxy-5-methoxyphenylcarbamoyl)methyl]-1,4-dimethylpiperazin-1-ium chloride;
- 1-[(2-Hydroxy-4-methyl-5-methoxyphenylcarbamoyl)methyl]-1,4-dimethylpiperazin-1-ium chloride;
- 1-[(2-Hydroxy-4-amino-5-methoxyphenylcarbamoyl)methyl]-1,4-dimethylpiperazin-1-ium chloride;
- 1-[(2-Hydroxy-4-acetylamino-5-methoxyphenylcarbamoyl)methyl]-1,4-dimethylpiperazin-1-ium chloride;
- 1-[(2-Hydroxy-4-methoxycarbonylamino-5-methoxyphenylcarbamoyl)methyl]-1,4-dimethylpiperazin-1-ium chloride;
- 1-[(3-Hydroxy-4-acetylamino-6-methoxyphenylcarbamoyl)methyl]-1,4-dimethylpiperazin-1-ium chloride;
- 1-[(3-Hydroxy-4-methoxycarbonylamino-6-methoxyphenylcarbamoyl)methyl]-1,4-dimethylpiperazin-1-ium chloride;
- 1-[(2-Hydroxy-6-aminophenylcarbamoyl)methyl]-1,4-dimethylpiperazin-1-ium chloride;
- 1-[(2-Hydroxy-6-acetylaminophenylcarbamoyl)methyl]-1,4-dimethylpiperazin-1-ium chloride;
- 1-[(2-Hydroxy-4,6-diaminophenylcarbamoyl)methyl]-1,4dimethylpiperazin-1-ium chloride;
- 1-[(2-Hydroxy-4-acetylamino-6-aminophenylcarbamoyl)methyl]-1,4-dimethylpiperazin-1-ium chloride;
- 1-[(2-Hydroxy-3,5-dichloro-4-methylphenylcarbamoyl)methyl]-1,4-dimethylpiperazin-1-ium chloride;
- 1-[(2-Hydroxy-3,5-dichloro-4-aminophenylcarbamoyl)methyl]-1,4-dimethylpiperazin-1-ium chloride;
- 1-[(2-Hydroxy-3,5-dichloro-4-acetylaminophenylcarbamoyl)methyl]-1,4-dimethylpiperazin-1-ium chloride;
- 1-[(2-Hydroxy-3,5-dichloro-4-methoxycarbonylaminophenylcarbamoyl)methyl]-1,4-dimethylpiperazin-1-ium chloride;
- 1-[(2-Hydroxy-3-acetylaminophenylcarbamoyl)methyl]1,4-dimethylpiperazin-1-ium chloride;
and their addition salts with an acid.

14. Composition according to any one of the preceding claims, **characterized in that** the compound(s) of formula (I) and/or the or their addition salt(s) with an acid represent from 0.0005 to 12% by weight of the total weight of the dyeing composition.

15. Composition according to any one of the preceding claims, **characterized in that** the oxidation base(s) are chosen from para-phenylenediamines, bisphenylalkylenediamines, para-aminophenols, ortho-aminophenols and heterocyclic bases.

16. Composition according to any one of the preceding claims, **characterized in that** it contains one or more additional couplers chosen from meta-phenylenediamines, meta-aminophenols, meta-diphenols and heterocyclic couplers, and their addition salts with an acid, and/or one or more direct dyes.

17. Composition according to any one of the preceding claims, **characterized in that** the addition salts with an acid are chosen from hydrochlorides, hydrobromides, sulphates, citrates, succinates, tartrates, lactates and acetates.

18. Method of oxidation dyeing of keratinous fibres, **characterized in that** at least one dyeing composition as defined in any one of Claims 1 to 17 is applied to these fibres, and **in that** the colour is developed at acidic, neutral or alkaline pH with the aid of an oxidizing agent which is added just at the time of use to the dyeing composition or which is present in an oxidizing composition applied simultaneously or sequentially in a separate manner.

19. Method according to Claim 18, **characterized in that** the oxidizing agent is chosen from hydxogen peroxide, urea peroxide, alkali metal bromates, persalts and enzymes.

20. Multicompartment device, or multicompartment dyeing "kit", in which a first compartment contains a dyeing composition as defined in any one of Claims 1 to 17 and a second compartment contains an oxidizing composition.

21. Compounds of the following formula (I'), and their addition salts with an acid: 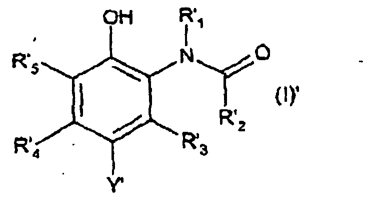 in which R'₁, R'₂, R'₃, R'₄, R'₅ and Y can have the same meanings as those indicated in any one of Claims 1 to 20 for R₁, R₂, R₃, R₄, R₅ and Y, in which at least one of the groups R'₁ to R'₅ represents a group Z' which can have the same meanings as those indicated above for the group Z in Claim 1; excluding the following compounds: 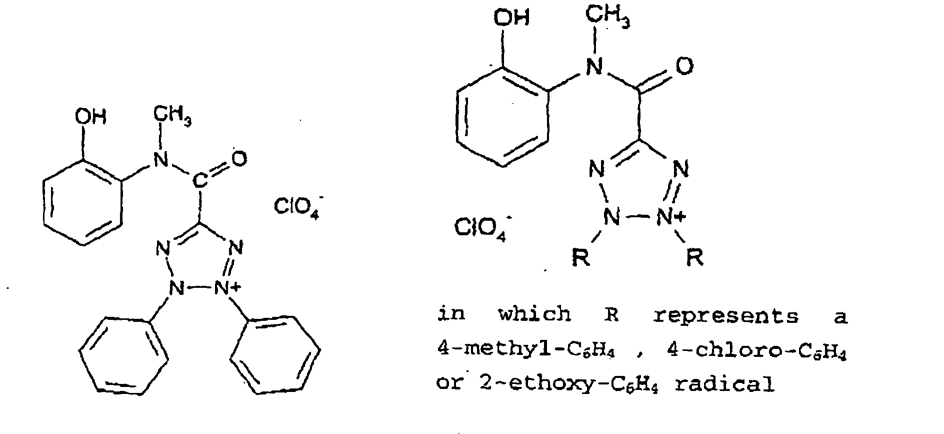 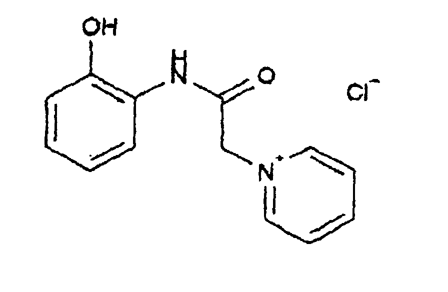 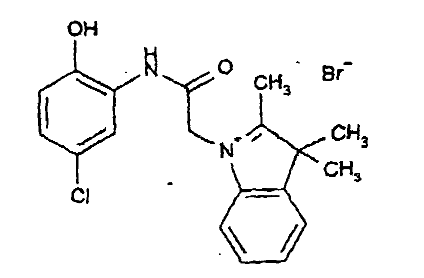 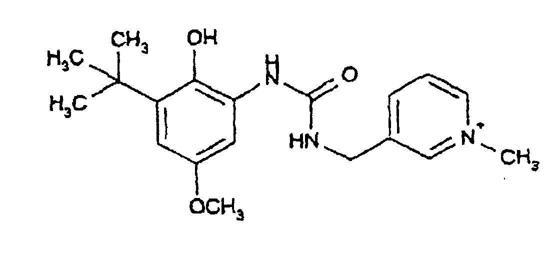 and

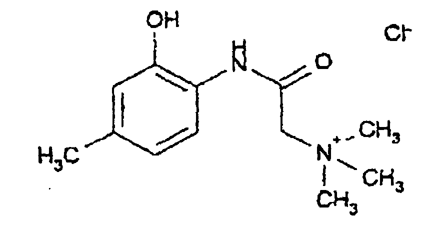

22. Compounds according to Claim 21, **characterized in that** they are chosen from:
- 3-[(2-Hydroxyphenylcarbamoyl)methyl]-1-methyl-3H-imidazol-1-ium chloride;
- 3-[(2-Hydroxy-3-(2-(3-methyl-1H-imidazol-3-ium-1-yl)acetylamino)phenylcarbamoyl)methyl]-1-methyl-3H-imidazol-1-ium dichloride;
- 3-[(2-Hydroxy-4-(2-(3-methyl-1H-imidazol-3-ium-1-yl)acetylamino)phenylcarbamoyl)methyl]-1-methyl-3H-imidazol-1-ium dichloride;
- 3-[(2-Hydroxy-4-methylphenylcarbamoyl)methyl]-1-methyl-3H-imidazol-1-ium chloride;
- 3-[(2-Hydroxy-4-aminophenylcarbamoyl)methyl]-1-methyl-3H-imidazol-1-ium chloride;
- 3-[(2-Hydroxy-4-acetylaminophenylcarbamoyl)methyl]-1-methyl-3H-imidazol-1-ium chloride;
- 3-[(2-Hydroxy-4-methoxycarbonylaminophenylcarbamoyl)methyl]-1-methyl-3H-imidazol-1-ium chloride;
- 3-[(3-Hydroxy-4-acetylaminophenylcarbamoyl)methyl]-1-methyl-3H-imidazol-1-ium chloride;
- 3-[(3-Hydroxy-4-methoxycarbonylaminophenylcarbamoyl)methyl]-1-methyl-3H-imidazol-1-ium chloride;
- 3-[(2-Hydroxy-5-chlorophenylcarbamoyl)methyl]-1-methyl-3H-imidazol-1-ium chloride;
- 3-[(2-Hydroxy-4-methyl-5-chlorophenylcarbamoyl)methyl]-1-methyl-3H-imidazol-1-ium chloride;
- 3-[(2-Hydroxy-4-amino-5-chlorophenylcarbamoyl)methyl]-1-methyl-3H-imidazol-1-ium chloride;
- 3-[(2-Hydroxy-4-acetylamino-5-chlorophenylcarbamoyl)methyl)-1-methyl-3H-imidazol-1-ium chloride;
- 3-[(2-Hydroxy-4-methoxycarbonylamino-5-chlorophenylcarbamoyl)methyl]-1-methyl-3H-imidazol-1-ium chloride;
- 3-[(3-Hydroxy-4-acetylamino-5-chlorophenylcarhamoyl)methyl]-1-methyl-3H-imidazol-1-ium chloride;
- 3-[(3-Hydrory-4-methoxycarbonylamino-6-chlorophenylcarbamoyl)methyl]-1-methyl-3H-imidazol-1-ium chloride;
- 3-[(2-Hydroxy-5-methoxyphenylcarbamoyl)methyl]-1-methyl-3H-imidazol-2-ium chloride;
- 3-[(2-Hydroxy-4-methyl-5-methoxyphenylcarbamoyl)methyl]-1-methyl-3H-imidazol-1-ium chloride;
- 3-[(2-Hydroxy-4-amino-5-methoxyphenylcarbamoyl)methyl]-1-methyl-3H-imidazol-1-ium chloride;
- 3-[(2-Hydroxy-4-acetylamino-5-methoxyphenylcarbamoyl)methyl]-1-methyl-3H-imidazol-1-ium chloride;
- 3-[(2-Hydroxy-4-methoxycarbonylamino-5-methoxyphenylcarbamoyl)methyl]-1-methyl-3H-imidazol-1-ium chloride;
- 3-[(3-Hydroxy-4-acetylamino-6-methoxyphenylcarbamoyl)methyl]-1-methyl-3H-imidazol-1-ium chloride;
- 3-[(3-Hydroxy-4-methoxycarbonylamino-6-methoxyphenylcarbamoyl)methyl]-1-methyl-3H-imidazol-1-ium chloride;
- 3-[(2-Hydroxy-6-aminophenylcarbamoyl)methyl]-1-methyl-3H-imidazol-1-ium chloride;
- 3-[(2-Hydroxy-6-acetylaminophenylcarbamoyl)methyl]-1-methyl-3H-imidazol-1-ium chloride;
- 3-[(2-Hydroxy-4,6-diaminophenylcarbamoyl)methyl)-1-methyl-3H-imidazol-1-ium chloride;
- 3-[(2-Hydroxy-4-acetylamino-6-aminophenylcarbamoyl)methyl]-1-methyl-3H-imidazol-1-ium chloride;
- 3-[(2-Hydroxy-3,5-dichloro-4-methylphenylcarbamoyl)methyl]-1-methyl-3H-imidazol-1-ium chloride;
- 3-[(2-Hydroxy-3,5-dichloro-4-aminophenylcarbamoyl)methyl]-1-methyl-3H-imidazol-1-ium chloride;
- 3-[(2-Hydroxy-3,5-dichloro-4-acetylaminophenylcarbamoyl)methyl]-1-methyl-3H-immidazol-1-ium chloride;
- 3-[(2-Hydroxy-3,5-dichloro-4-methoxycarbonylaminophenylcarbamoyl)methyl]-1-methyl-3H-imidazol-1-ium chloride;
- 3-[(2-Hydroxy-3-acetylaminophenylcarbamoyl)methyl]-1methyl-3H-imidazol-1-ium chloride;
- 1-[(2-Hydroxyphenylcarbamoyl)methyl]pyridinium chloride;
- 1-[(2-Hydroxy-3-(2-(pyridinium-1-yl)acetylamino)phenylcarbamoyl)methyl]pyridinium dichloride;
- 1-[(2-Hydroxy-4-(2-(pyridinium-1-yl)acetylamino)phenylcarbamoyl)methyl]pyridinium dichloride;
- 1-[(2-Hydroxy-4-methylphenylcarbamoyl)methyl]pyridinium chloride;
- 1-[(2-Hydroxy-4-aminophenylcarbamoyl)methyl]pyridinium chloride;
- 1-[(2-Hydroxy-4-acetylaminophenylcarbamoyl]methyl]pyridinium chloride;
- 1-[(2-Hydroxy-4-methoxycarbonylaminophenylcarbamoyl)methyl]pyridinium chloride;
- 2-[(3-Hydroxy-4-acetylaminophenylcarbamoyl)methyl]pyridinium chloride;
- 1-[(3-Hydroxy-4-methoxycarbonylaminophenylcarbamoyl)methyl]pyridinium chloride;
- 1-[(2-Hydroxy-5-chlorophenylcarbamoyl)methyl]-pyridinium chloride;
- 1-[(2-Hydroxy-4-methyl-5-chlorophenylcarbamoyl)methyl]pyridinium chloride;
- 1-[(2-Hydroxy-4-amino-5-chlorophenylcarbamoyl)methyl]pyridinium chloride;
- 1-[(2-Hydroxy-4-acetylamino-5-chlorophenylcarbamoyl)methyl)pyridinium chloride;
- 1-[(2-Hydroxy-4-methoxycarbonylamino-5-chlorophenylcarbamoyl)methyl]pyridinium chloride;
- 1-[(3-Hydroxy-4-acetylamino-6-chlorophenylcarbamoyl)methyl]pyridinium chloride;
- 1-[(3-Hydroxy-4-methoxycarbonylamino-6-chlorophenylcarbamoyl)methyl]pyridinium chloride;
- 1-[(2-Hydroxy-5-methoxyphenylcarbamoyl)methyl]pyridinium chloride;
- 1-[(2-Hydroxy-4-methyl-5-methoxyphenylcarbamoyl)methyl]pyridinium chloride;
- 1-[(2-Hydroxy-4-amino-5-methoxyphenylcarbamoyl)methyl]pyridinium chloride;
- 1-[(2-Hydroxy-4-acetylamino-5-methoxyphenylcarbamoyl)methyl]pyridinium chloride;
- 1-[(2-Hydroxy-4-methoxycarbonylamino-5-methoxyphenylcarbamoyl)methyl]pyridinium chloride;
- 1-[(3-Hydroxy-4-acetylamino-6-methoxyphenylcarbamoyl)methyl]pyridinium chloride;
- 1-[(3-Hydroxy-4-methoxycarbonylamino-6-methoxyphenylcarbamoyl)methyl]pyridinium chloride;
- 1-[(2-Hydroxy-6-aminophenylcarbamoyl)methyl]pyridinium chloride;
- 1-[(2-Hydroxy-6-acetylaminophenylcarbamoyl)methyl]pyridinium chloride;
- 1-[(2-Hydroxy-4,6-diaminophenylcarbamoyl)methyl]pyridinium chloride;
- 1-[(2-Hydroxy-4-acetylamino-6-aminophenylcarbamoyl)methyl]pyridinium chloride;
- 1-[(2-Hydroxy-3,5-dichloro-4-methylphenylcarbamoyl)methyl]pyridinium chloride;
- 1-[(2-Hydroxy-3,5-dichloro-4-aminophenylcarbamoyl)methyl]pyridinium chloride;
- 1-[(2-Hydroxy-3,5-dichloro-4-acetylaminophenylcarbamoyl)methyl]pyridinium chloride;
- 1-[(2-Hydroxy-3, 5-dichloro-4-methoxycarbonylaminophenylcarbamoyl)methyl]pyridinium chloride;
- 1-[(2-Hydroxy-3-acetylaminophenylcarbamoyl)methyl]pyridinium chloride;
- 1-[(2-Hydroxyphenylcarbamoyl)methyl]-1,4-dimethylpiperazin-1-ium chloride;
- 1-[(2-Hydroxy-3-(2-(1,4-dimethylpiperazin-1-ium-1-yl)acetyl)aminophenylcarbamoyl)methyl]-1,4-dimethylpiperazin-1-ium dichloride;
- 1-[(2-Hydroxy-4-(2-(1,4-dimethylpiperazin-1-ium-1-yl)acetyl)aminophenylcarbamoyl)methyl]-1,4-dimethyl-piperazin-1-ium dichloride;
- 1-[(2-Hydroxy-4-methylphenylcarbamoyl)methyl]-1,4dimethylpiperazin-1-ium chloride;
- 1-[(2-Hydroxy-4-aminophenylcarbamoyl)methyl]-1,4-dimethylpiperazin-1-ium chloride;
- 1-[(2-Hydroxy-4-acetylaminophenylcarbamoyl)methyl]-1,4-dimethylpiperazin-1-ium chloride;
- 1-[(2-Hydroxy-4-methoxycarbonylaminophenylcarbamoyl)methyl]-2,4-dimethylpiperazin-1-ium chloride;
- 1-[(3-Hydroxy-4-acetylaminophenylcarbamoyl)methyl]1,4-dimethylpiperazin-1-ium chloride;
- 1-[(3-Hydroxy-4-methoxycarbonylaminophenylcarbamoyl)methyl]-1,4-dimethylpiperazin-1-ium chloride;
- 1-[(2-Hydroxy-5-chlorophenylcarhamoyl)methyl]-1,4dimethylpiperazin-1-ium chloride;
- 1-[(2-Hydroxy-4-methyl-5-chlorophenylcarbamoyl)-methyl]-1,4-dimethylpiperazin-1-ium chloride;
- 1-[(2-Hydroxy-4-amino-5-chlorophenylcarbamoyl)methyl]-1,4-dimethylpiperazin-1-ium chloride;
- 1-[(2-Hydroxy-4-acetylamino-5-chlorophenylcarbamoyl)methyl]-1,4-dimethylpiperazin-1-ium chloride;
- 1-[(2-Hydroxy-4-methoxycarbonylamino-5-chlorophenylcarbamoyl)methyl]-1,4-dimethylpiperazin-1-ium chloride;
- 1-[(3-Hydroxy-4-acetylamino-6-chlorophenylcarbamoyl)methyl]-1,4-dimethylpiperazin-1-ium chloride;
- 1-[(3-Hydroxy-4-methoxycarbonylamino-6-chlorophenylcarbamoyl)methyl]-1,4-dimethylpiperazin-1-ium chloride;
- 1-[(2-Hydroxy-5-methoxyphenylcarbamoyl)methyl]-1,4-dimethylpiperazin-1-ium chloride;
- 1-[(2-Hydroxy-4-methyl-5-methyl-5-methoxyphenylcarbamoyl)methyl]-1,4-dimethylpiperazin-1-ium chloride;
- 1-[(2-Hydroxy-4-amino-5-methoxyphenylcarbamoyl)methyl]-1,4-dimethylpiperazin-1-ium chloride;
- 1-[(2-Hydroxy-4-acetylamino-5-methoxyphenylcarbamoyl)methyl]-1,4-dimethylpiperazin-1-ium chloride;
- 1-[(2-Hydroxy-4-methoxycarbonylamino-5-methoxyphenylcarbamoyl)methyl]-1,4-dimethylpiperazin-1-ium chloride;
- 1-[(3-Hydroxy-4-acetylamino-6-methoxyphenylcarbamoyl)methyl]-1,4-dimethylpiperazin-1-ium chloride;
- 1-[(3-Hydroxy-4-methoxycarbonylamino-6-methoxyphenylcarbamoyl]methyl]-1,4-dimethylpiperazin-1-ium chloride;
- 1-[(2-Hydroxy-6-aminophenylcarbamoyl)methyl]-1,4-dimethylpiperazin-1-ium chloride;
- 1-[(2-Hydroxy-6-acetylaminophenylcarbamoyl)methyl]1,4-dimethylpiperazin-1-ium chloride;
- 1-[(2-Hydroxy-4,6-diaminophenylcarbamoyl)methyl]-1,4-dimethylpiperazin-1-ium chloride;
- 1-[(2-Hydroxy-4-acetylamino-6-aminophenylcarbamoyl)methyl]-1,4-dimethylpiperazin-1-ium chloride;
- 1-[(2-Hydroxy-3,5-dichloro-4-methylphenylcarbamoyl)methyl]-1,4-dimethylpiperazin-1-ium chloride;
- 1-[(2-Hydroxy-3,5-dichloro-4-aminophenylcarbamoyl)methyl]-1,4-dimethylpiperazin-1-ium chloride;
- 1-[(2-Hydroxy-3,5-dichloro-4-acetylaminophenylcarbamoyl]methyl]-1,4-dimethylpiperazin-1-ium chloride;
- 1-[(2-Hydroxy-3,5-dichloro-4-methoxycarbonylaminophenylcarbamoyl)methyl]-1,4-dimethylpiperazin-1-ium chloride;
- 1-[(2-Hydroxy-3-acetylaminophenylcarbamoyl)methyl]-1,4-dimethylpiperazin-1-ium chloride;
and their addition salts with an acid.

23. Use of the compounds of formula (I') as defined in either of Claims 21 and 22 as coupler for the oxidation dyeing of keratinous fibres.
